# EUROPEAN PATENT SPECIFICATION

(11) **EP 2 953 957 B1**
(45) Date of publication and mention of the grant of the patent: **10.04.2019**
(21) Application number: 14706210.3
(22) Date of filing: 07.02.2014
(51) Int. Cl.: C07H 15/18, A61K 31/7032, A61P 31/00, A61P 33/00, A61P 35/00, A61P 37/00, A61P 29/00

(54) **MODIFIED GLYCOLIPIDS AND METHODS OF MAKING AND USING THE SAME**
MODIFIZIERTE GLYKOLIPIDEN UND VERFAHREN ZU DEREN HERSTELLUNG UND VERWEDUNG
GLYCOLIPIDES MODIFIÉS ET MÉTHODES POUR LEUR PRÉPARATION ET UTILISATION

(30) Priority: 08.02.2013 US 201361762591 P; 14.03.2013 US 201313803972; 02.07.2013 US 201361842149 P
(43) Date of publication of application: 16.12.2015
(73) Proprietor: Vaccinex, Inc., Rochester, NY 14620 (US); Albert Einstein College of Medicine, Inc., Bronx, NY 10461 (US)
(72) Inventor: PORCELLI, Steven A., Hartsdale, New York 10530-1657 (US); ZAUDERER, Maurice, Pittsford, New York 14534 (US)
(74) Representative: J A Kemp
(86) International application number: PCT/US2014/015286
(87) International publication number: WO 2014/124245

(56) References cited:
- US-A1- 2006 052 316
- HASHIMOTO M ET AL: "Versatile synthesis of phenoxydiazirine-based fatty acid analogs and photoreactive galactosylceramide", BIOORGANIC & MEDICINAL CHEMISTRY LETTERS, PERGAMON, AMSTERDAM, NL, vol. 12, 1 January 2002 (2002-01-01), pages 89-91, XP002229065, ISSN: 0960-894X, DOI: 10.1016/S0960-894X(01)00669-2
- M M ZEGERS ET AL: "Use of photoactivatable sphingolipid analogues to monitor lipid transport in mammalian cells", BIOCHEMICAL JOURNAL, vol. 328 ( Pt 2), no. Pt 2, 1 December 1997 (1997-12-01), pages 489-498, XP055114434, ISSN: 0264-6021
- RAVI&EMSP14;S. LANKALAPALLI ET AL: "Synthesis and Properties of a Photoactivatable Analogue of Psychosine ([beta]-Galactosylsphingosine)", CHEMMEDCHEM, vol. 5, no. 5, 3 May 2010 (2010-05-03), pages 682-686, XP055114402, ISSN: 1860-7179, DOI: 10.1002/cmdc.201000018
- VALERIE W HU ET AL: "Photoreactive labeling of M13 coat protein in model membranes by use of a glycolipid probe (photogenerated nitrene/membrane mapping/membrane restriction)", PROC. NATL. ACAD. SC., vol. 76, no. 11, 1 November 1979 (1979-11-01), pages 5460-5464, XP055114438,
- ITSURO TOMATSU ET AL: "Photoresponsive hydrogels for biomedical applications", ADVANCED DRUG DELIVERY REVIEWS, ELSEVIER, AMSTERDAM, NL, vol. 63, no. 14, 20 June 2011 (2011-06-20) , pages 1257-1266, XP028114519, ISSN: 0169-409X, DOI: 10.1016/J.ADDR.2011.06.009 [retrieved on 2011-07-02]
- SCHIEFNER A ET AL: "Structural Evaluation of Potent NKT Cell Agonists: Implications for Design of Novel Stimulatory Ligands", JOURNAL OF MOLECULAR BIOLOGY, ACADEMIC PRESS, UNITED KINGDOM, vol. 394, no. 1, 20 November 2009 (2009-11-20), pages 71-82, XP026739091, ISSN: 0022-2836, DOI: 10.1016/J.JMB.2009.08.061 [retrieved on 2009-09-02]

## Description

### FIELD OF THE INVENTION

The invention generally relates to the field of immunology. In particular, the invention involves the modification of glycolipids that are useful in modulating an immune response.

### BACKGROUND OF THE INVENTION

Natural killer T (NKT) cells represent a subset of T lymphocytes expressing both T-cell receptor and NK-cell receptor, and play a role in bridging innate immunity to adaptive immunity. Kronenberg M and Gapin L, Nat Rev Immunol 2: 557-568 (2002). Upon activation, NKT cells can have a pronounced impact on early and delayed immunity to various pathogens, including *L. monocytogenes, M. tuberculosis* and *Leishmania major.* Kronenberg (2002); Behar SM and Porcelli SA, Curr Top Microbiol Immunol 314: 215-250 (2007); Emoto M et al., Eur J Immunol 29: 650-659 (1999); Ishikawa H et al., Int Immunol 12: 1267-1274 (2000); and Ranson T et al., J Immunol 175: 1137-1144 (2005). NKT cell activation has been reported to lead to enhanced CD4 and CD8 T cell responses, and to induce dendritic cell (DC) maturation. Nishimura T et al., Int Immunol 12: 987-994 (2000) and Silk JD et al., J Clin Invest 114: 1800-1811 (2004).

Unlike conventional T cells that recognize MHC-bound peptides, NKT cells are specific for lipid antigens presented by the MHC class I-like protein CD1d. Several glycolipid antigens, including self-derived and bacterial-derived glycolipids, which can be presented by CD1d to activate NKT cells, have been identified to date. Tsuji M Cell Mol Life Sci 63: 1889-1898 (2006). NKT cells that have T-cell receptors with invariant Va14-Jα18 rearrangements (iNKT cells) possess reactivity to a glycosphingolipid, α-galactosylceramide (αGalCer), when presented by CD1d. Kronenberg M and Gapin L, Nat Rev Immunol 2: 557-568 (2002); Kronenberg M, Annu Rev Immunol 23: 877-900 (2005). Recent studies have shown that vaccines against Plasmodia, *Leishmania donovanii*, *Listeria monocytogenes* and HIV could be improved by activating iNKT cells through co-administration of αGalCer as an adjuvant. Gonzalez-Aseguinolaza G et al., J Exp Med 195: 617-624 (2002*)*; Dondji B et al., European Journal of Immunology 38: 706-719 (2008); Huang YX et al., Vaccine 26: 1807-1816 (2008); and Enomoto N et al., FEMS Immunol Med Microbiol 51: 350-362 (2007).

As a therapeutic, aGalCer has been shown to reduce malarial parasite load in mice and prolong the survival of *M. tuberculosis* infected mice. Gonzalez-Aseguinolaza G et al., Proc Natl Acad Sci USA 97: 8461-8466 (2000); Chackerian A et al., Infection and Immunity 70: 6302-6309 (2002). A single injection of aGalCer in mice induces secretion of IFNy, IL-12 and IL-4 in serum. Fujii S et al., Immunol Rev 220: 183-198 (2007). Stimulation of CD1d-restricted iNKT cells by aGalCer also leads to a rapid cascade of activation of immune and inflammatory cells including NK cells, dendritic cells, B cells, and conventional T cells. Nishimura T et al., Int Immunol 12: 987-994 (2000); Kitamura H et al., J Exp Med 189: 1121-1128 (1999); Fujii S et al., J Exp Med 198: 267-279 (2003). iNKT cells produce large amounts of IL-4 and IFNγ and the production requires direct contact between iNKT cells and DCs through CD40-CD40 ligand interactions. Nishimura T et al., Int Immunol 12: 987-994 (2000). IFNγ produced by iNKT cells has been shown to have a critical role in the antimetastatic effect of aGalCer in murine tumor models. Hayakawa Y et al., Eur J Immunol 31: 1720-1727 (2001); Smyth MJ et al., Blood 99: 1259-1266 (2002). Thus, activation of iNKT cells may play a role in modulating adaptive immune responses by influencing the early cytokine environment, thereby enhancing host resistance to infectious and inflammatory diseases.

Soluble CD1d proteins loaded with glycolipids such as α-galactosylceramides are being developed as immunotherapeutic agents. However, their utility is limited by the instability of the noncovalent interaction between the glycolipid and the protein, which allows them to rapidly dissociate or be displaced by natural competitive inhibitors and lose activity *in vivo.* Webb TJ et al. Cancer Res 72: 3744-3752 (2012). There remains a need to develop a method for covalently linking the glycolipid to the CD1d protein to create stable and long-lived complexes that maintain potent NKT-cell activating properties.

US 2006/052316 discloses α-galactosylceramides that modulate NKT cells. Schiefher *et al* discloses the structural evaluation of potent NKT cell agonists.

### SUMMARY OF THE INVENTION

The present invention relates to modified glycolipids comprising a functional group that allows for a stable covalent linkage to a protein, such as CD1d, complexes, cells, and vaccines comprising the same, and methods for producing and using the same. Modified glycolipid/protein complexes of the invention comprise a modified glycolipid physically associated with a protein (CD1d). The modified glycolipids of the invention comprise a benzophenone group. Thus, the modified glycolipid/protein complexes comprise the glycolipid covalently bound to a protein (CD1d) via a benzophenone linkage. Modified glycolipids find use in serving as a means for covalently binding glycolipids to other molecules, such as proteins. Modified ceramide-like glycolipid/CD1d complexes find use in methods for enhancing or eliciting an immune response, methods for enhancing the activity of Natural Killer T cells, and methods for treating or preventing a disease.

Specifically, the present invention provides:
(1) A ceramide-like glycolipid comprising a photoreactive group, wherein:
   - said photoreactive group is a benzophenone group covalently bound to the N-acyl lipophilic moiety of said ceramide-like glycolipid; and
   - said ceramide-like glycolipid is an α-galactosylceramide;
   wherein said ceramide-like glycolipid is capable of covalently binding to CD1d and enhancing the activity of natural killer (NKT) cells.
(2) The ceramide-like glycolipid of (1), wherein said N-acyl lipophilic moiety is a linear acyl chain, and wherein said linear acyl chain optionally has at least 11 atoms, optionally between 11 and 14 atoms, wherein the numbering of atoms excludes hydrogens.
(3) The ceramide-like glycolipid of (2), wherein said acyl chain is selected from the group consisting of: 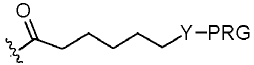 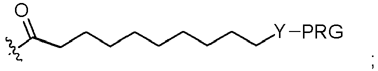 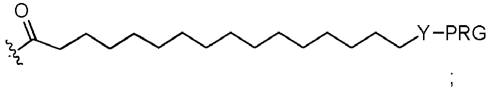 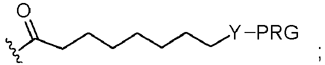 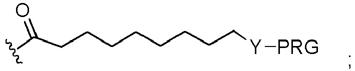 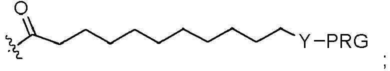 and 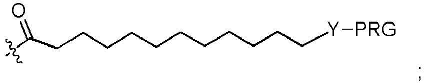 wherein Y is -O-, -CH₂-, -S-, -OCH₂-, -SCH₂-, -CH₂CH₂-; or a bond, and PRG is said photoreactive group. 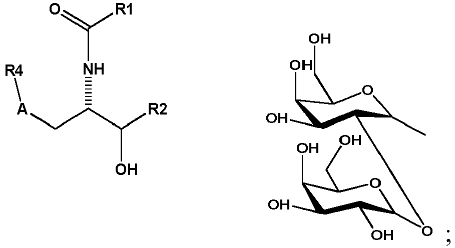
(4) The ceramide-like glycolipid of (1), wherein said α-galactosylceramide is selected from the group consisting of:
   a) Formula II: 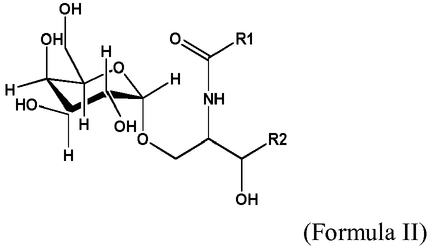 wherein
      R1 is a linear or branched C₁-C₂₇ alkane or C₂-C₂₇ alkene; R1 is -C(OH)-R3; or wherein R1 is selected from the group consisting of: (CH₂)₉CH=CH-CH₂-CH=CH(CH₂)₄CH₃, (CH₂)₈CH=CH-CH₂-CH=CH(CH₂)₄CH₃, (CH₂)₇CH=CH-CH₂-CH=CH(CH₂)₄CH₃, (CH₂)₃CH=CH-CH₂-CH=CH-CH₂-CH=CH-CH₂-CH=CH-(CH₂)₄CH₃, (CH₂)₃CH=CH-CH₂-CH=CH-CH₂-CH=CH-CH₂-CH=CH-CH₂-CH=CH-CH₂CH₃, (CH₂)₇CH=CH-CH₂-CH=CH=(CH₂)₄CH₃, (CH₂)₇CH=CH-CH=CH(CH₂)₅CH₃, (CH₂)₈CH=CH-CH=CH(CH₂)₄CH₃, (CH₂)₉CH=CH-CH=CH(CH₂)₅CH₃, (CH₂)₆CH=CH-CH=CH-CH=CH(CH₂)₄CH₃, (CH₂)₆CH=CH-CH=CH-CH=CH(CH₂)₄CH₃ and (CH₂)₇CH=CH-CH=CH-CH=CH(CH₂)₃CH₃;
      wherein R3 is linear or branched C₁-C₂₆ alkane or C₂-C₂₆ alkene; and
      R2 is one of the following (a)-(e):
         (a) -CH₂(CH₂)ₓCH₃,
         (b) -CH(OH)(CH₂)ₓCH₃,
         (c) -CH(OH)(CH₂)ₓCH(CH₃)₂,
         (d) -CH=CH(CH₂)ₓCH₃,
         (e) -CH(OH)(CH₂)ₓCH(CH₃)CH₂CH₃,
      wherein X is an integer ranging from 4-17,
      wherein R2 is optionally -CH(OH)(CH₂)ₓCH₃, wherein X is an integer ranging from 4-13,
      wherein R2 is optionally -CH(OH)-(CH₂)₁₃CH₃;
   b) Formula III: 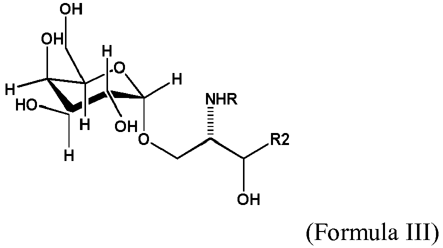 wherein R is -C(O)R1, wherein R1 is a linear or branched C₁-C₂₇ alkane or C₂-C₂₇ alkene; or R1 is -C(OH)-R3 wherein R3 is a linear or branched C₁-C₂₆ alkane or C₂-C₂₆ alkene; or R1 is a C₆-C₂₇ alkane or alkene wherein (i) the C₆-C₂₇ alkane or alkene is substituted with a C₅-C₁₅ cycloalkane, C₅-C₁₅ cycloalkene, heterocycle, or aromatic ring or (ii) the C₆-C₂₇ alkane or alkene includes, within the C₆-C₂₇ alkyl or alkenyl chain, a C₅-C₁₅ cycloalkane, C₅-C₁₅ cycloalkene, heterocycle, or aromatic ring; or R1 is an optionally substituted aromatic ring, or an aralkyl, or R1 is selected from the group consisting of: 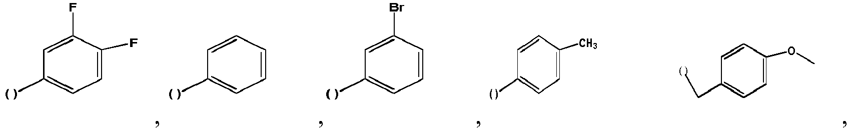 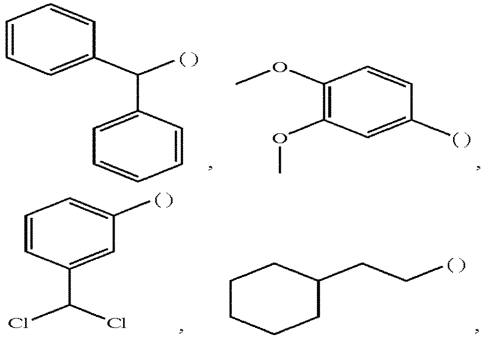 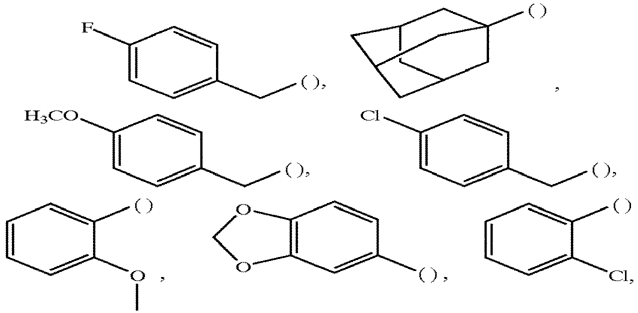 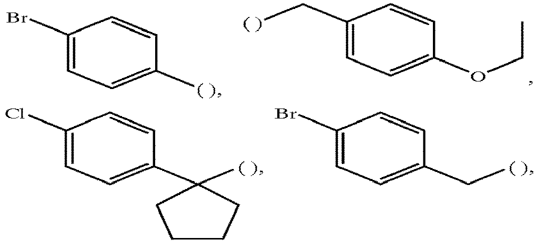 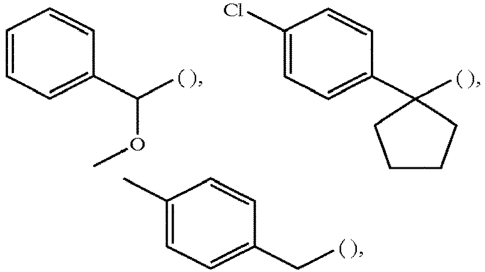 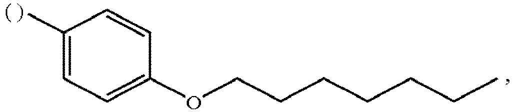 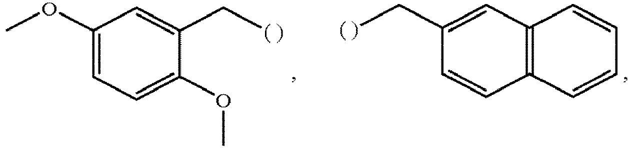 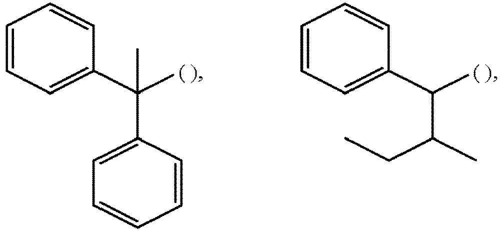 and 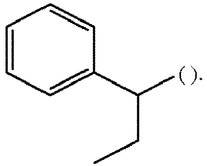
      where ( ) represent the point of attachment of R1 to the compound of Formula III; and
      R2 is one of the following (a)-(e):
         (a) -CH₂(CH₂)ₓCH₃,
         (b) -CH(OH)(CH₂)ₓCH₃,
         (c) -CH(OH)(CH₂)ₓCH(CH₃)₂,
         (d) -CH=CH(CH₂)ₓCH₃,
         (e) -CH(OH)(CH₂)ₓCH(CH₃)CH₂CH₃,
      wherein X is an integer ranging from 4-17,
      wherein optionally R1 is substituted with oxo; hydroxy; halogen; phenyl; - OC(O)R6; -OR6; -C(O)R6; or N(R6)₂,
      wherein each R6 is independently a C₁-C₆ substituted alkyl, or a substituted aromatic ring optionally substituted with halogen; hydroxy; -OC(O)R7; -OR7; -C(O)R7 or N(R7)₂, and
      wherein each R7 is a substituted C₁-C₆ alkyl; and
   c) (2S, 3S, 4R)-1-O-(α-D-galactopyranosyl)-N-hexacosanoyl-2-amino-1,3,4-octadecanetriol (KRN7000), (2S,3S)-1-O-(α-D-galactopyranosyl)-N-hexacosanoyl-2-amino-1,3-octadecanediol), or (2S, 3S, 4R)-1-CH₂-(α-galactopyranosyl)-N-hexacosanoyl-2-amino-1,3,4-octadecanetriol (α-C-GalCer).
(5) The ceramide-like glycolipid of (1), wherein said ceramide-like glycolipid has the structure selected from the group consisting of:
   a)
   b) 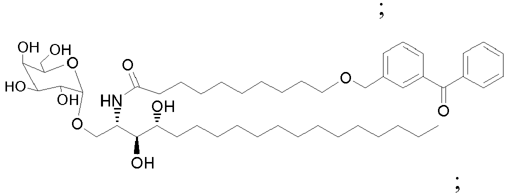
   c) 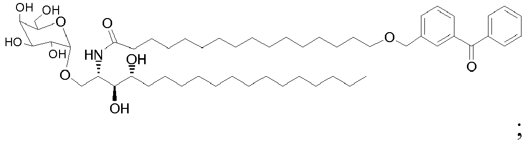
   d) 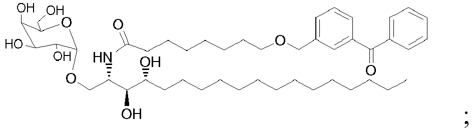
   e) 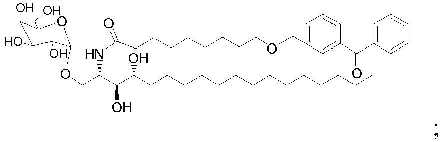
   f) 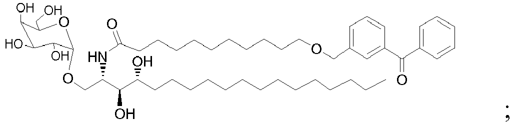 and
   g) 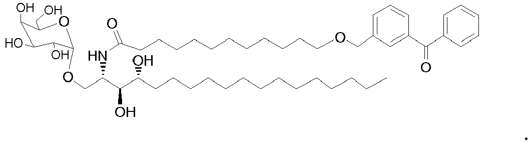
(6) A ceramide-like glycolipid/protein complex comprising a CD1d protein and the ceramide-like glycolipid of any one of (1)-(6), wherein said ceramide-like glycolipid is covalently linked to said protein via photoactivation of the benzophenone group.
(7) The ceramide-like glycolipid/protein complex of (6), wherein said CD1d:
   a) has an amino acid sequence selected from the group consisting of:
      i) an amino acid sequence at least 90% identical to amino acids 21 to 295 of SEQ ID NO:1;
      ii) the amino acid sequence set forth as amino acids 21 to 295 of SEQ ID NO:1;
      iii) an amino acid sequence identical to amino acids 21 to 295 of SEQ ID NO:1, except for at least one but less than 10 conservative amino acid substitutions;
      iv) the amino acid sequence set forth as amino acids 1 to 295 of SEQ ID NO:1;
      v) an amino acid sequence at least 90% identical to amino acids 1 to 295 of SEQ ID NO:1;
      vi) an amino acid sequence identical to amino acids 21 to 295 of SEQ ID NO:1, except for at least one but less than 10 conservative amino acid substitutions;
      vii) the amino acid sequence set forth in SEQ ID NO:1;
      viii) an amino acid sequence at least 90% identical to SEQ ID NO:1; and
      ix) an amino acid sequence identical to SEQ ID NO:1, except for at least one but less than 10 conservative amino acid substitutions; or
   b) is physically associated with a β2-microglobulin,
      wherein said β2-microglobulin optionally has an amino acid sequence selected from the group consisting of:
      i) an amino acid sequence at least 90% identical to amino acids 21 to 113 of SEQ ID NO:2;
      ii) the amino acid sequence set forth as amino acids 21 to 113 of SEQ ID NO:2;
      iii) an amino acid sequence identical to amino acids 21 to 113 of SEQ ID NO:2, except for at least one but less than 10 conservative amino acid substitutions;
      iv) the amino acid sequence set forth in SEQ ID NO:2;
      v) an amino acid sequence at least 90% identical to SEQ ID NO:2; and
      vi) an amino acid sequence identical to SEQ ID NO:2, except for at least one but less than 10 conservative amino acid substitutions.
(8) The ceramide-like glycolipid/protein complex of (6) or (7), wherein said ceramide-like glycolipid/protein complex:
   a) further comprises an antibody or fragment thereof specific for a target antigen, and wherein said antibody or antigen-binding fragment thereof is linked to said CD1d,
      wherein said antigen-binding fragment is optionally selected from the group consisting of a F(ab) fragment, a F(ab')2 fragment, and a single-chain antibody,
      wherein said target antigen is optionally a cell surface marker of tumor cells,
      wherein said cell surface marker is optionally selected from the group consisting of CEA, Her2/neu, EGFR type I or type II, CD19, CD20, CD22, Muc-1, PSMA, or STEAP,
      wherein said CD1d is optionally attached to the heavy chain or light chain of said antibody or heavy chain fragment or light chain fragment of said antigen-binding antibody fragment; or
   b) further comprises an antigen,
      wherein said antigen is optionally an immunogenic polypeptide of a virus or a cell selected from the group consisting of a virus, bacterium, fungus, and tumor cell.
(9) A composition comprising the ceramide-like glycolipid/protein complex of any one of (6)-(8), and a pharmaceutical carrier,
   wherein said pharmaceutical carrier is optionally selected from the group consisting of saline, buffered saline, dextrose, water, glycerol, and combinations thereof.
(10) The ceramide-like glycolipid/protein complex of any one of (6)-(8) or the composition of (9) for use in a method of treating or preventing a disease in a subject, said method comprising administering to a subject in need of said treatment or prevention the ceramide-like glycolipid/protein complex or composition, wherein said ceramide-like glycolipid/protein complex is administered in an amount sufficient to alter the progression of said disease
   wherein an immune response is optionally enhanced or modified relative to an immune response produced by the protein not covalently linked to the glycolipid,
   wherein said disease is optionally selected from the group consisting of a viral disease, a bacterial disease, a fungal disease, a parasitic disease, a proliferative disease, an autoimmune disease, an inflammatory disease, tuberculosis, pulmonary disease resembling tuberculosis, lymphadenitis, skin disease, disseminated disease, bubonic plague, pneumonic plague, tularemia, Legionairre's disease, anthrax, typhoid fever, paratyphoid fever, foodborne illness, listeriosis, malaria, HIV, SIV, HPV, RSV, influenza, hepatitis (HAV, HBV, and HCV), multiple sclerosis, diabetes, Sjogren's Syndrome, and cancer;
   wherein said subject is optionally a mammal, and wherein said mammal is optionally a human.
(11) The ceramide-like glycolipid/protein complex of any one of (6)-(8) or the composition of (9) for use in a method of inducing an immune response against an antigen in a subject, said method comprising administering to said subject the ceramide-like glycolipid/protein complex or composition
   wherein said ceramide-like glycolipid/protein complex optionally comprises said antigen,
   wherein said antigen is optionally covalently bound to said protein,
   wherein said ceramide-like glycolipid/protein complex is optionally administered in an amount sufficient to enhance the activity of Natural Killer T (NKT) cells in said subject,
   wherein said immune response optionally comprises an antibody response,
   wherein said subject is optionally a mammal, and wherein said mammal is optionally a human.
(12) A heterologous antigen and the ceramide-like glycolipid/protein complex of any one of (6)-(8) or the composition of (9) for use in a method for enhancing an immune response in a subject to the heterologous antigen, said method comprising administering to said subject the heterologous antigen and the ceramide-like glycolipid/protein complex or composition simultaneously, prior to, or subsequent to the administration of said heterologous antigen
   wherein said subject is optionally a mammal, and wherein said mammal is optionally a human.
(13) A method for synthesizing a ceramide-like glycolipid of Formula (VI) comprising a benzophenone group, said method comprising the steps of:
   activating a carboxylic acid derivative of Formula (IV) to form an activated acyl derivative 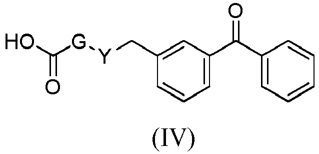 ; and
   coupling the activated acyl derivative with a ceramide-like-glycolipid of Formula (V), wherein said ceramide-like glycolipid is capable of covalently binding to CD1d and enhancing the activity of natural killer T (NKT) cells 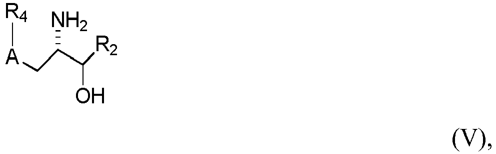
   thereby forming the ceramide-like glycolipid comprising a benzophenone group of Formula (VI) 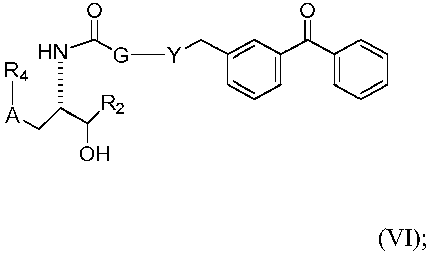 wherein
   G is a linear or branched C₁-C₂₇ alkane or C₂-C₂₇ alkene; or G is -C(OH)-R3 wherein R3 is a linear or branched C₁-C₂₆ alkane or C₂-C₂₆ alkene; or R1 is a C₆-C₂₇ alkane or alkene wherein (i) the C₆-C₂₇ alkane or alkene is substituted with a C₅-C₁₅ cycloalkane, C₅-C₁₅ cycloalkene, heterocycle, or aromatic ring or (ii) the C₆-C₂₇ alkane or alkene includes, within the C₆-C₂₇ alkyl or alkenyl chain, a C₅-C₁₅ cycloalkane, C₅-C₁₅ cycloalkene, heterocycle, or aromatic ring; or R1 is an optionally substituted aromatic ring, or an aralkyl;
   R₄ is an α-galactosyl;
   A is -O-, -CH₂-; or is a single bond,
   Y is -O-, -CH₂- or -S-, and
   R₂ is a substituted or an unsubstituted C3-C40 alkyl, alkenyl or alkynyl chain,
   wherein R2 of Formula (VI) is optionally one of the following (a)-(e):
      (a) -CH₂(CH₂)ₓCH₃,
      (b) -CH(OH)(CH₂)ₓCH₃,
      (c) -CH(OH)(CH₂)ₓCH(CH₃)₂,
      (d) -CH=CH(CH₂)ₓCH₃,
      (e) -CH(OH)(CH₂)ₓCH(CH₃)CH₂CH₃, wherein x is an integer ranging from 0-40, or
   wherein R2 of Formula (VI) is optionally:
      -CH(OH)(CH₂)ₓCH₃, wherein x is an integer ranging from 4-17; or
      -CH(OH)(CH₂)₁₃CH₃; or
   wherein optionally G is -(CH₂)n-, wherein n is an integer ranging from 0-20 and Y is O; or
   wherein:
      G is -(CH₂)n-, wherein n is an integer ranging from 0-20 and Y is O;
      R2 is -CH(OH)(CH₂)ₓCH₃, wherein x is an integer ranging from 4-17;
      A is O;
      Y is O; and
   wherein said ceramide-like glycolipid is capable of covalently binding to CD1d and enhancing the activity of natural killer T (NKT) cells.
(14) A method for producing a ceramide-like glycolipid/protein complex, said method comprising the steps of:
   a) contacting the ceramide-like glycolipid of any one of (1)-(5) or a ceramide-like glycolipid produced by the method of (13) with a CD1d protein; and
   b) irradiating said ceramide-like glycolipid and said CD1d protein with ultraviolet light to produce a ceramide-like glycolipid/protein complex,
   wherein said modified glycolipid/protein complex enhances the activity of natural killer T (NKT) cells.

### BRIEF DESCRIPTION OF THE FIGURES

Figure 1: shows a schematic for the synthesis of aGalCer-benzophenone derivatives.
Figure 2: shows a schematic for the synthesis of aGalCer-benzophenone/CDld complex.
Figure 3: shows fluorescence spectra of mouse CD1d with and without glycolipid ligands as an approach to estimating glycolipid binding. Structures of the various glycolipid ligands can be found in Table 1. 7DW8-5 is an α-GalCer analogue with a C10-fluoro-phenyl acyl moiety that does not contain photoreactive groups for formation of covalent bonds with CD1d.
Figure 4: shows iNKT hybridoma stimulation assay (measuring IL-2 secretion) using murine CD1d-expressing antigen presenting cells.
Figure 5: shows iNKT hybridoma stimulation assay (measuring IL-2 secretion) using human CD1d-expressing HeLa cells.
Figures 6A-6B: show detection of ligand binding affinity of plate-bound mCD1d by ELISA using mAb L363 to specifically detect murine CD1d/αGalCer complexes. Figure 6A shows samples that had not been irradiated with UV and Figure 6B shows samples that had been irradiated with UV. ND=not dissociated; D=dissociated
Figures 7A-7B: show detection of ligand binding affinity of plate-bound mCD1d by ELISA using iNKT DN3A4-1.2 hybridoma to detect murine CD1d/αGalCer complexes. Figure 7A shows samples that had not been irradiated with UV and Figure 7B shows samples that had been irradiated with UV.
Figures 8A-8B: show a direct comparison of DB12-8 and 7DW8-5 complexes with mCD1d by ELISA using mAb L363. Figure 8A shows samples that had not been irradiated with UV and Figure 8B shows samples that had been irradiated with UV. NDiss or NoDiss=not dissociated; Diss=dissociated
Figures 9A-9D: Figures 9A-9B show a direct comparison of DB12-8 and 7DW8-5 complexes with mCD1d.CEA fusion protein by ELISA using mAb L363. Figures 9C-9D show the effect of a 10 fold scale-up on UV crosslinking and dissociation of DB12-8:mCD1d.CEA. Figures 9A and 9C show samples that had not been irradiated with UV and Figures 9B and 9D show samples that had been irradiated with UV. NDiss or NoDiss=not dissociated; Diss=dissociated
Figure 10: shows the kinetics and magnitude of interferon γ (IFNg) and Interleukin-2 (IL-2) cytokines in serum of mice 2, 10 and 24 hrs after injection with saline or with 30 µg of either aGalCer non-covalently loaded on sCD1d-anti-CEA or UV cross-linked DB12-8:sCD1d-anti-CEA complexes.

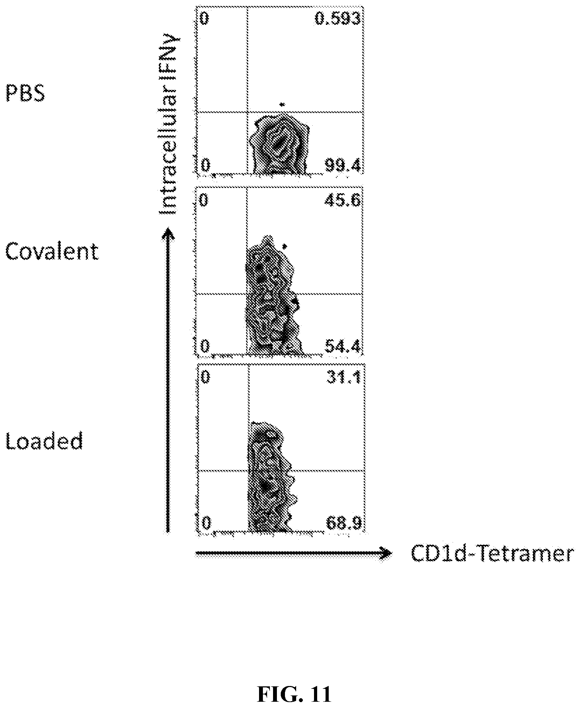

Figure 11: shows intracellular IFNg production by iNKT cells following three injections with saline or with 30 µg of either αGalCer non-covalently loaded on sCD1d-anti-CEA or UV cross-linked DB12-8:sCD1d-anti-CEA complexes at initiation of the experiment and again on day 2 and day 8. Mice were sacrificed 1h after the third injection and spleen iNKT cells were stained for intracellular IFNg with anti-IFNg-APC after fixation and permeabilization with Cytofix/Cytoperm (BD). To determine the percentage of iNKT cells that produce IFNg, cells were gated on positive staining for both CD3-FITC and aGalCer-loaded CD1d tetramer-PE and scored for expression of intracellular IFNg by flow cytometry on a FACS Calibur.
Figure 12: shows *in vivo* growth inhibition of carcino-embryonic antigen (CEA)-expressing MC38 tumor cells by α-galactosylceramide covalently linked to the sCD1d-anti-CEA fusion protein. Seven mice were treated on days 7, 11, 15, and 20 post-graft with PBS (saline), 3 µg of free αGalCer, or the molar equivalent of αGalCer covalently linked to sCD1 d-anti-CEA (sCD1d-antiCEA/α-GalCer Covalent). Tumor growth was measured every 2 or 3 days and mice were sacrificed at day 24. ** p=0.0079 (Two-way ANOVA)

### DETAILED DESCRIPTION OF THE INVENTION

The present invention relates to compositions, vaccines, and methods, which are useful for enhancing, *i.e.,* eliciting, stimulating or increasing, an immune response, *e.g.,* a primary and/or secondary immune response. Described herein are modified glycolipids comprising a functional group, such as benzophenone, that allow for a stable covalent linkage with a protein, such as CD1d. Complexes comprising the modified glycolipids and a physically associated protein (e.g., CD1d) and methods for synthesizing and using the same are also provided herein. The modified glycolipid or modified glycolipid/protein complex of the invention enhances an immune response by affecting the activity of CD1d-restricted natural killer T ("NKT") cells. The modified glycolipids and modified glycolipid/protein complexes as described herein are useful for stimulating desirable immune responses, for example, immune responses against mycobacterial antigens, tumors, heterologous antigens, or a targeting molecule. The immune response can be useful for preventing, treating or ameliorating diseases caused by bacterial pathogens; *e.g.,* mycobacteria, *e.g., Mycobacterium tuberculosis,* which causes TB in humans; other infectious agents; or tumors. In some aspects of the disclosure, employing certain forms of the glycolipid, as described elsewhere, the disclosure can be employed to redirect an inflammatory or autoimmune response from a predominantly TH1 to a TH2 response. Examples can be found, *e.g*., in U.S. Patent Appl. Publ. Nos. 2006/0052316 and 2010/0183549.

The natural immune system strikes a complex balance between highly aggressive, protective immune responses to foreign pathogens and the need to maintain tolerance to normal tissues. In recent years there has been increasing recognition that interactions among many different cell types contribute to maintaining this balance. Such interactions can, for example, result in polarized responses with either production of pro-inflammatory cytokines (*e.g*., interferon-gamma) by TH1 type T cells or production of interleukin-4 (IL-4) by TH2 type T cells that suppress TH1 activity. In a number of different animal models, T cell polarization to TH1 has been shown to favor protective immunity to tumors or infectious pathogens whereas T cell polarization to TH2 can be a critical factor in preventing development of cell-mediated autoimmune disease. The conditions that determine whether immune stimulation will result in aggressive cell-mediated immunity or in down regulation of such responses are highly localized in the sense that each tissue is comprised of a distinctive set of antigen presenting cells (APC) and lymphocyte lineages that interact to favor different immune responses. For example, under optimal conditions, the dendritic cells (DC) localized in a normal tissue can represent predominantly a lineage and stage of maturation that favors tolerogenic interactions and serves as a barrier to cell-mediated autoimmunity whereas a tumor or site of infection will attract mature myeloid dendritic cells that stimulate potent cell-mediated immune responses.

NKT cells have an unusual ability of secreting both TH1 and TH2 cytokines and potent cytotoxic as well as regulatory functions have been documented in inflammation, autoimmunity and tumor immunity (Bendelac et al., Science 268:863 (1995); Chen and Paul, J Immunol 159:2240 (1997); and Exley et al., J Exp Med 186:109 (1997)). They are also known for their rapid production of large quantities of both IL-4 and IFN-γ upon stimulation through their α-βTCRs (Exley et al., J. Exp. Med. 186:109 (1997).

CD1d-restricted NKT cells are a unique class of non-conventional T cells that appear to play an important role in defining the outcome of immune stimulation in the local environment. They share with the larger class of NKT cells the expression of markers of both the T cell and natural killer (NK) cell lineages. As such, NKT cells are considered as part of innate immunity like NK cells and in humans their frequency in normal individuals can be as high as 2.0% of total T lymphocytes (Gumperz et al., J Exp Med 195:625 (2002); Lee et al., J Exp Med 195:637 (2002)).

CD1d-restricted NKT cells are distinguished from other NKT cells by their specificity for lipid and glycolipid antigens presented by the monomorphic MHC class Ib molecule, CD1d (Kawano et al., Science 278:1626-1629 (1997)). CD1d is a non-MHC encoded molecule that associates with β2-microglobulin and is structurally related to classical MHC class I molecules. CD1d has a hydrophobic antigen-binding pocket that is specialized for binding the hydrocarbon chains of lipid tails or hydrophobic peptides. Zeng et al., Science 277: 339-345, (1997). CD1d is known to bind a marine sponge derived α-glycosylated sphingolipid, α-galactosylceramide (a-GalCer), and related molecules such as ceramide-like glycolipid antigens with α-linked galactose or glucose but not mannose. Kawano et al., Science 278:1626-1629 (1997); and Zeng et al., Science 277: 339-345 (1997). As discussed herein, the ability to activate CD1d-restricted NKT cells by stimulation with α-GalCer or related molecules bound to CD1d of antigen presenting cells has greatly facilitated functional analysis of this non-conventional T cell subset. In the absence of inflammation, CD1d-restricted NKT cells have been shown to localize preferentially in certain tissues like thymus, liver and bone marrow (Wilson et al., Trends Mol Med 8:225 (2002)) and antitumor activity of NKT cells has been investigated in, for example, mouse liver metastasis.

Among the CD1d-restricted NKT cells is a subset, referred to herein as "iNKT cells," that express a highly conserved αβT cell receptor (TCR). In humans this invariant TCR is comprised of Vα24Jα15 in association with Vβ11 whereas in mice the receptor comprises the highly homologous Vα14Jα18 and Vβ8.2. Other CD1d-restricted NKT cells express more variable TCR. Both TCR invariant and TCR variant classes of CD1d-restricted T cells can be detected by binding of CD1d-tetramers loaded with α-GalCer (Benlagha et al., J Exp Med 191:1895-1903 (2000); Matsuda et al., J Exp Med 192:741-754 (2000); and Karadimitris et al., Proc Natl Acad Sci USA 98:3294-3298 (2001)). CD1d-restricted NKT cells, as defined in this application (CD1d-restricted NKT), include cells that express either invariant or variant TCR and that bind or are activated by CD1d loaded with either α-GalCer or with related ceramide-like glycolipid antigens. CD1d-restricted NKT cells, as defined in this application (CD1d-NKT), include cells that express either invariant or variant TCR and that bind or are activated by CD1d loaded with either α-GalCer or with related sphingolipids that have α-linked galactose or glucose including molecules such as OCH, which differs from α-GalCer by having a shortened long-chain sphingosine base (C5 vs. C14) and acyl chain (C24 vs. C26) (Miyamoto et al., Nature 413:531-4 (2001)).

CD1d-restricted NKT have been shown to have direct cytotoxic activity against targets that express CD1d. It is likely, however, that the effect of CD1d-restricted NKT on immune responses is amplified through recruitment of other lymphocytes either by direct interaction or, perhaps even more importantly, by indirect recruitment through interaction with DC. CD1d-restricted NKT have the unique ability to secrete large quantities of IL-4 and IFN-γ early in an immune response. Secretion of IFN-γ induces activation of DC which produces interleukin-12 (IL-12). IL-12 stimulates further IFN-γ secretion by NKT cells and also leads to activation of NK cells which secrete more IFN-γ.

Since CD1d-restricted NKT are able to rapidly secrete large amounts of both IL-4 and IFN-γ, the polarization of immune responses will depend on whether the effect of pro-inflammatory IFN-γ or anti-inflammatory IL-4 cytokines predominate. This has been reported to be, in part, a function of the relative frequency of different subsets of CD1d-restricted NKT. These subsets include (i) an invariant CD1d-restricted NKT population that is negative for both CD4 and CD8 and that gives rise to predominantly a TH1 type response including secretion of pro-inflammatory IFN-γ and TNF-α and (ii) a separate population of CD1d-restricted NKT that is CD4+ and that gives rise to both a TH1 type and TH2 type response including secretion of the anti-inflammatory Th2-type cytokines IL-4, IL-5, IL-10 and IL-13 (Lee et al., J Exp Med 195:637-41 (2002); and Gumperz et al., J Exp Med 195:625-36 (2002)). In addition, NKT cell activity is differentially modulated by depending on the particular ceramide-like glycolipid bound to CD1d (see, *e.g.,* US Patent No. 7,772,380)). Compositions of the present disclosure include aspects with selective association of CD1d with these functionally different ceramide-like glycolipids and the related α-glycosylceramides. Local factors that influence activation of CD1d-restricted NKT subsets include the cytokine environment and, importantly, the DC that are recruited to that environment.

A family of ceramide-like glycolipids (*i.e*., α-galactosylceramide (α-GalCer) and related α-glycosyl ceramides), have been shown to stimulate strong CD1d-restricted responses by murine NKT cells (Kawano *et al.,* 1997). These compounds contain an α-anomeric hexose sugar (galactose or glucose being active for NKT cell recognition), distinguishing them from the ceramides that commonly occur in mammalian tissues which contain only β-anomeric sugars. These compounds are known to occur naturally in marine sponges, the source from which they were originally isolated, and became of interest to immunologists when it was demonstrated that α-GalCer induced dramatic tumor rejection as a result of immune activation when injected into tumor bearing mice (Kobayashi et al., Oncol.Res. 7:529-534 (1995)). Subsequently, this activity was linked to the ability of α-GalCer to rapidly activate NKT cells through a CD1d dependent mechanism. It has now been shown that α-GalCer binds to CD1d, thus creating a molecular complex that has a measurable affinity for the TCRs of NKT cells (Naidenko et al., J Exp.Med. 190:1069-1080 (1999); Matsuda et al., J Exp.Med. 192:741 (2000); Benlagha et al., J Exp.Med. 191:1895-1903 (2000)). Thus, α-GalCer provides a potent agent that can enable activation of the majority of NKT cells both *in vitro* and *in vivo.*

The most extensively studied NKT activating α-GalCer, called KRN7000 in the literature, is a synthetic molecule that has a structure similar to natural forms of α-GalCer that were originally isolated from a marine sponge on the basis of their anticancer activity in rodents (Kawano et al., Science 278:1626-1629 (1997); Kobayashi *et al*., 1995; Iijima et al., Bioorg. Med. Chem. 6:1905-1910 (1998); Inoue et al., Exp. Hematol. 25:935-944 (1997); Kobayashi et al., Bioorg. Med. Chem. 4:615-619 (1996a) and Biol. Pharm. Bull. 19:350-353 (1996b); Uchimura et al., Bioorg. Med. Chem. 5:2245-2249 (1997a); Uchimura et al., Bioorg. Med. Chem. 5:1447-1452 (1997b); Motoki et al., Biol. Pharm. Bull. 19:952-955 (1996a); Nakagawa et al., Oncol. Res. 10:561-568 (1998); Yamaguchi et al., Oncol. Res. 8:399-407 (1996)). One synthetic analogue of KRN7000 with a truncated sphingosine base showed an enhanced ability to suppress autoimmunity in a mouse model of experimental allergic encephalomyelitis (EAE) (Miyamoyo et al., Nature 413:531-534 (2001)). Other variants altered in the α-GalCer sphingosine base are identified in U.S. Pat. No. 5,936,076.

A large body of literature dating from November 1997 to the present time has studied the mechanism by which KRN7000 activates the immune system of mammals (Kawano et al., Science 278:1626-1629 (1997); Benlagha et al., J Exp. Med. 191:1895-1903 (2000); Burdin et al., Eur. J Immunol. 29:2014-2025 (1999); Crowe et al., J. Immunol. 171:4020-4027 (2003); Naidenko et al., J Exp. Med. 190:1069-1080 (1999); Sidobre et al., J. Immunol. 169:1340-1348 (2002); Godfrey et al., Immunol. Today 21:573-583 (2000); Smyth and Godfrey, Nat. Immunol. 1:459-460 (2000)). These studies uniformly show that the proximal mechanism for the effect of KRN7000 is the binding of this compound to a CD1d protein, which is expressed on most hematopoietic cells, as well as some epithelial and other cell lineages. The binding of KRN7000 to CD1d creates a molecular complex that is recognized with high affinity by the T cell antigen receptors (TCRs) of a subset of T lymphocytes called natural killer T cells (NKT cells). Recognition of the KRN7000/CD1d complex leads to rapid activation of the NKT cells, which reside in the liver, spleen and other lymphoid organs and have the potential to traffic to potentially any tissue. Activated NKT cells rapidly secrete a wide range of chemokines and cytokines, and also have the capability of activating other cell types such as dendritic cells and natural killer (NK) cells. The chain of events that follows the activation of NKT cells by KRN7000/CD1d complexes has been shown to have many potential downstream effects on the immune system. For example, in the setting of certain types of infections this can lead to an adjuvant effect that boosts the adaptive immunity to the infection and promotes healing. Or, in the setting of certain types of autoimmune diseases, the activation of NKT cells by KRN7000 can alter the course of the autoimmune response in a way that suppresses tissue destruction and ameliorates the disease.

A number of indirect mechanisms contribute to the protective effect of CD1d-restricted NKT cells. Activation of NKT cells by administration of α-GalCer *in vivo* results in concomitant activation of NK cells (Eberl and MacDonald, Eur. J. Immunol. 30:985-992 (2000),; and Carnaud et al., J. Immunol. 163:4647-4650 (1999)). In mice deficient in NKT cells, α-GalCer is unable to induce cytotoxic activity by NK cells. NKT cells also enhance the induction of classical MHC class I restricted cytotoxic T cells (Nishimura et al., Int Immunol 12:987-94 (2000); and Stober et al., J Immunol 170:2540-8 (2003)).

As discussed in more detail below, the present disclosure includes a glycolipid. As used herein, a "glycolipid" refers to a chemical compound comprising a carbohydrate moiety bound to a lipophilic moiety represented by general structure:
G-L
where L is a lipid and G is a carbohydrate. As used herein, a "lipid" refers to an organic compound that is soluble in fats, oils, and non-polar solvents, such as organic solvents. The term "lipophilic" refers to the property of an organic compound to dissolve in fats, oils, lipids, and non-polar solvents, such as organic solvents. Lipophilic compounds are sparingly soluble or insoluble in water. G may be a saccharide, which may be a hexose or pentose, and may be a mono-, di-, tri-, oligo or polysaccharide, or a derivative thereof. The sugars of interest include allose, altrose, glucose, mannose, gulose, idose, galactose, talose, fructose, maltose, lactose and sucrose. The linkage between the sugar and the lipid may be at any of the O atoms, usually at position 1, 2, 3 or 4, more usually at position 1. The linkage may be in the alpha or beta configuration. The linkage may be in the alpha configuration.

In some aspects of the disclosure, the glycolipids may have the structure: 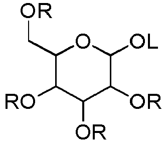 where L is a lipid, and R is selected from the group consisting of H, a pentose sugar, a hexose sugar, an oligo- or polysaccharide; or an alkyl, aryl or alkenyl group, such as a C1 to C6 lower alkyl, which alkyl is optionally substituted, which substituent may include, without limitation, an alkyl, aryl, alkenyl, aralkyl, aralkenyl, cycloalkyl, cycloalkylalkyl or cycloalkylalkenyl group; and may contain one or more N, S or O heteroatoms. Each of the O atoms may be in the α or β orientation, e.g. glucose, galactose, mannose, etc.

A number of lipids find use as L, including C8 to C30 fatty acids, long chain secondary alcohols, long chain amino alcohols, amides of fatty acids with long-chain di- or trihydroxy bases; and the like. For example, a glycosyl moiety (one or several units) may be linked to one hydroxyl group of a fatty alcohol or hydroxy fatty alcohol, or to a carbonyl group of a fatty acid. Non-limiting examples of suitable lipids include ceramides, sphingomyelin, cerebrosides, and the like, including sphiongosine, dihydrosphingosine, C20-dihydrosphingosine, phytosphingosine, C20-phytosphingosine, dehydrophytosphingosine, and sphingadienine.

In the invention, the glycolipid is a ceramide-like glycolipid, specifically, an α-galactosylceramide, also referred to herein as α-GalCer. Analogs thereof, such as α-C-GalCer, which contains a C-glycoside bond instead of an O-glycosidic linkage between the sugar and the sphingoid base are also disclosed.

Ceramides are N-acyl lipophilic derivatives of a long chain base or sphingoid, the commonest in animals being sphingosine and in plants phytosphingosine. Generally, the N-acyl lipophilic moiety is a fatty acid, which in some embodiments, comprise C3-C27 long carbon chains. These fatty acid carbon chains can be substituted, unsubstituted, saturated, unsaturated, branched, unbranched alkyl, alkenyl or alkynyl chains. As used herein, the term "ceramide" will include any of the ceramide analogs, synthetic or natural.

As used herein, the terms "ceramide-like glycolipid" and "glycolipid ceramide" are used interchangeably. A ceramide-like glycolipid is a glycolipid comprising an N-acyl lipophilic moiety and a sphingosine moiety or an analog thereof (e.g., a ceramide), and is represented by the general Formula (VII), wherein R2 of Formula (VII) can be a linear or branched, substituted or unsubstituted alkyl, alkenyl or alkynyl chain, or substituted or unsubstituted, saturated or unsaturated cyclic ring, or a substituted or unsubstituted aryl ring:

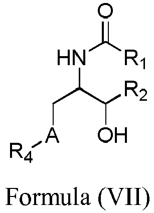

In this Formula (VII), R4 is a carbohydrate and the rest of the molecule is a ceramide or an analog thereof. In some aspects of the disclosure, R4 can be a saccharide. Ceramide-like glycolipids wherein the R4 is a saccharide is referred to herein as a glycosylceramide. The saccharide may be a hexose or pentose or analogs thereof. The hexose or pentose or analogs thereof can be mono-, di-, tri-, oligo or polysaccharide, or a derivative thereof. Ceramide-like glycolipids wherein the saccharide of R4 is a galactoside (α-or β-linked) are referred to herein as galactosylceramides. In the invention, R4 is an α-galactoside. In some aspects, A, if present can be -O-, -CH2- or - S; or is a single bond. In the invention, A is O.

R1 and R2 can be the same or different. In some embodiments R1, R2, or both can be a substituted or unsubstituted alkane, alkene, cycloalkyl; alkanes or alkenes substituted with heteroatoms; or substituted or unsubstituted aryls or heteroaryls. In some particular embodiments, R1, R2, or both can be substituted or unsubstituted alkyl or alkenyl chains with heteroatoms, cyclic rings, or aryl rings incorporated within the chain. In some embodiments R1, R2, or both contain contiguous or non-contiguous double bonds. These double bonds can be of E/Z configuration. In some embodiments, R2 is a saturated or unsaturated long chain C3-C27 alkyl or alkene group with at least one hydroxyl group attached therein.

In particular embodiments, R1 is a linear or branched C₁-C₂₇ alkane or C₂-C₂₇ alkene; or R1 is -C(OH)-R3 wherein R3 is a linear or branched C₁-C₂₆ alkane or C₂-C₂₆ alkene; or R1 is a C₆-C₂₇ alkane or alkene wherein (i) the C₆-C₂₇ alkane or alkene is substituted with a C₅-C₁₅ cycloalkane, C₅-C₁₅ cycloalkene, heterocycle, or aromatic ring or (ii) the C₆-C₂₇ alkane or alkene includes, within the C₆-C₂₇ alkyl or alkenyl chain, a C₅-C₁₅ cycloalkane, C₅-C₁₅ cycloalkene, heterocycle, or aromatic ring; or R1 is an optionally substituted aromatic ring, or an aralkyl.

In some of the above embodiments, R2 is a substituted or unsubstituted, branched or unbranched alkyl, alkenyl or alkynyl chain. In some particular embodiments, R2 is one of the following (a)-(e):
(a) -CH₂(CH₂)ₓCH₃,
(b) -CH(OH)(CH₂)ₓCH₃,
(c) -CH(OH)(CH₂)ₓCH(CH₃)₂,
(d) -CH=CH(CH₂)ₓCH₃,
(e) -CH(OH)(CH₂)ₓCH(CH₃)CH₂CH₃,
wherein x is an integer ranging from 0-40.

In some other embodiments, R2 is -CH(OH)(CH₂)ₓCH₃, wherein x is an integer ranging from 4-17. In some particular embodiments, R2 is -CH(OH)(CH₂)₁₃CH₃.

Non-limiting examples of ceramide-like glycolipids are described herein and also can be found, *e.g.,* in Porcelli, U.S. Patent Appl. Publ. No. 2006/0052316, Tsuji, U.S. Patent Appl. Publ. No. 2006/0211856, Jiang, U.S. Patent Appl. Publ. No. 2006/0116331, Hirokazu et al., U.S. Patent Appl. Publ. No. 2006/0074235, Tsuji et al., U.S. Patent Appl. Publ. No. 2005/0192248, Tsuji, U.S. Patent Application No. 2004/0127429, and Tsuji et al., U.S. Patent Application No. 2003/0157135.

A modified ceramide-like glycolipid of the invention comprises an α-galactosylceramide.

In some aspects of the disclosure, a glycosylceramide or analog thereof comprises Formula I: 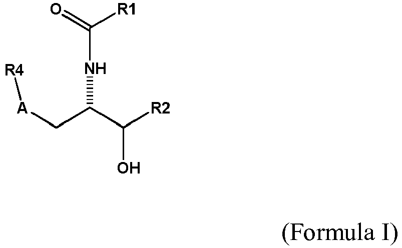 wherein R1 is a linear or branched C₁-C₂₇ alkane or C₂-C₂₇ alkene; or R1 is -C(OH)-R3 wherein R3 is a linear or branched C₁-C₂₆ alkane or C₂-C₂₆ alkene; or R1 is a C₆-C₂₇ alkane or alkene wherein (i) the C₆-C₂₇ alkane or alkene is substituted with a C₅-C₁₅ cycloalkane, C₅-C₁₅ cycloalkene, heterocycle, or aromatic ring or (ii) the C₆-C₂₇ alkane or alkene includes, within the C₆-C₂₇ alkyl or alkenyl chain, a C₅-C₁₅ cycloalkane, C₅-C₁₅ cycloalkene, heterocycle, or aromatic ring;
R2 is one of the following (a)-(e):
(a) -CH₂(CH₂)ₓCH₃,
(b) -CH(OH)(CH₂)ₓCH₃,
(c) -CH(OH)(CH₂)ₓCH(CH₃)₂,
(d) -CH=CH(CH₂)ₓCH₃,
(e) -CH(OH)(CH₂)ₓCH(CH₃)CH₂CH₃,
wherein X is an integer ranging from 4-17;
R4 is an α-linked or a β-linked monosaccharide, or when R1 is a linear or branched C₁-C₂₇ alkane, R4 is: 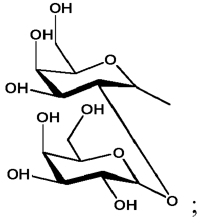 and
A is O or -CH₂.

In an embodiment of the invention, the α-galactosylceramide comprises Formula II: 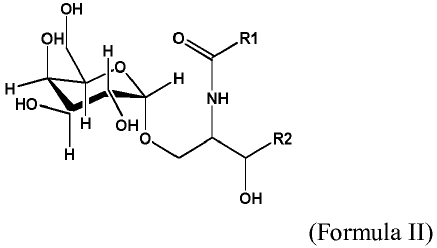 wherein
R1 is a linear or branched C₁-C₂₇ alkane or C₂-C₂₇ alkene; or R1 is -C(OH)-R3 wherein
R3 is linear or branched C₁-C₂₆ alkane or C₂-C₂₆ alkene; and
R2 is one of the following (a)-(e):
   (a) -CH₂(CH₂)ₓCH₃,
   (b) -CH(OH)(CH₂)ₓCH₃,
   (c) -CH(OH)(CH₂)ₓCH(CH₃)₂,
   (d) -CH=CH(CH₂)ₓCH₃,
   (e) -CH(OH)(CH₂)ₓCH(CH₃)CH₂CH₃,
wherein X is an integer ranging from 4-17.

In another embodiment, the modified ceramide-like glycolipid comprises an α-galactosylceramide comprising Formula III: 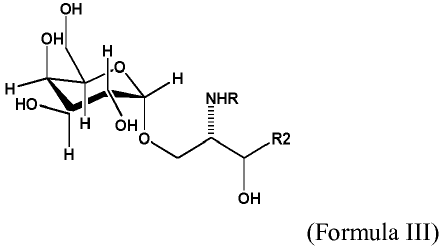 wherein R is -C(O)R1, wherein R1 is a linear or branched C₁-C₂₇ alkane or C₂-C₂₇ alkene; or R1 is -C(OH)-R3 wherein R3 is a linear or branched C₁-C₂₆ alkane or C₂-C₂₆ alkene; or R1 is a C₆-C₂₇ alkane or alkene wherein (i) the C₆-C₂₇ alkane or alkene is substituted with a C₅-C₁₅ cycloalkane, C₅-C₁₅ cycloalkene, heterocycle, or aromatic ring or (ii) the C₆-C₂₇ alkane or alkene includes, within the C₆-C₂₇ alkyl or alkenyl chain, a C₅-C₁₅ cycloalkane, C₅-C₁₅ cycloalkene, heterocycle, or aromatic ring; or R1 is an optionally substituted aromatic ring, or an aralkyl, and
R2 is one of the following (a)-(e):
(a) -CH₂(CH₂)ₓCH₃,
(b) -CH(OH)(CH₂)ₓCH₃,
(c) -CH(OH)(CH₂)ₓCH(CH₃)₂,
(d) -CH=CH(CH₂)ₓCH₃,
(e) -CH(OH)(CH₂)ₓCH(CH₃)CH₂CH₃,
wherein X is an integer ranging from 4-17.
In a further embodiment, R1 is selected from the group consisting of 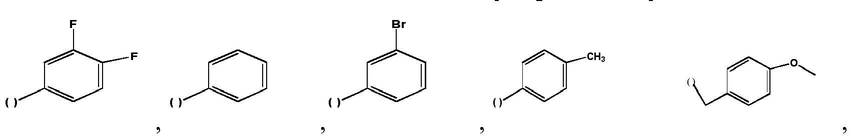 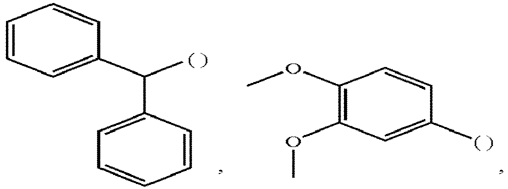 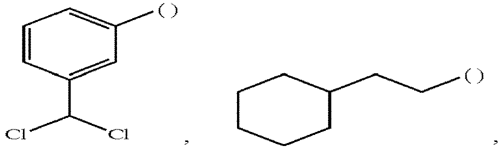 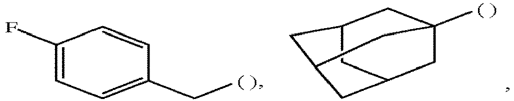 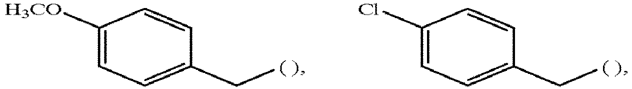 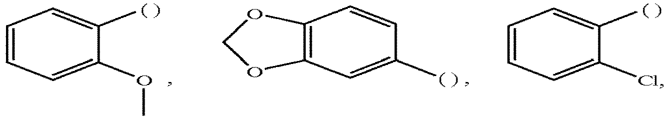 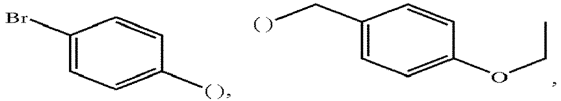 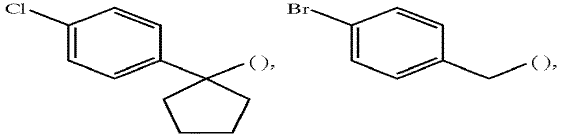 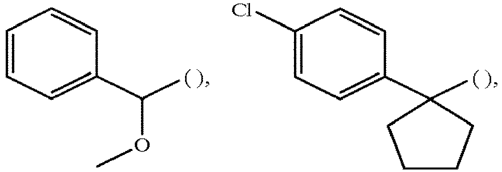 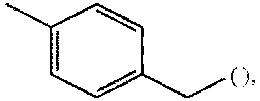 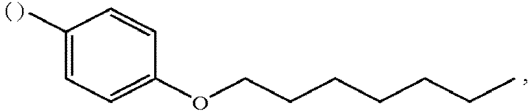 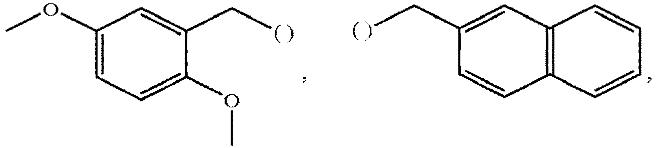 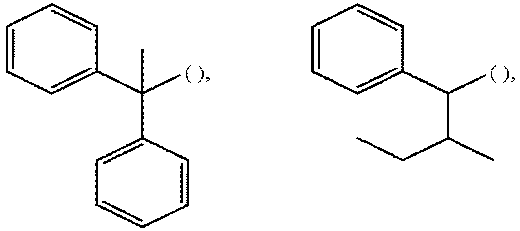 and 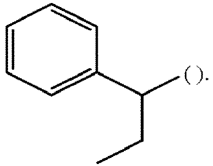 where ( ) represent the point of attachment of R1 to the compound of Formula III. In the invention, the R1 is substituted with a photoreactive group. The photoreactive group is attached to the R1 group at any appropriate position of the R1 group. For example, in some embodiments, the photoreactive group is attached to the phenyl ring of the R1 group. In some other embodiments, the photoreactive group is attached to the pendent substitutents of the phenyl ring.

In another embodiment, the α-galactosylceramide comprises (2S, 3S, 4R)-1-O-(α-D-galactopyranosyl)-N-hexacosanoyl-2-amino-1,3,4-octadecanetriol (KRN7000) or (2S,3S)-1-O-(α-D-galactopyranosyl)-N-hexacosanoyl-2-amino-1,3-octadecanediol).

In another embodiment, the α-galactosylceramide comprises (2S, 3S, 4R)-1-CH₂-(α-galactopyranosyl)-N-hexacosanoyl-2-amino-1,3,4-octadecanetriol (α-C-GalCer).

Other non-limiting examples of ceramide-like glycolipids are described in U.S. Patent No. 7,273,852; U.S. Patent No. 6,531,453; and U.S. Patent No. 5,936,076, U.S. Patent No. 7,772, 380.

"Modified glycolipids" as referred to herein include glycolipids that comprise a functional group that allows for a stable covalent linkage to a protein (e.g., CD1d). The term encompasses naturally-occurring glycolipids that have been modified through human intervention to comprise such a functional group and also refers to synthetic glycolipids.

The modified glycolipid of the present invention comprises a functional group that allows for a stable covalent linkage to a protein (e.g., CD1d) within or at the terminus of an acyl chain of the glycolipid. In the invention, the ceramide-like glycolipid is modified within or at the terminus of the N-acyl lipophilic moiety, such as an acyl chain.

In some aspects of the disclosure, a functional group that forms a stable covalent linkage to a protein such as CD1d, is a group that can be activated photochemically or thermally. In some aspects, such a functional group can be activated photochemically. As used herein, the term "photoreactive group" or "photoreactive functional group" refers to a functional group that is chemically inert, but becomes reactive when exposed to energy from a light source. The photoreactive group can become reactive when irradiated with visible light or in some embodiments, ultraviolet light.

In particular aspects, photoreactive groups that can be activated using an UV light are chosen because a compound possessing such a group (for example, a glycolipid with a photoreactive group) can be added to the protein (e.g., CD1d) and the timing of covalent bond formation can be controlled by UV irradiation at any given timepoint.

Any photoreactive functional group can be used in the present disclosure. In some aspects, the photoreactive groups that can be used to form a covalent bond between a glycolipid and the CD1d protein include any one of aryl azides, azidomethyl coumarins, benzophenones, anthraquinones, certain diazo compounds, diazirines and psoralen derivatives. Generally, a photoactivated reaction between a compound possessing a photoreactive functional group and CD1d protein can be illustrated as follows in Scheme 1.

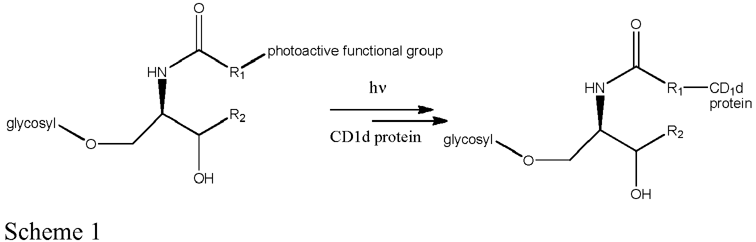

In some aspects, aryl azides and diazirines can be used as photoreactive functional groups. In some of the specific aspects, the following photoreactive functional groups can be used (Scheme 2)

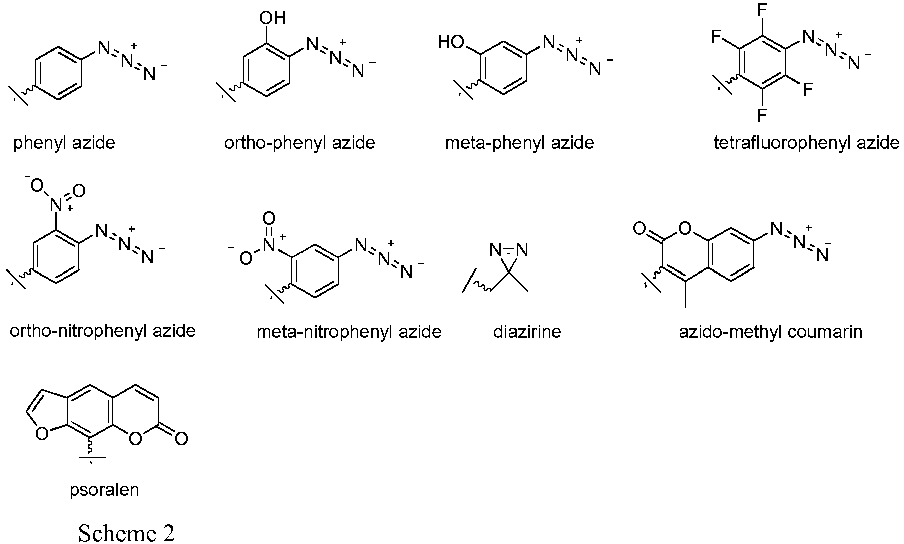

Squiggle bonds represent position of attachment of these photoreactive functional groups to the glycolipid, such as within or at the terminus of an acyl side chain of the glycolipid.

In some aspects, photoreactive functional groups that can be activated with long ultraviolet irradiation (e.g., 300 to 460 nm) are used in the presently disclosed methods and compositions.

In some aspects of the disclosure, the lipophilic group of a glycolipid is connected to the carbohydrate group via an acyl bond. The acyl bond could be either through an O or N or the like. In that respect, the "acyl side chain" is shown as below:

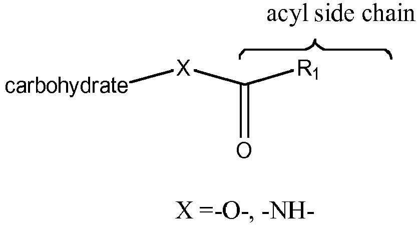

The acyl side chain can be linear or branched. In those embodiments wherein the acyl side chain is linear, when referring to the length of the linear acyl side chain (not including the functional group that allows for a stable covalent linkage to a protein), the carbonyl carbon is included. In some embodiments, the linear acyl side chain has about 5, 6, 7, 8, 9, 10, 11, 12, 13, 14, 15, 16, 17, 18, or more atoms (excluding the hydrogens), which can include one or more heteroatoms.

In some of these embodiments, the linear acyl side chain has at least 11 atoms (excluding the hydrogens), which can include one or more heteroatoms. In certain embodiments, the acyl side chain comprises about 11 to about 14 atoms (excluding the hydrogens).

In certain embodiments, the linear acyl side chain has one of the following structures: 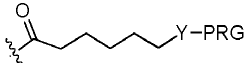 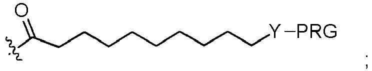 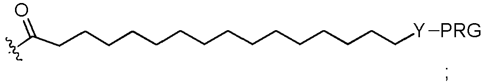 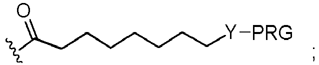 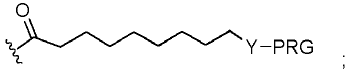 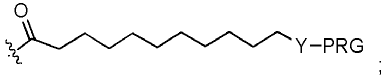 and 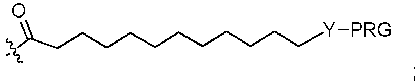 wherein Y if present is -O-, -CH₂-, -S-, -OCH₂-, -SCH₂-, -CH₂CH₂-; or a bond, and PRG is a photoreactive group.

In the invention, the glycolipid is a α-galactosylceramide. The photoreactive functional group is attached to the N-acyl lipophilic moiety. In particular embodiments, the photoreactive functional group is attached within or at the terminus of the fatty acid acyl side chain.

In the invention, the photoreactive functional group that is added to a glycolipid is a benzophenone group. The term "benzophenone" is well-understood by a person skilled in the art. Generally, benzophenones are represented by the general formula C₆H₅-CO-C₆H₅ as shown below.

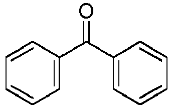

As used herein, benzophenones or analogs thereof can be used in certain aspects of the disclosure. For example, in some aspects isosteres of phenyl groups (i.e. thiophenes and the like) can be used in the place of phenyl groups. In some other aspects, the phenyl groups of analogs thereof can be substituted with other functional groups. In some other aspects, the phenyl groups and analogs thereof are unsubstituted.

Thus, the modified glycolipid of the present invention comprises a benzophenone group. The benzophenone group is covalently bound to the N-acyl lipophilic moiety. In some embodiments, the benzophenone group is found at the terminus of the acyl chain of a ceramide-like glycolipid. Non-limiting examples of said modified glycolipids are provided in Table 1.

**TABLE 1. Non-limiting examples of benzophenone-modified ceramide-like glycolipids.**

| **Compound** | **CHO group** | **Structure** |
|---|---|---|
| DB11-1 | α-D-Gal | 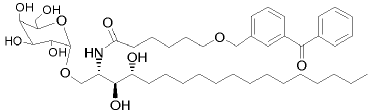 |
| DB11-2 | α-D-Gal | 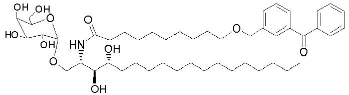 |
| DB11-3 | α-D-Gal | 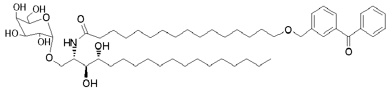 |
| DB12-6 | α-D-Gal | 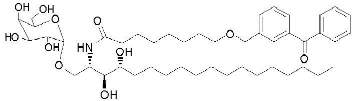 |
| DB12-7 | α-D-Gal | 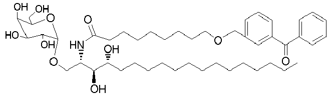 |
| DB12-8 | α-D-Gal | 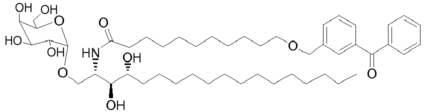 |
| DB12-9 | α-D-Gal | 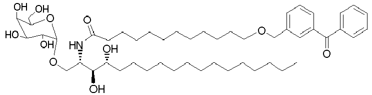 |

Thus, in the invention, R1 of Formula (VII) is substituted with a benzophenone moiety. In particular embodiments, the benzophenone moiety is substituted at the terminus. In some other embodiments, R1 of Formula (VII) is represented by the following Formula (VIII): 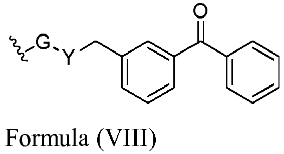 where Y is -O-, -CH₂-or -S-, and G is a linear or branched C₁-C₂₇ alkane or C₂-C₂₇ alkene; or G is -C(OH)-R3 wherein R3 is a linear or branched C₁-C₂₆ alkane or C₂-C₂₆ alkene; or R1 is a C₆-C₂₇ alkane or alkene wherein (i) the C₆-C₂₇ alkane or alkene is substituted with a C₅-C₁₅ cycloalkane, C₅-C₁₅ cycloalkene, heterocycle, or aromatic ring or (ii) the C₆-C₂₇ alkane or alkene includes, within the C₆-C₂₇ alkyl or alkenyl chain, a C₅-C₁₅ cycloalkane, C₅-C₁₅ cycloalkene, heterocycle, or aromatic ring; or R1 is an optionally substituted aromatic ring, or an aralkyl.

In some embodiments, R1 of Formula (VII) is represented by the following Formula (IX): 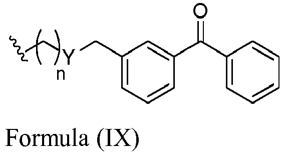 where Y is -O-, -CH₂-or -S-, and n is an integer ranging from 0-30. In some embodiments, n is an integer ranging from 3-17.

The presently disclosed modified glycolipids and modified glycolipid/protein complexes can be synthesized using any method known in the art, including those methods disclosed herein.

According to one embodiment of the invention, the general synthesis of benzophenone modified glycolipids is shown in Scheme 3. Accordingly, a benzophenone carboxylic acid derivative of Formula (IV) is activated with a suitable activating agent and reacted via peptide coupling with a ceramide-like glycolipid of Formula (V) to afford the desired benzophenone-modified glycolipids. Any activating agents, reagents and solvents known in the skill of art as suitable for a peptide coupling between an amine and a carboxylic acid can be used for the coupling reaction of benzophenone carboxylic acid derivative (IV) and the ceramide-like glycolipid (V).

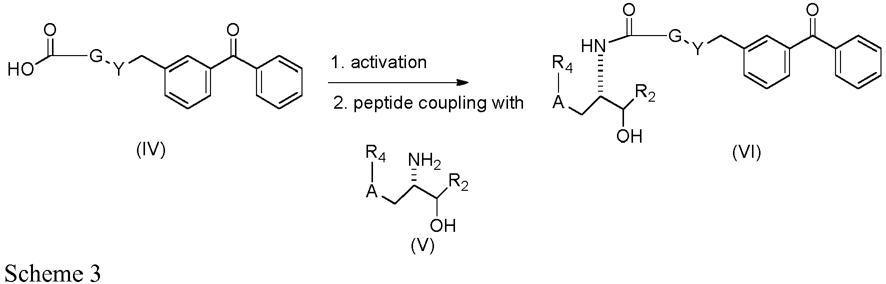

In some embodiments, Y is -O-, -CH₂-or -S-; G is a linear or branched C₁-C₂₇ alkane or C₂-C₂₇ alkene; or G is -C(OH)-R3 wherein R3 is a linear or branched C₁-C₂₆ alkane or C₂-C₂₆ alkene; or R1 is a C₆-C₂₇ alkane or alkene wherein (i) the C₆-C₂₇ alkane or alkene is substituted with a C₅-C₁₅ cycloalkane, C₅-C₁₅ cycloalkene, heterocycle, or aromatic ring or (ii) the C₆-C₂₇ alkane or alkene includes, within the C₆-C₂₇ alkyl or alkenyl chain, a C₅-C₁₅ cycloalkane, C₅-C₁₅ cycloalkene, heterocycle, or aromatic ring; R₄ is an α-galactosyl; A, if present, is -O-, -CH₂-; or is a single bond; and R₂ is substituted or unsubstituted C3-C40 alkyl, alkenyl or alkynyl chain. In some embodiments, G is -(CH₂)ₙ-, wherein n is an integer ranging from 0-20 and Y is O. In some other embodiments, R2 of the modified glycolipid comprising a benzophenone group of Formula (VI) is one of the following (a)-(e):
(a) -CH₂(CH₂)ₓCH₃,
(b) -CH(OH)(CH₂)ₓCH₃,
(c) -CH(OH)(CH₂)ₓCH(CH₃)₂,
(d) -CH=CH(CH₂)ₓCH₃,
(e) -CH(OH)(CH₂)ₓCH(CH₃)CH₂CH₃, wherein x is an integer ranging from 0-40.

In some of the other embodiments, R2 of the modified glycolipid comprising a benzophenone group of Formula (VI) is -CH(OH)(CH₂)ₓCH₃, wherein x is an integer ranging from 4-17. In some particular embodiments, R2 of the modified glycolipid comprising a benzophenone group of Formula (VI) is -CH(OH)(CH₂)₁₃CH₃. According to some embodiments of the invention, the benzophenone carboxylic acid derivative of Formula (IV) wherein G is -(CH₂)ₙ-, wherein n is an integer ranging from 0-20 and Y is O, can be synthesized according to the Schemes 4 and 5.

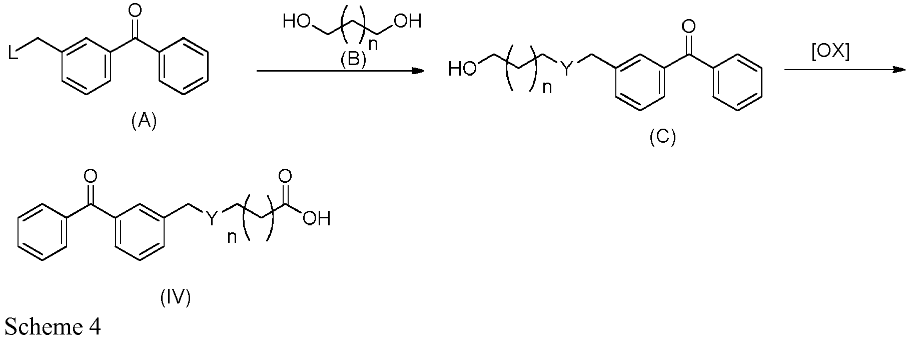

Accordingly, a benzophenone derivative (A) comprising a suitable leaving group is reacted with a diol derivative (B) to form the hydroxyl derivative (C). Any suitable leaving group L known in the skill of art can be used for this reaction. In some embodiments, the leaving group L can be Br, Cl, I, tosyl, mesyl and the like. In some particular embodiments, the leaving group L is bromine. The hydroxyl derivative is then oxidized to the carboxyl acid derivative (IV) wherein Y is O using any of the oxidization reagents suitable for oxidizing an alcohol to an acid. In particular, oxidizing agents that oxidize primary alcohol to acids are well known in the art. In some embodiments, the oxidizing agent is pyridinium dichromate (PDC).

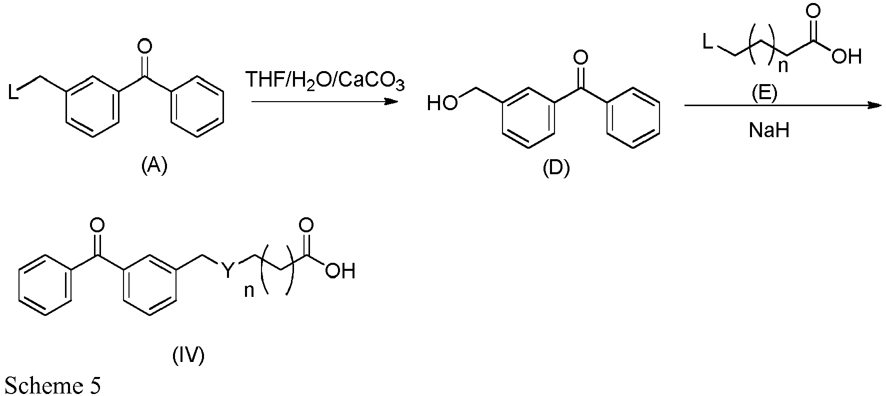

In some embodiments, the benzophenone carboxylic acid derivative of formula (V) wherein G is -(CH₂)ₙ-, wherein n is an integer ranging from 0-20 and Y is O can be synthesized as illustrated in Scheme 3. Accordingly, benzophenone derivative (A) is reacted with THF/H₂O in the presence of CaCO₃ to form the corresponding alcohol derivative (D). The alcohol derivative D is then reacted with a carboxylic acid derivative comprising a leaving group L under basic conditions to yield the benzophenone carboxylic acid derivative (IV) wherein Y is O. Any suitable leaving group L known in the skill of art can be used for this reaction. In some embodiments, the leaving group L can be Br, Cl, I, tosyl, mesyl and the like. In some particular embodiments, the leaving group L is bromine.

In a modified glycolipid/protein complex of the disclosure, a modified glycolipid is "physically associated" with a protein, *e.g.,* CD1d protein, to produce a "modified glycolipid/CD1d complex." In certain embodiments, the modified glycolipid/CD1d complex further comprises a heterologous antigen or a targeting molecule. By "physically associated" is meant a direct interaction with the CD1d protein. In the invention, the ceramide-like glycolipid is physically associated with a CD1d protein through covalent means. In some embodiments, the heterologous antigen or targeting molecule, or both are physically associated with a CD1d protein (e.g., through covalent means).

A modified glycolipid comprising a photoreactive group can be covalently bound to a protein (e.g., CD1d) by contacting the modified glycolipid with the protein and irradiating the modified glycolipid with light (e.g., visible light or ultraviolet light. When the photoreactive group is a benzophenone group, the modified glycolipid comprising the benzophenone group can be covalently bound to a protein (e.g., CD1d) by contacting the modified glycolipid with the protein and irradiating the benzophenone-modified glycolipid and the protein with ultraviolet light (i.e., light having a wavelength of about 10 nm-about 400 nm). In particular embodiments, the modified glycolipid is contacted with the protein by incubating the two molecules in a solution. In some of these embodiments, the solution comprises a buffered saline solution. In certain embodiments, the modified glycolipid and protein are irradiated with ultraviolet light with a long wavelength (i.e., about 300 nm to about 460 nm, including but not limited to about 300, 305, 310, 315, 320, 325, 330, 335, 340, 345, 350, 355, 360, 365, 370, 375, 380, 385, 390, 395, 400, 405, 410, 415, 420, 425, 430, 435, 440, 445, 450, 455, and 460 nm). In certain embodiments, the modified glycolipid and protein are irradiated with UV with a wavelength of about 365 nm. In some embodiments, the modified glycolipid and protein are irradiated using a UV lamp that is placed about 1 inch above the sample. In some embodiments, the modified glycolipid and protein are irradiated for about 5 minutes, about 10 minutes, about 15 minutes, about 30 minutes, about 40 minutes, about 50 minutes, about 1 hour, about 2 hours, or longer. In particular embodiments, the modified glycolipid and protein are irradiated for about 1 hour.

Detection of the modified glycolipid physically associated with the protein can be accomplished by methods known to one of skill in the art. By stably binding a modified glycolipid to a protein, *e.g.,* CD1d protein, a modified glycolipid/CD1d complex can be made. In certain embodiments, the compositions of the invention allow for simultaneous administration of a glycolipid and a protein, (CD1d protein) to an antigen presenting cell.

A modified glycolipid/protein complex of the present invention can comprise a single glycolipid, or can comprise heterogeneous mixtures of glycolipids. That is, a protein or multiple proteins can be physically associated with a single glycolipid or can be physically associated with a mixture of glycolipids.

The term "optionally substituted" as used herein means either unsubstituted or substituted with one or more substituents including halogen (F, Cl, Br, I), alkyl, substituted alkyl, aryl, substituted aryl, or alkoxy.

The term "alkyl", as used herein by itself or part of another group refers to a straight-chain or branched saturated aliphatic hydrocarbon typically having from one to eighteen carbons or the number of carbons designated. In one such embodiment, the alkyl is methyl. Non-limiting exemplary alkyl groups include ethyl, n-propyl, isopropyl, and the like.

The term "substituted alkyl" as used herein refers to an alkyl as defined above having one or more halogen (F, Cl, Br, I) substitutes.

The term "heterocycle" as used herein means a 3- to 10-membered monocyclic or bicyclic heterocyclic ring which is either saturated, unsaturated non-aromatic, or aromatic containing up to 4 heteroatoms. Each heteroatom is independently selected from nitrogen, which can be quaternized; oxygen; and sulfur, including sulfoxide and sulfone. The heterocycle can be attached via a nitrogen, sulfur, or carbon atom. Representative heterocycles include pyridyl, furyl, thiophenyl, pyrrolyl, oxazolyl, imidazolyl, thiazolyl, thiadiazolyl, isoxazolyl, pyrazolyl, isothiazolyl, pyridazinyl, pyrimidinyl, pyrazinyl, triazinyl, morpholinyl, pyrrolidinonyl, pyrrolidinyl, piperidinyl, piperazinyl, hydantoinyl, valerolactamyl, oxiranyl, oxetanyl, tetrahydrofuranyl, tetrahydropyranyl, tetrahydropyrindinyl, tetrahydropyrimidinyl, tetrahydrothiophenyl, tetrahydrothiopyranyl, quinolinyl, -isoquinolinyl, -chromonyl, -coumarinyl, -indolyl, - indolizinyl, -benzo[b]furanyl, -benzo[b]thiophenyl, -indazolyl, -purinyl, -4H-quinolizinyl, -isoquinolyl, -quinolyl, -phthalazinyl, -naphthyridinyl, -carbazolyl, and the like. The term heterocycle also includes heteroaryls.

The term "aryl" as used herein by itself or part of another group refers to monocyclic and bicyclic aromatic ring systems typically having from six to fourteen carbon atoms (*i.e*., C₆-C₁₄ aryl) such as phenyl, 1-naphthyl, and the like.

The term "substituted aryl" as used herein refers to an aryl as defined above having one or more substitutes including halogen (F, Cl, Br, I) or alkoxy.

The term "aralkyl" as used herein by itself or part of another group refers to an alkyl as defined above having one or more aryl substituents. Non-limiting exemplary aralkyl groups include benzyl, phenylethyl, diphenylmethyl, and the like.

The term "alkoxy" as used herein by itself or part of another group refers to an alkyl attached to a terminal oxygen atom. Non-limiting exemplary alkoxy groups include methoxy, ethoxy and the like.

The term "alkane" as used herein means a straight chain or branched non-cyclic saturated hydrocarbon. Representative straight chain alkane include -methyl, -ethyl, -n-propyl, -n-butyl, -n-pentyl, -n-hexyl, -n-heptyl, -n-octyl, -n-nonyl and -n-decyl. Representative branched alkane include -isopropyl, -sec-butyl, -isobutyl, -tert-butyl, - isopentyl, -neopentyl, 1-methylbutyl, 2-methylbutyl, 3-methylbutyl, 1,1-dimethylpropyl, 1,2-dimethylpropyl, 1-methylpentyl, 2-methylpentyl, 3-methylpentyl, 4-methylpentyl, 1-ethylbutyl, 2-ethylbutyl, 3-ethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-methylhexyl, 2-methylhexyl, 3-methylhexyl, 4-methylhexyl, 5-methylhexyl, 1,2-dimethylpentyl, 1,3-dimethylpentyl, 1,2-dimethylhexyl, 1,3-dimethylhexyl, 3,3-dimethylhexyl, 1,2-dimethylheptyl, 1,3-dimethylheptyl, and 3,3-dimethylheptyl.

The term "alkene" as used herein means a straight chain or branched non-cyclic hydrocarbon having at least one carbon-carbon double bond. Representative straight chain and branched alkene include -vinyl, -allyl, -1-butenyl, -2-butenyl, -isobutylenyl, - 1-pentenyl, -2-pentenyl, -3-methyl-1-butenyl, -2-methyl-2-butenyl, -2,3 -dimethyl-2-butenyl, -1-hexenyl, -2-hexenyl, -3-hexenyl, -1-heptenyl, -2-heptenyl, -3-heptenyl, -1 - octenyl, -2-octenyl, -3-octenyl, -1 -nonenyl, -2-nonenyl, -3-nonenyl, -1-decenyl, -2-decenyl, -3-decenyl and the like.

The term "cycloalkane" as used herein means a saturated cyclic hydrocarbon having from 3 to 15 carbon atoms. Representative cycloalkanes are cyclopropyl, cyclopentyl and the like.

The term "alkylcycloalkene" as used herein by itself or part of another group refers to an alkyl as defined above attached a cylcoalkane as defined above.

The term "cylcoalkene" as used herein means refers to a mono-cyclic non-aromatic hydrocarbon having at least one carbon-carbon double bond in the cyclic system and from 5 to 15 carbon atoms. Representative cycloalkenes include - cyclopentenyl, -cyclopentadienyl, -cyclohexenyl, -cyclohexadienyl, -cycloheptenyl, - cycloheptadienyl, -cycloheptatrienyl, -cyclooctenyl, -cyclooctadienyl, -cyclooctatrienyl, - cyclooctatetraenyl, -cyclononenyl -cyclononadienyl, -cyclodecenyl, -cyclodecadienyl and the like. The term "cycloalkene" also include bicycloalkenes and tricycloalkenes. The term "bicycloalkene" as used herein means a bicyclic hydrocarbon ring system having at least one carbon-carbon double bond in one of the rings and from 8 to 15 carbon atoms. Representative bicycloalkenes include, but are not limited to, -indenyl, - pentalenyl, -naphthalenyl, -azulenyl, -heptalenyl, -1,2,7,8-tetrahydronaphthalenyl, and the like. The term "tricycloalkene" as used herein, means a tri-cyclic hydrocarbon ring system having at least one carbon-carbon double bond in one of the rings and from 8 to 15 carbon atoms. Representative tricycloalkenes include, but are not limited to, - anthracenyl, -phenanthrenyl, -phenalenyl, and the like.

The term "aromatic ring" as used herein means a 5 to 14 membered aromatic carbocyclic ring, including both mono, bicyclic, and tricyclic ring systems. Representative aromatic rings are phenyl, napthyl, anthryl and phenanthryl.

The term "oxo" as used herein, means a double bond to oxygen. *i.e*., C=O.

The term "monosaccharide" as used herein means any of the simple sugars that serve as building blocks for carbohydrates. Examples of monosaccharides include glucose, fucose, galactose, and mannose.

The term "hydroxyl" as used herein refers to a functional group composed of one oxygen bonded to one hydrogen, with the oxygen covalently bonded to another atom, *e.g.,* a carbon. A "hydroxyl-protected glycolipid" of the invention includes a glycolipid, *e.g.,* a synthetic glycolipid, having a hydroxyl group coupled to a protection group.

The modified glycolipids of the invention have been modified to comprise a functional group that can form a stable covalent linkage to a protein (e.g., CD1d). When the protein is CD1d and the modified glycolipid is a ceramide-like glycolipid, a "stable modified ceramide-like glycolipid/CD1d complex" is one wherein the modified ceramide-like glycolipid is covalently bound to CD1d and the complex is able to retain significant Natural Killer T (NKT) cell-stimulating activity or the ceramide-like glycolipid remains associated with CD1d in a conformation detected by an antibody such as L363 which is specific for a biologically functional complex after an *in vitro* incubation (Yu et al. (2007) J Immunol Methods 323:11-23). In some embodiments, a stable modified ceramide-like glycolipid/CD1d complex retains at least about 50%, at least about 55%, at least about 60%, at least about 65%, at least about 70%, at least about 75%, at least about 80%, at least about 85%, at least about 95%, or more NKT cell-stimulating activity or functional complex detected by an antibody such as L363 after about a 1 day, about 2 days, about 3 days, about 4 days, about 5 days, about 6 days, about 1 week, or longer *in vitro* incubation. In some of these embodiments, the *in vitro* incubation occurs at room temperature. In particular embodiments, the *in vitro* incubation occurs in a buffered saline solution (e.g., PBS+0.05% Triton X-100). In certain embodiments, a stable modified ceramide-like glycolipid is covalently bound to CD1d and the complex is able to retain at least about 75% NKT cell-stimulating activity after an *in vitro* incubation at room temperature for about 2 or about 3 days.

The modified glycolipid is able to physically associate with a protein, *e.g*., CD1d, through covalent interactions. "Modified glycolipid complex" or "modified glycolipid/protein complex", as referred to herein include modified glycolipids that are physically associated with a protein, such as CD1d. As used herein, the term "physically associated" refers to an interaction between two molecules wherein the two molecules are bonded to each other directly or indirectly (e.g., via intervening molecules) either through covalent or non-covalent interactions (e.g., hydrogen bonds, ionic bonds, van der Waals forces, hydrophobic interactions). In the invention, the modified glycolipid/protein complex comprises a modified glycoprotein that is covalently bound to the protein through the functional group of the modified glycoprotein that allows for the covalent interaction (the benzophenone group). The modified glycolipids of the invention can be physically associated with (covalently bound to) any protein. In some embodiments, the modified glycolipid is physically associated with (covalently bound to) CD1d. A modified glycolipid/protein complex wherein the protein is CD1d is referred to herein as a "modified glycolipid/CD1d complex".

As used herein, the term "CD1d protein" encompasses a full-length CD1d protein, fragments, or variants thereof that are capable of binding to a glycolipid and β2-microglobulin or a fragment or variant thereof. The CD1d molecule is a member of the family of major histocompatibility complex antigen-like glycoproteins which associate with β₂-microglobulin and are expressed at the surface of cortical thymocytes, B cells, dendritic cells, Langerhans cells in the skin, and gastrointestinal epithelial cells. CD1d is mainly expressed on dendritic cells or epithelial cells of the gastrointestinal tract. The CD1 family members are involved in the presentation of glycolipids as antigens. In particular, CD1d regulates cytokine tone through activation of a distinct subset of T-lymphocytes, namely NK1 T cells which secrete IL-4 and INF-γ. All of the CD1 glycoproteins have been cloned and analyzed. For a detailed discussion of CD1 glycoproteins, and in particular CD1d, *see*, *e.g*., Balk et al., Proc. Natl. Acad. Sci. USA 86:252-256 (1989); Kojo et al., Biochem. Biophy. Res. Comm. 276:107-111 (2000); Kojo et al., J. Rheumatology 30:2524-2528 (2003); Kang and Cresswell, Nature Immunology 5:175-181 (2004); Im et al., J. Biol. Chem. 279:299-310 (2004); Dutronc and Porcelli, Tissue Antigens 60:337-353 (2002).

Full-length CD1d consists of a signal sequence, an extracellular domain, a transmembrane domain and a cytoplasmic domain. The full-length CD1d polypeptide is 335 amino acids in length.

The human CD1d sequence is known in the art and has the accession number NP_001757 in Genbank, which is set forth in SEQ ID NO: 1.

A variant of human CD1d includes, but is not limited to, a polypeptide with the following mutation:T64S.

The sequence of mouse CD1d can be found on Genbank with the following accession number: NP_031665. The sequence of rat CD1d can be found on Genbank with the following accession number: NP058775. The sequence of sheep CD1d can be found on Genbank with the following accession numbers: O62848 and Q29422. The sequence of chimpanzee CD1d can be found on Genbank with the following accession number: NP_001065272. The sequence of rabbit CD1d can be found on Genbank with the following accession number: P23043.

The extracellular domain of CD1d consists of three domains: the α1 domain, the α2 domain, and the α3 domain. The α1 and α2 domains comprise the antigen binding sites. The α3 domain includes a β₂-microglobulin association site.

The CD1d domain designations used herein are defined as in Table 2.

**Table 2. CD1d domains**

| **Domain** | **CD1d (human)** |
|---|---|
| Signal Seq. | 1-19 |
| Extracellular | 20-301 |
| α1 domain | 20-108 |
| α2 domain | 109-201 |
| α3 domain | 202-295 |
| Transmembrane | 302-322 |
| Cytoplasmic | 323-335 |

As one of skill in the art will appreciate, the beginning and ending residues of the domains listed above may vary depending upon the computer modeling program used or the method used for determining the domain.

Some embodiments of the invention provide a modified glycolipid/CD1d complex, which comprises a soluble CD1d polypeptide. Table 1 above describes the various domains of the CD1d polypeptide. Soluble CD1d polypeptides generally comprise a portion or all of the extracellular domain of the polypeptides, including the α1, α2, and α3 domains. Soluble CD1d polypeptides generally lack some or all of the transmembrane domain and cytoplasmic domain. As one of skill in the art would appreciate, the entire extracellular domain of CD1d may comprise additional or fewer amino acids on either the C-terminal or N-terminal end of the extracellular domain polypeptide.

CD1d polypeptides for use in the methods and compositions of the present invention include, but are not limited to, a CD1d polypeptide comprising, consisting essentially of, or consisting of an amino acid sequence identical to a reference amino acid sequence, except for up to twenty amino acid substitutions. In some embodiments, the reference amino acid sequence is selected from the group consisting of amino acids a to 295 of SEQ ID NO:1, amino acids 21 to b of SEQ ID NO:1, and a to b of SEQ ID NO:1, wherein a is any integer from 1 to 100, and b is any integer from 201 to 301, and wherein the CD1d polypeptide associates with β₂-microglobulin and binds a ceramide-like glycolipid. In one embodiment, the soluble CD1d polypeptide comprises amino acids 21 to 295 of SEQ ID NO:1. In another embodiment, the soluble CD1d polypeptide comprises amino acids 20-295, 20-296, 20-297, 20-298, 20-299, 20-300 and 20 to 301 of SEQ ID NO:1.

For the purposes of the presently disclosed methods and compositions, in those embodiments wherein the modified glycolipid/protein complex comprises a CD1d protein, the complex further comprises a β2-microglobulin physically associated (e.g., covalently bound) with the CD1d protein. In some of these embodiments, the β2-microglobulin is covalently linked to the amino terminus of CD1d.

In certain embodiments, a CD1d complex of the invention comprises a β₂-microglobulin polypeptide, which associates with a soluble CD1d polypeptide or polypeptide fragment. β₂-microglobulin is present on the surface of all nucleated cells as the small extracellular subunit of the major histocompatibility complex (MHC) class I molecule and actively participates in the immune response. For a detailed discussion of β₂-microglobulin, *see, e.g.,* Peterson et al., Adv. Cancer Res. 24:115-163 (1977); Sege et al., Biochemistry 20:4523-4530 (1981).

Full-length β₂-microglobulin is a secreted protein which comprises a signal sequence and Ig-like domain. The full-length CD1d polypeptide is 119 amino acids in length.

The human β₂-microglobulin sequence is known in the art and has the accession number NP_004039 in Genbank, which is set forth herein as SEQ ID NO: 2.

Variants of human β₂-microglobulin include, but are not limited to, polypeptides with one or more of the following mutations: A20G, P52Q, S55V, and Y86YS.

The sequence of mouse β₂-microglobulin can be found on Genbank with the following accession number: NP_033865. The sequence of pig β₂-microglobulin can be found on Genbank with the following accession number: NP_999143. The sequence of rat β₂-microglobulin can be found on Genbank with the following accession number: NP_036644. The sequence of chimpanzee β₂-microglobulin can be found on Genbank with the following accession number: NP_001009066. The sequence of rabbit β₂-microglobulin can be found on Genbank with the following accession number:P01885. The sequence of sheep β₂-microglobulin can be found on Genbank with the following accession number: NP_001009284.

The β₂-microglobulin domain designations used herein are defined as in Table 3.

**Table 3. β₂-microglobulin domains**

| **Domain** | **β₂-microglobulin (human)** |
|---|---|
| Signal Seq. | 1-20 |
| β₂-microglobulin | 21-119 |
| Ig domain | 25-113 22-116 |

As one of skill in the art will appreciate, the beginning and ending residues of the domains listed above may vary depending upon the computer modeling program used or the method used for determining the domain.

Modified glycolipid/CD1d complexes of the invention may comprise fragments, variants, or derivative thereof of a β₂-microglobulin polypeptide. Table 3 above describes the various domains of the β₂-microglobulin polypeptide. β₂-microglobulin polypeptides generally comprise a portion or all of the secreted portion of the polypeptides.

Human β₂-microglobulin polypeptides for use in the methods of the present invention include, but are not limited to, a β₂-microglobulin polypeptide comprising, consisting essentially of, or consisting of an amino acid sequence identical to a reference amino acid sequence (e.g., SEQ ID NO: 2), except for up to twenty amino acid substitutions. In some embodiments, the reference amino acid sequence is selected from the group consisting of amino acids a to 119 of SEQ ID NO:2, amino acids 21 to b of SEQ ID NO: 2, and a to b of SEQ ID NO:2,wherein a is any integer from 15 to 25, and b is any integer from 100 to 119, wherein said β₂-microglobulin polypeptide associates with CD1d and supports binding of ceramide-like glycolipids. In one embodiment, the β₂-microglobulin polypeptide comprises amino acids 21 to 113 of SEQ ID NO:2. In one embodiment, the β₂-microglobulin polypeptide comprises amino acids 21 to 119 of SEQ ID NO:2.

By "a reference amino acid sequence" is meant the specified sequence without the introduction of any amino acid substitutions. As one of ordinary skill in the art would understand, if there are no substitutions, the "isolated polypeptide" of the invention comprises an amino acid sequence which is identical to the reference amino acid sequence.

CD1d or β2-microglobulin polypeptides described herein may have various alterations such as substitutions, insertions or deletions. Exemplary amino acids that can be substituted in the polypeptide include amino acids with basic side chains (*e.g*., lysine, arginine, histidine), acidic side chains (*e.g*., aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g*., threonine, valine, isoleucine) and aromatic side chains (*e.g*., tyrosine, phenylalanine, tryptophan, histidine).

Corresponding fragments of CD1d or β2-microglobulin polypeptides at least 70%, 75%, 80%, 85%, 90%, or 95% identical to the polypeptides and reference polypeptides described herein are also contemplated.

As known in the art, "sequence identity" between two polypeptides is determined by comparing the amino acid sequence of one polypeptide to the sequence of a second polypeptide. When discussed herein, whether any particular polypeptide is at least about 70%, 75%, 80%, 85%, 90% or 95% identical to another polypeptide can be determined using methods and computer programs/software known in the art such as, but not limited to, the BESTFIT program (Wisconsin Sequence Analysis Package, Version 8 for Unix, Genetics Computer Group, University Research Park, 575 Science Drive, Madison, WI 53711). BESTFIT uses the local homology algorithm of Smith and Waterman, Advances in Applied Mathematics 2:482-489 (1981), to find the best segment of homology between two sequences. When using BESTFIT or any other sequence alignment program to determine whether a particular sequence is, for example, 95% identical to a reference sequence according to the present invention, the parameters are set, of course, such that the percentage of identity is calculated over the full length of the reference polypeptide sequence and that gaps in homology of up to 5% of the total number of amino acids in the reference sequence are allowed.

A soluble CD1d polypeptide may contain some or all of the amino acids from the transmembrane domain, provided that the polypeptide is still capable of remaining soluble in an aqueous, *e.g*., a physiological solution. Preferably, not more than about 20, 19, 18, 17, 16, 15, 14, 13, 12, 11, 10, 9, 8, 7, 6, 5, 4, 3, 2 or 1, and preferably none of the amino acids of the transmembrane domain will be included.

Additionally, fragments of β₂-microglobulin are useful in the present invention. To be useful in the present invention, the fragment of β₂-microglobulin would have to retain the ability to associate with the CD1d molecule. Preferably, not more than about 20, 19, 18, 17, 16, 15, 14, 13, 12, 11, 10, 9, 8, 7, 6, 5, 4, 3, 2 or 1, and preferably none of the amino acids of β₂-microglobulin will be deleted.

One may wish to introduce a small number of amino acids at the polypeptide termini of either the soluble CD1d polypeptide or the β₂-microglobulin polypeptide, usually not more than 20, 19, 18, 17, 16, 15, 14, 13, 12, 11, 10, 9, 8, 7, 6, 5, 4, 3, 2 or 1. The deletion or insertion of amino acids will usually be as a result of the needs of the construction, providing for convenient restriction sites, addition of processing signals, ease of manipulation, improvement in levels of expression, or the like. In addition, one may wish to substitute one or more amino acids with a different amino acid for similar reasons, usually not substituting more than about 10, 9, 8, 7, 6, 5, 4, 3, 2 or 1 amino acids in any one domain.

The CD1d and β2-microglobulin may be autologous to any mammalian or avian species, for example, primates (esp. humans), rodents, rabbits, equines, bovines, canines, felines, etc. β2-microglobulin is typically not inflammatory *in vivo.* However, it is preferable to employ β2-microglobulin derived from the same species as is to be administered the modified glycolipid/CD1d complex so as to reduce the risk of a xenogeneic immune response.

The soluble CD1d polypeptide and β₂-microglobulin polypeptide may be separately produced and allowed to associate to form a stable heteroduplex complex, or both of the subunits may be expressed in a single cell.

Soluble CD1d polypeptides and β₂-microglobulin polypeptides for use in the methods and compositions of the present invention may be isolated from a multiplicity of cells, *e.g*., transformed cell lines JY, BM92, WIN, MOC, and MG, and CHO using a variety of techniques known to those skilled in the art. It is also possible to construct a functional fusion molecule in which the carboxyl terminus of β₂-microglobulin or a fragment thereof is connected through a linker to the amino terminus of CD1d or a fragment thereof.

Additionally, the amino acid sequences of CD1d and β2-microglobulin from a variety of species are known, and the polynucleotides encoding these polypeptides have been cloned, therefore, the polypeptides can be made using recombinant methods. The coding regions for the CD1d and β₂ microglobulin chains or their fusion products are inserted into expression vectors, expressed separately in an appropriate host, such as E. *coli,* yeast, insect cells, mammalian cells or other suitable cells.

The term "vaccine" refers to a composition, which when administered to an animal is useful in stimulating an immune response, *e.g.,* against an antigen, *e.g.,* a heterologous antigen. As used herein, a "heterologous" antigen is an antigen that originates from a foreign species, or, if from the same species, is substantially modified from its native form in composition by deliberate human intervention.

In certain embodiments, a modified glycolipid complex may be administered with a heterologous antigen or a targeting molecule. In other embodiments, a modified glycolipid/protein complex of the invention is used as an "antigen carrier" for the delivery of a heterologous antigen, *e.g*., an immunogenic polypeptide, or a targeting molecule. For example, a modified glycolipid/protein complex can be used as an antigen carrier for the delivery of antigens from another pathogen (*e.g.,* bacterial (*e.g., Salmonella, Listeria, Bacillus anthracis,* and *Shigella* antigens), fungal, parasitic (*e.g.,* a malarial antigen from *Plasmodium*), or viral antigens (*e.g.,* a viral antigen from HIV, SIV, HPV, RSV, influenza or hepatitis (HAV, HBV, and HCV)) or tumor specific antigens.

Compositions of the invention include cells (e.g., viral, fungal, bacterial cells) physically associated with a modified glycolipid. As described in U.S. Application Publication No. 2010/0183549, glycolipids, such as ceramide-like glycolipids can be physically associated with bacterial cells by culturing the bacterial cells with the glycolipid. Thus, in some embodiments, the modified glycolipid can be physically associated with a bacterial cell by first culturing the bacterial cell with a modified glycolipid comprising a photoreactive group to allow for association of the modified glycolipid with the cell wall of the bacterial cell, followed by irradiation with a light source (e.g., ultraviolet light) to allow for covalent linkage of the modified glycolipid to the surface of the bacterial cell.

In some embodiments, the modified glycolipid/protein complexes of the invention comprise a targeting molecule (e.g., an antibody, including but not limited to a monoclonal, chimeric, humanized, human, bifunctional antibody, or antigen-binding fragment thereof, such as, for example, Fab and F(ab')2 portions and Fv fragments, and single-chain antibodies) that serves to target the complex to a particular cell, tissue, organ, or region of a subject's body. In some of these embodiments, the targeting molecule is an antibody or an antigen-binding fragment that specifically binds to a target antigen, such as a cell surface marker, as taught in U.S. Application Publication No. 2006/0269540. The protein (CD1d) may be directly linked or fused to the targeting molecule (e.g., antibody or antigen-binding fragment thereof), either directly or through a linker sequence or molecule. In certain embodiments, the protein (CD1d) is linked to the targeting molecule (e.g., antibody or fragment thereof) through a multivalent compound.

The targeting molecule can either be linked to the CD1d molecule or the β2 microglobulin. In those embodiments wherein the targeting molecule is an antibody, the protein (e.g., CD1d or β2 microglobulin) may be linked to either the light chain or the heavy chain of the antibody. As taught by International Application Publication No. WO 9964597 it is possible to introduce mutations into β2-microglobulin that increase affinity for the class I heavy chain so as to facilitate assembly and increase stability of the fusion protein.

The protein (e.g., CD1d or β2 microglobulin) may be linked to either the carboxyl or amino terminus of the antibody, or it may be linked to the antibody at a site other than the carboxyl or amino termini. Wherein CD1d is the protein within the modified glycolipid/protein complex, the antibody or antigen-binding fragment thereof may be linked to the carboxyl terminus of CD1d. In some of these embodiments, the amino terminus of a single chain Fv fragment (scFv) is linked to the carboxyl terminus of CD1d.

In one embodiment, the targeting molecule (e.g., antibody or antigen-binding fragment thereof) is specific for a cell surface marker of a tumor cell. In some embodiments of the present invention, the cancer is a head and neck cancer, gastric cancer, esophageal cancer, stomach cancer, colorectal cancer, colon carcinoma, cancer of liver and intrahepatic bile ducts, pancreatic cancer, lung cancer, small cell lung cancer, cancer of the larynx, breast cancer, malignant melanoma, multiple myeloma, sarcomas, rhabdomyosarcoma, lymphomas, follicular non-Hodgkin-lymphoma, leukemias, T- and B-cell-leukemias, Hodgkin-lymphoma, B-cell lymphoma, ovarian cancer, cancer of the uterus, cervical cancer, prostate cancer, genital cancer, renal cancer, cancer of the testis, thyroid cancer, bladder cancer, plasmacytoma or brain cancer.

Tumor associated antigens comprise pan-carcinoma antigens like CEA (Sundblad Hum. Pathol. 27, (1996) 297-301, llantzis Lab. Invest. 76(1997), 703-16), EGFR type I (Nouri, Int. J. Mol. Med. 6 (2000), 495-500) and EpCAM (17-1A/KSA/GA733-2, Balzar J. Mol. Med. 77 (1999), 699-712). EGFR type I is especially overexpressed in glioma and EpCAM in colon carcinoma. EGFR type II (Her-2/neu, ERBB2 Sugano Int. J. Cancer 89 (2000), 329-36) and TAG-72 glycoprotein (sTN antigen, Kathan Arch. Pathol. Lab. Med. 124 (2000), 234-9) are upregulated in breast cancer. EGFR deletion neoepitope might also play a role as tumor associated antigen (Sampson Proc. Natl. Acad. Sci. U S A 97 (2000), 7503-8). The antigens A33 (Ritter Biochem. Biophys. Res. Commun. 236 (1997), 682-6), Lewis-Y (DiCarlo Oncol. Rep. 8 (2001), 387-92), Cora Antigen (CEA-related Cell Adhesion Molecule CEACAM 6, CD66c, NCA-90, Kinugasa Int. J. Cancer 76 (1998), 148-53) and MUC-1 (Mucin) are associated with colon carcinoma (lida Oncol. Res. 10 (1998), 407-14). Thomsen-Friedenreich-antigen (TF, Gal1B-3GaINAca1-0-Thr/Ser) is not only found in colon carcinoma (Baldus Cancer 82 (1998), 1019-27) but also in breast cancer (Glinsky Cancer. Res. 60 (2000), 2584-8). Overexpression of Ly-6 (Eshel J. Biol. Chem. 275 (2000), 12833-40) and desmoglein 4 in head and neck cancer and of E-cadherin neoepitope in gastric carcinoma has been described (Fukudome Int. J. Cancer 88 (2000), 579-83). Prostate-specific membrane antigen (PSMA, Lapidus Prostate 45 (2000), 350-4), prostate stem cell antigen (PSCA, Gu Oncogene 191 (2000) 288-96) and STEAP (Hubert, Proc Natl Acad Sci U S A 96 (1999), 14523-8) are associated with prostate cancer. The alpha and gamma subunit of the fetal type acetylcholine receptor (AChR) are specific immunohistochemical markers for rhabdomyosarcoma (RMS, Gattenlohner Diagn. Mol. Pathol. 3 (1998), 129-34).

Association of CD20 with follicular non-Hodgkin lymphoma (Yatabe Blood 95 (2000), 2253-61, Vose Oncology (Huntingt) 2 (2001) 141-7), of CD19 with B-cell lymphoma (Kroft Am. J. Clin. Pathol. 115 (2001), 385-95), of Wue-1 plasma cell antigen with multiple myeloma (Greiner Virchows Arch 437 (2000), 372-9), of CD22 with B cell leukemia (dArena Am. J. Hematol. 64 (2000), 275-81), of CD7 with T-cell leukemia (Porwit-MacDonald Leukemia 14 (2000), 816-25) and CD25 with certain T and B cell leukemias has been described (Wu Arch. Pathol. Lab. Med. 124 (2000), 1710-3). CD30 is associated with Hodgkin-lymphoma (Mir Blood 96 (2000), 4307-12). Expression of melanoma chondroitin sulfate proteoglycan (MCSP, Eisenmann Nat. Cell. Biol. 8 (1999), 507-13) and ganglioside GD3 is observed in melanoma (Welte Exp Dermatol 2 (1997), 64-9), while GD3 is also found in small cell lung cancer (SCLC, Brezicka Lung Cancer 1 (2000), 29-36). Expression of ganglioside GD2 is, also upregulated in SCLC and in neuroblastoma (Cheresh et al. Cancer Res. 10 (1986), 5112-8). Ovarian carcinoma is associated with Muellerian Inhibitory Substance (MIS) receptor type II (Masiakos Clin. Cancer Res. 11 (1999), 3488-99) and renal as well as cervical carcinoma with expression of carboanhydrase 9 (MN/CAIX, Grabmaier lnt. J. Cancer 85 (2000) 865-70). Elevated expression levels of CA 19-9 were found in pancreatic cancer (Nazli Hepatogastroenterology 47 (2000), 1750-2).

Other examples of tumor cell surface antigens are Her2/neu, expressed in breast and ovarian carcinomas (Zhang, H. et al., Experimental & Molecular Pathology 67:15-25 (1999)); CM-1, expressed in breast cancer (Chen, L. et al., Acta Academiae Medicinae Sinicae 19(2):150-3); 28K2, expressed in lung adenocarcinoma and large cell carcinoma (Yoshinari, K. et al., Lung Cancer 25:95-103 (1999)); E48 and U 36 expressed in head and neck squamous cell carcinoma (Van Dongen, G.A.M.S. et al., Anticancer Res. 16:2409-14 (1996)); NY-ESO-1, expressed in esophageal carcinoma, and melanoma Jager, E. et al., J. Exp. Med. 187:265-70 (1998); Jager, E. et al., International J. Cancer 84:506-10 (1999)); KU-BL 1-5, expressed in bladder carcinoma (Ito, K. et al., AUA 2000 Annual Meeting, Abstract 3291 (2000)); NY CO 1-48, expressed in colon carcinoma (Scanlan, M.J. et al., International J. Cancer 76:652-8 (1998)); HOM MEL 40, expressed in melanoma (Tureci, O. et al., Cancer Res. 56:4766-72 (1996)); OV569, expressed in ovarian carcinoma (Scholler, N. et al., Proc. Natl. Acad. Sci. USA 96:11531-6 (1999)); ChCE7, expressed in neuroblastoma and renal cell carcinoma (Meli, M.L. et al., International J. Cancer 83:401-8 (1999)); CA19-9, expressed in colon carcinoma (Han, J.S. et al., Cancer 76:195-200 (1995)); CA125, expressed in ovarian carcinoma (O'Brien, T.J. et al., International J. Biological Markers 13:188-95 (1998)); and Gangliosides (GM2, GD2, 9-o-acetyl-GD3, GD3), expressed in melanoma and neuroblastoma (Zhang, S. et al., Cancer Immunol. Immunotherapy 40:88-94 (1995)).

In particular embodiments of the presently disclosed methods, the tumor-associated antigen is selected from the group consisting of Lewis Y, CEA, Muc-1, erbB-2, -3 and -4, Ep-CAM, E-cadherin neoepitope, EGF-receptor (e.g. EGFR type I or EGFR type II), EGFR deletion neoepitope, CA19-9, Muc-1, LeY, TF-, Tn- and sTn-antigen, TAG-72, PSMA, STEAP, Cora antigen, CD7, CD19 and CD20, CD22, CD25, Ig-a and Ig-B, A33 and G250, CD30, MCSP and gp100, CD44-v6, MT-MMPS, (MIS) receptor type II, carboanhydrase 9, F19-antigen, Ly6, desmoglein 4, PSCA, Wue-1, TM4SF-antigens (CD63, L6, CO-29, SAS), the alpha and gamma subunit of the fetal type acetylcholine receptor (AChR), CM-1, 28K2, E48, U36, NY-ESO-1, KU-BL 1-5, NY CO 1-48, HOM MEL 40, OV569, ChCE7, CA19-9, CA125, GM2, GD2, 9-o-acetyl-GD3, and GD3.

In another embodiment, the targeting molecule (e.g., antibody or antigen-binding fragment thereof) is specific for a cell surface marker of CD1d-restricted NKT cells. Non-limiting examples of suitable NKT cell markers to which to target specific antibodies are CD161, CD56, or (for NKT subsets) CCR4 on CD4+ CD1d-restricted NKT or CCR1 or CCR6 on double negative (CD4 and CD8 negative) CD1d-restricted NKT. This is particularly useful in treating diseases or symptoms which are the result of low NKT activity such as cancer or infection. In addition, since NKT cell activity is differentially modulated depending on the particular ceramide-like glycolipid bound to CD1d (see, *e.g.,* US Patent No. 7,772,380,), compositions of the present disclosure include aspects with selective association of CD1d with these functionally different ceramide-like glycolipids and the related α-glycosylceramides which may modulate diseases which are a result of high or deleterious NKT activity (e.g. excessive Th1 activity) such as myasthenia gravis (Reinhardt et al. Neurology 52:1485-87,1999), psoriasis (Bonish , J. Immunol. 165:4076-85,2000), ulcerative colitis (Saubermann et al. Gastroenterology 119:119-128, 2000), and primary biliary cirrhosis (Kita et al. Gastroenterology 123:1031-43,2002).

In another embodiment, the targeting molecule (e.g., antibody or antigen-binding fragment thereof) is specific for a cell surface marker of a target tissue of autoimmune disease or inflammatory response. In a preferred embodiment, the modified glycolipid/CD1d complex is targeted to such sites by coupling the complex to a targeting molecule (e.g., an antibody or antigen binding-fragment thereof) specific for a local tissue antigen. The modified glycolipid/CD1d complex concentrated on local tissue cells will then lead to recruitment and activation of CD1d-restricted NKT cells and trigger a cascade of events that regulate the autoimmune or inflammatory response. The relevant specific tissue antigens may be different for different autoimmune and inflammatory diseases.

In the case of demyelinating diseases including, most especially, multiple sclerosis, the targeting molecule (e.g., antibody or antigen-binding fragment thereof) is specific for MBP (myelin basic protein), PLP (myelin proteolipid protein), or MOG (myelin oligodendrocyte glycoprotein). In particular embodiments, the targeting molecule (e.g., antibody or antigen-binding fragment thereof) is specific for MOG.

In the case of juvenile onset type I diabetes which follows from destruction of insulin-producing pancreatic islet beta cells, the targeting molecule (e.g., antibody or antigen-binding fragment thereof) is specific for target antigens of the islet beta cells such as GT3 ganglioside, IGRP (islet-specific glucose-6-phosphatase related protein), or SUR1 (Proks P et al., Diabetes:51 Suppl 3:S368-76, 2002). Recently, peri-islet Schwann cells have been described as an early target of autoimmune destruction in this disease (Winer S et al: Nature Medicine 9:198-205, 2003). In another preferred embodiment of the invention, therefore, the targeting molecule (e.g., antibody or antigen-binding fragment thereof) is specific for Schwann cell specific antigens glial fibrillary acidic protein (GFAP), and S100beta for treatment or diagnosis of juvenile onset type I diabetes.

In other embodiments of the presently disclosed methods for treatment of autoimmune and inflammatory diseases, conjugates comprising modified glycolipid/CD1d complexes and targeting molecules (e.g., antibodies or antigen-binding fragment thereof) specific for type II collagen are injected into affected joints for treatment of rheumatoid arthritis; targeting molecules specific for thyroglobulin or TSH receptor are targeted to the thyroid for treatment of Hashimoto's thyroiditis and Graves' disease; and targeting molecules specific for the K+/H+ ATPase are employed for treatment of pernicious anemia or atrophic gastritis (targeting the gastric parietal cells).

In other embodiments, the targeting molecule (e.g., antibody or antigen-binding fragment thereof) is specific for a cell surface marker of an infected cell or tissue. In a preferred embodiment, a modified glycolipid/CD1d complex is targeted to the site of infection by coupling the complex to a targeting molecule (e.g., antibody or antigen-binding fragment thereof) specific for an antigen encoded by the infectious agent. The relevant antigens are different in the case of different infectious agents. In particular embodiments of the presently disclosed methods, the surface marker for an infected cell is selected from the group consisting of viral envelope antigens, e.g. of human retroviruses (HTLV I and II, HIV1 and 2) or human herpes viruses (HSV1 and 2, CMV, EBV), haemagglutinin e.g. of influenza virus (influenza A, B or C), glycoproteins E1 and E2 from rubella virus or RGP of rabies virus.

In other embodiments, the targeting molecule is specific for antigens encoded by other infectious viruses, bacteria, fungi, protozoa or helminthes.

In one embodiment, the targeting molecule (e.g., antibody or antigen-binding fragment thereof) is specific for a cell surface marker of a professional antigen presenting cell. In some of these embodiments, the targeting molecule is specific for a cell surface marker of a dendritic cell, for example, CD83, DEC205, CMRF-44 (Fearnley DB et al. Blood 89:3708-16,1997), CMRF-56 (Hock BD et al. Tissue Antigens 53:320-34,1999), BDCA-1, BDCA-2, BDCA-3, and BDCA-4 (Dzionek A et al. J. Immunol. 165:6037-6046,2000). In other embodiments, the targeting molecule is specific for markers of antigen presenting cells including Toll-like receptors (TLR1, TLR2, TLR3, TLR4, TLR5, TLR7, TLR9) mannose receptor, and mannan-binding lectin (MBL); as well as additional markers specific to dendritic cells, including, DC-SIGN (the C-type lectin, non-integrin, ICAM-3 receptor on DC), ALCAM, DC-LAMP, and any of a number of other receptors for apoptotic cells including phosphatidylserine receptor. The targeting molecule may be specific for a cell surface marker of another professional antigen presenting cell, such as a B cell or a macrophage. CD19, CD20 and CD22 are expressed on B cells, and other markers have been described for other antigen presenting cells.

In other embodiments, the targeting molecule (e.g., antibody or antigen-binding fragment thereof) is specific for a cell surface marker of a dendritic cell subset. In a particular embodiment of the invention, a modified glycolipid/CD1d complex is targeted to DC by coupling the complex to a targeting molecule specific for a surface antigen marker of DC such as CD83, DEC205, CMRF-44, CMRF-56, DC-SIGN, Toll-like Receptors (TLR) including TLR1, TLR2, TLR3, TLR4, TLR5, TLR7, TLR9, mannose receptor, mannan-binding lectin (MBL), ALCAM, DC-LAMP, phosphatidylserine receptor, BDCA-1, BDCA-2, BDCA-3 or BDCA-4 (neuropilin, Tordjman R et al. Nature Immunol. 3:477-82,2002).

The term "antigen" and the related term "antigenic" as used herein refer to a substance that binds specifically to an antibody or to a T-cell receptor.

The term "immunogen" and the related term "immunogenic" as used herein refer to the ability to induce an immune response, including an antibody and/or a cellular immune response in an animal, for example a mammal. It is likely that an immunogen will also be antigenic, but an "antigen," because of its size or conformation, may not necessarily be an "immunogen." An "immunogenic composition" induces an immune response in a subject, *e*.*g*., antibodies that specifically recognize one or more antigens, contained within that "immunogenic composition." In certain embodiments, the immunogenic composition of the invention comprises a modified glycolipid/CD1d complex and a heterologous antigen or a targeting molecule.

The term "immune response" is meant to include an activity of cells of the immune system in response to an antigen or immunogen. Such activities include, but are not limited to production of antibodies, cytotoxicity, lymphocyte proliferation, release of cytokines, inflammation, phagocytosis, antigen presentation, and the like. An immune response which is highly specific to a given antigen or immunogen, *e*.*g*., production of specific antibodies or production of specific T lymphocytes is referred to herein as an "adaptive immune response." An immune response which is not specific to a given antigen, *e*.*g*., release of cytokines by NK and NKT cells, is referred to herein as an "innate immune response." Examples of immune responses include an antibody response or a cellular, *e*.*g*., cytotoxic T-cell or NKT cell, response.

The terms "protective immune response" or "therapeutic immune response" refer to an immune response to an immunogen, which in some way prevents or at least partially arrests disease symptoms, side effects or progression. By "protective" is meant that the immune response is induced in a subject animal which has not contracted a disease, where the immune response alleviates, reduces, moderates or, in some cases fully prevents disease symptoms if the animal later contracts or is susceptible to that disease, *e.g.,* exposure to *M. tuberculosis.* By "therapeutic" is meant that the immune response is induced in a subject animal which has the disease, *e.g.,* a human with tuberculosis, where the immune response alleviates, reduces, moderates, or in some cases fully eliminates disease symptoms. In certain embodiments, the compositions of the invention are used to induce a therapeutic immune response in an animal, *e*.*g*., a human, having an infectious disease or cancer or an autoimmune or inflammatory disease.

The term "modulating an immune response" is meant to refer to any way in which a given immune response is increased, decreased, or changed by a composition or treatment relative to the immune response without that composition or treatment. For example, use of an adjuvant, *e*.*g*., a modified glycolipid/CD1d complex comprising a heterologous antigen or a targeting molecule, to increase an immune response to an antigen, *e*.*g*., the heterologous antigen, is considered modulation of that immune response. Decrease in an immune response, *e*.*g*., prevention of autoimmunity, is also a modulation. In addition, changing an immune response, *e*.*g*., from a primary TH2 response to a primary TH1 response or vice versa, is a modulation of an immune response. The present disclosure provides methods of modulating an immune response by administering to an animal a composition which comprises a modified glycolipid/protein complex (e.g., modified glycolipid/CD1d complex) and a heterologous antigen or a targeting molecule.

The present invention provides compositions useful for enhancing both primary and secondary immune responses. In one aspect, the primary and/or secondary immune response is against a heterologous antigen or a targeting molecule that is administered together with, prior to, or soon after the modified glycolipid/protein complex of the invention (modified glycolipid/CD1d complex). In one aspect, the modified glycolipid/protein complex is used as an antigen carrier for delivery of antigens, *e*.*g*., heterologous antigens or targeting molecules that enhance an immune response against an infectious agent or tumor.

The term "adjuvant" refers to a material having the ability to (1) alter or increase the immune response to a particular antigen or (2) increase or aid an effect of a pharmacological agent. In certain embodiments, a ceramide-like glycolipid functions as an adjuvant upon simultaneous administration with a CD1d protein. In certain embodiments, the ceramide-like glycolipid, (αGalCer), functions as an adjuvant when administered with a CD1d protein and heterologous antigen or a targeting molecule. In another embodiment, a second adjuvant is included. Other suitable adjuvants include, but are not limited to, LPS derivatives (*e*.*g*., monophosphoryl lipid A (MPL)), TLR9 agonists (*e.g.,* CPG ODNS), TLR7/8 agonists (*e.g.,* imiquimod), cytokines and growth factors; bacterial components (*e*.*g*., endotoxins, in particular superantigens, exotoxins and cell wall components); aluminum-based salts; calcium-based salts; silica; polynucleotides; toxoids; serum proteins, viruses and virally-derived materials, poisons, venoms, imidazoquiniline compounds, poloxamers, and cationic lipids.

A great variety of materials have been shown to have adjuvant activity through a variety of mechanisms. Any compound which can increase the expression, antigenicity or immunogenicity of an immunogen is a potential adjuvant. Other potential adjuvants of the invention include, but are not limited to: glycolipids; chemokines; compounds that induces the production of cytokines and chemokines; interferons; inert carriers, such as alum, bentonite, latex, and acrylic particles; pluronic block polymers, such as TiterMax® (block copolymer CRL-8941, squalene (a metabolizable oil) and a microparticulate silica stabilizer); depot formers, such as Freunds adjuvant; surface active materials, such as saponin, lysolecithin, retinal, Quil A, liposomes, and pluronic polymer formulations; macrophage stimulators, such as bacterial lipopolysaccharide; alternate pathway complement activators, such as insulin, zymosan, endotoxin, and levamisole; non-ionic surfactants; poly(oxyethylene)-poly(oxypropylene) tri-block copolymers; mLT; MF59™; SAF; Ribi™ adjuvant system; trehalose dimycolate (TDM); cell wall skeleton (CWS); Detox™; QS21; Stimulon™; complete Freund's adjuvant; incomplete Freund's adjuvant; macrophage colony stimulating factor (M-CSF); tumor necrosis factor (TNF); 3-O-deacylated MPL; CpG oligonucleotides; polyoxyethylene ethers, polyoxyethylene esters, and combinations of more than one adjuvant.

In certain embodiments, the adjuvant is a cytokine. A composition of the present invention can comprise one or more cytokines, chemokines, or compounds that induce the production of cytokines and chemokines. Examples include, but are not limited to granulocyte macrophage colony stimulating factor (GM-CSF), granulocyte colony stimulating factor (G-CSF), macrophage colony stimulating factor (M-CSF), colony stimulating factor (CSF), erythropoietin (EPO), interleukin 2 (IL-2), interleukin-3 (IL-3), interleukin 4 (IL-4), interleukin 5 (IL-5), interleukin 6 (IL-6), interleukin 7 (IL-7), interleukin 8 (IL-8), interleukin 10 (IL-10), interleukin 12 (IL-12), interleukin 15 (IL-15), interleukin 18 (IL-18), interferon alpha (IFNα), interferon beta (IFNβ), interferon gamma (IFNγ), interferon omega (IFNω), interferon tau (IFNτ), interferon gamma inducing factor I (IGIF), transforming growth factor beta (TGF-β), RANTES (regulated upon activation, normal T-cell expressed and presumably secreted), macrophage inflammatory proteins (*e.g.,* MIP-1 alpha and MIP-1 beta), *Leishmania* elongation initiating factor (LEIF), and Flt-3 ligand.

In certain embodiments, compositions of the invention further comprise another component, *e*.*g*., a polypeptide with immunological activity. For example, the protein with immunological activity is a costimulatory molecule, such as a toll-like receptor ("TLR"), B7.1 or B7.2. "B7" is used herein to generically refer to either B7.1 or B7.2. A costimulatory molecule, *e*.*g*., the extracellular domain of B7-1 (CD80) or B7-2 (CD86) that interacts with CD28 on T- and NK-cells can be administered as an amino terminal fusion to β2-microglobulin incorporated into the structure of a soluble CD1d complex for use in the present invention. See, *e.g.,* WO 9964597, published 16 Dec 1999. In certain embodiments, incorporation of a costimulatory molecule, *e.g.,* a B7 signaling molecule, with the compositions of the invention allows more effective and prolonged activation of NKT cells by a modified glycolipid/CD1d complex of the invention.

In other embodiments, the compositions of the invention further comprise additional adjuvant components, *e*.*g*., any of the adjuvants described above, such as, LPS derivatives (*e.g.,* MPL), TLR9 agonists (*e.g.,* CPG ODNS), TLR7/8 agonists (*e.g.,* imiquimod), cytokines and growth factors; bacterial components (*e*.*g*., endotoxins, in particular superantigens, exotoxins and cell wall components); aluminum-based salts; calcium-based salts; silica; polynucleotides; toxoids; serum proteins, viruses and virally-derived materials, poisons, venoms, imidazoquiniline compounds, poloxamers, cationic lipids, and Toll-like receptor (TLR) agonists. Examples of TLR agonist adjuvants which can be effective, include, but are not limited to: N-acetylmuramyl-L-alanine-D-isoglutamine (MDP), lipopolysaccharides (LPS), genetically modified and/or degraded LPS, alum, glucan, colony stimulating factors (e.g., EPO, GM-CSF, G-CSF, M-CSF, PEGylated G-CSF, SCF, IL-3, IL6, PIXY 321), interferons (e.g., γ-interferon, α-interferon), interleukins (e.g IL-1, IL-2, IL-3, IL-4, IL-5, IL-6, IL-10, IL-12, IL-15, IL-18), saponins (e.g., QS21), monophosphoryl lipid A (MPL), 3 De-O-acylated monophosphoryl lipid A (3D-MPL), unmethylated CpG sequences, 1-methyl tryptophan, arginase inhibitors, cyclophosphamide, antibodies that block immunosuppressive functions (e.g., anti-CTLA4 antibodies), lipids (such as palmitic acid residues), tripalmitoyl-S-glycerylcystein lyseryl-serine (P₃ CSS), and Freund's adjuvant. Alternatively or additionally, compositions of the present invention may further comprise a lymphokine or cytokine that modulates immune cell activation such as transforming growth factor (TGF, *e.g.,* TGFα and TGFβ); α interferons (*e.g.* IFNα); β interferons (*e.g.* IFNβ); γ interferons (*e.g*. IFNγ) or lymphocyte function-associated protein, such as LFA-1 or LFA-3; or an intercellular adhesion molecule, such as ICAM-1 or ICAM-2.

Other adjuvant examples include compounds such as isatoribin and it derivatives (Anadys Pharmaceuticals) or imidazoquinolinamines, such as imiquimod and resiquimod (Dockrell & Kinghom, J. Antimicrob. Chemother., 48:751-755 (2001) and Hemmi et al., Nat. Immunol., 3:196-200 (2002), guanine ribonucleosides, such as C8-substituted or N7, C-8-disubstituted guanine ribonucleosides (Lee et al., Proc. Natl. Acad. Sci. USA, 100:6646-6651 (2003) and the compounds that are disclosed in Pat. Pub. Nos. JP-2005-089,334; WO99/32122; WO98/01448 WO05/092893; and WO05/092892, and TLR-7 agonist SM360320 (9-benzyl-8-hydroxy-2-(2-methoxy-ethoxy)adenine) disclosed in Lee et al., Proc Natl Acad Sci USA, 103(6):1828-1833 (2006).

In addition to isatoribin, other TLR agonist adjuvants include 9-benzyl-8-hydroxy-2-(2-methoxyethoxy)adenine (SM360320) and Actilon™ (Coley Pharmaceutical Group, Inc.). Other adjuvants which can be used in conjunction with the composition of the present invention are disclosed in PCT Pub. No. WO 2005/000348, U.S. Pat. Pub. No. 2007/0292418, and U.S. Pat. Pub. No. 2007/0287664.

Compositions of the invention can further comprise an immunogenic polypeptide. In certain embodiments, a modified glycolipid/protein complex of the invention (modified glycolipid/CD1d complex) can be used as antigen carriers for the delivery of heterologous antigens, targeting molecules or immunogens. Heterologous antigens, targeting molecules or immunogens can include, but are not limited to, immunogenic polypeptides.

An "immunogenic polypeptide" is meant to encompass antigenic or immunogenic polypeptides, *e*.*g*., poly-amino acid materials having epitopes or combinations of epitopes. As used herein, an immunogenic polypeptide is a polypeptide which, when introduced into a vertebrate, reacts with the immune system molecules of the vertebrate, *i.e.,* is antigenic, and/or induces an immune response in the vertebrate, *i*.*e*., is immunogenic. It is likely that an immunogenic polypeptide will also be antigenic, but an antigenic polypeptide, because of its size or conformation, may not necessarily be immunogenic. Examples of antigenic and immunogenic polypeptides include, but are not limited to, polypeptides from infectious agents such as bacteria, viruses, parasites, or fungi, allergens such as those from pet dander, plants, dust, and other environmental sources, as well as certain self-polypeptides, for example, tumor-associated antigens.

Modified glycolipid complexes of the invention comprising antigenic or immunogenic polypeptides can be used to prevent or treat, *e.g.,* cure, ameliorate, lessen the severity of, or prevent or reduce contagion of viral, bacterial, fungal, and parasitic infectious diseases, as well as to treat allergies and proliferative diseases such as cancer.

In addition, modified glycolipid/protein complexes of the invention comprising antigenic and immunogenic polypeptides can be used to prevent or treat, *e.g.,* cure, ameliorate, or lessen the severity of cancer including, but not limited to, cancers of oral cavity and pharynx (*e.g.,* tongue, mouth, pharynx), digestive system (*e.g.,* esophagus, stomach, small intestine, colon, rectum, anus, anal canal, anorectum, liver, gallbladder, pancreas), respiratory system (*e*.*g*., larynx, lung), bones, joints, soft tissues (including heart), skin, melanoma, breast, reproductive organs (*e*.*g*., cervix, endometrium, ovary, vulva, vagina, prostate, testis, penis), urinary system (*e*.*g*., urinary bladder, kidney, ureter, and other urinary organs), eye, brain, endocrine system (*e*.*g*., thyroid and other endocrine), lymphoma (*e*.*g*., Hodgkin's disease, non-Hodgkin's lymphoma), multiple myeloma, leukemia (*e*.*g*., acute lymphocytic leukemia, chronic lymphocytic leukemia, acute myeloid leukemia, chronic myeloid leukemia).

Modified glycolipid/protein complexes of the invention comprising antigenic and immunogenic polypeptides can be used to prevent or treat, *e.g.,* cure, ameliorate, or lessen the severity of a disease caused by an infectious agent, *e*.*g*., viral, bacterial, fungal, and parasitic agents.

Examples of viral antigenic and immunogenic polypeptides include, but are not limited to, adenovirus polypeptides, alphavirus polypeptides, calicivirus polypeptides, *e.g.,* a calicivirus capsid antigen, coronavirus polypeptides, distemper virus polypeptides, Ebola virus polypeptides, enterovirus polypeptides, flavivirus polypeptides, hepatitis virus (AE) polypeptides, *e*.*g*., a hepatitis B core or surface antigen, herpes virus polypeptides, *e*.*g*., a herpes simplex virus or varicella zoster virus glycoprotein, immunodeficiency virus polypeptides, *e*.*g*., the human immunodeficiency virus envelope or protease, infectious peritonitis virus polypeptides, influenza virus polypeptides, *e*.*g*., an influenza A hemagglutinin, neuraminidase, or nucleoprotein, leukemia virus polypeptides, Marburg virus polypeptides, orthomyxovirus polypeptides, papilloma virus polypeptides, parainfluenza virus polypeptides, *e*.*g*., the hemagglutinin/neuraminidase, paramyxovirus polypeptides, parvovirus polypeptides, pestivirus polypeptides, picorna virus polypeptides, *e.g*., a poliovirus capsid polypeptide, pox virus polypeptides, *e.g.,* a vaccinia virus polypeptide, rabies virus polypeptides, *e*.*g*., a rabies virus glycoprotein G, reovirus polypeptides, retrovirus polypeptides, and rotavirus polypeptides.

Examples of bacterial antigenic and immunogenic polypeptides include, but are not limited to, *Actinomyces* polypeptides, *Bacillus* polypeptides, *e.g.,* immunogenic polypeptides from *Bacillus anthracis, Bacteroides* polypeptides, *Bordetella* polypeptides, *Bartonella* polypeptides, *Borrelia* polypeptides, *e.g., B. burgdorferi* OspA, *Brucella* polypeptides, *Campylobacter* polypeptides, *Capnocytophaga* polypeptides, *Chlamydia* polypeptides, *Clostridium* polypeptides, *Corynebacterium* polypeptides, *Coxiella* polypeptides, *Dermatophilus* polypeptides, *Enterococcus* polypeptides, *Ehrlichia* polypeptides, *Escherichia* polypeptides, *Francisella* polypeptides, *Fusobacterium* polypeptides, *Haemobartonella* polypeptides, *Haemophilus* polypeptides, *e.g., H. influenzae* type b outer membrane protein, *Helicobacter* polypeptides, *Klebsiella* polypeptides, L form bacteria polypeptides, *Leptospira* polypeptides, *Listeria* polypeptides, *Mycobacteria* polypeptides, *Mycoplasma* polypeptides, *Neisseria* polypeptides, *Neorickettsia* polypeptides, Nocardia polypeptides, *Pasteurella* polypeptides, *Peptococcus* polypeptides, *Peptostreptococcus* polypeptides, *Pneumococcus* polypeptides, *Proteus* polypeptides, *Pseudomonas* polypeptides, *Rickettsia* polypeptides, *Rochalimaea* polypeptides, *Salmonella* polypeptides, *Shigella* polypeptides, *Staphylococcus* polypeptides, *Streptococcus* polypeptides, *e.g., S. pyogenes* M proteins, *Treponema* polypeptides, and *Yersinia* polypeptides, *e.g*., *Y. pestis* F1 and V antigens.

Examples of parasitic antigenic and immunogenic polypeptides include, but are not limited to *Balantidium coli* polypeptides, *Entamoeba histolytica* polypeptides, *Fasciola hepatica* polypeptides, *Giardia lamblia* polypeptides, *Leishmania* polypeptides, and *Plasmodium* polypeptides (*e.g*., *Plasmodium falciparum* polypeptides).

Examples of fungal antigenic and immunogenic polypeptides include, but are not limited to, *Aspergillus* polypeptides, *Candida* polypeptides, *Coccidiodes immitis* or *C*. *posadasii* polypeptides, *Cryptococcus* polypeptides, *Histoplasma* polypeptides, *Pneumocystis* polypeptides, and *Paracoccidiodes* polypeptides.

Examples of tumor-associated antigenic and immunogenic polypeptides include, but are not limited to, tumor-specific immunoglobulin variable regions, GM2, Tn, sTn, Thompson-Friedenreich antigen (TF), Globo H, Le(y), MUC1, MUC2, MUC3, MUC4, MUC5AC, MUC5B, MUC7, carcinoembryonic antigens, beta chain of human chorionic gonadotropin (hCG beta), C35, HER2/neu, CD20, PSMA, EGFRvIII, KSA, PSA, PSCA, GP100, MAGE 1, MAGE 2, TRP 1, TRP 2, tyrosinase, MART-1, PAP, CEA, BAGE, MAGE, RAGE, and related proteins.

Compositions of the invention can further comprise other therapeutic agents. Examples of therapeutic agents include, but are not limited to, antimetabolites, alkylating agents, anthracyclines, antibiotics, and anti-mitotic agents. Antimetabolites include methotrexate, 6-mercaptopurine, 6-thioguanine, cytarabine, 5-fluorouracil decarbazine. Alkylating agents include mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin. Anthracyclines include daunorubicin (formerly daunomycin) and doxorubicin (also referred to herein as adriamycin). Additional examples include mitozantrone and bisantrene. Antibiotics include dactinomycin (formerly actinomycin), bleomycin, mithramycin, and anthramycin (AMC). Antimitotic agents include vincristine and vinblastine (which are commonly referred to as vinca alkaloids). Other cytotoxic agents include procarbazine, hydroxyurea, asparaginase, corticosteroids, mitotane (O,P'-(DDD)), interferons. Further examples of cytotoxic agents include, but are not limited to, ricin, doxorubicin, taxol, cytochalasin B, gramicidin D, ethidium bromide, etoposide, tenoposide, colchicin, dihydroxy anthracin dione, 1-dehydrotestosterone, and glucocorticoid. Analogs and homologs of such therapeutic agents are encompassed by the present invention.

A modified glycolipid/protein complex of the invention, (a benzophenone modified glycolipid/CD1d complex) or a composition or a vaccine composition comprising the same can be labeled, so as to be directly detectable, or can be used in conjunction with secondary labeled immunoreagents which will specifically bind the compound, *e*.*g*., for detection or diagnostic purposes. Labels of interest can include dyes, enzymes, chemiluminescers, particles, radioisotopes, or other directly or indirectly detectable agent. Alternatively, a second stage label can be used, *e*.*g*. labeled antibody directed to one of the constituents of the compound of the invention.

Examples of suitable enzyme labels include, but are not limited to malate dehydrogenase, staphylococcal nuclease, delta-5-steroid isomerase, yeast-alcohol dehydrogenase, alpha-glycerol phosphate dehydrogenase, triose phosphate isomerase, peroxidase, alkaline phosphatase, asparaginase, glucose oxidase, beta-galactosidase, ribonuclease, urease, catalase, glucose-6-phosphate dehydrogenase, glucoamylase, and acetylcholine esterase.

Examples of suitable radioisotopic labels include ³H, ¹¹¹In , ¹²⁵I, ¹³¹I, ³²P, ³⁵S , ¹⁴C, ⁵¹Cr, ⁵⁷To, ⁵⁸Co, ⁵⁹Fe, ⁷⁵Se, ¹⁵²Eu, ⁹⁰Y, ⁶⁷Cu, ²¹⁷Ci, ²¹¹At, ²¹²Pb, ⁴⁷Sc, ¹⁰⁹Pd, etc. Examples of suitable non-radioactive isotopic labels include ¹⁵⁷Gd, ⁵⁵Mn, ¹⁶²Dy, ⁵²Tr, and ⁵⁶Fe.

Examples of suitable fluorescent labels include an ¹⁵²Eu label, a fluorescein label, an isothiocyanate label, a rhodamine label, a phycoerythrin label, a phycocyanin label, an allophycocyanin label, an o-phthaldehyde label, and a fluorescamine label.

Examples of chemiluminescent labels include a luminal label, an isoluminal label, an aromatic acridinium ester label, an imidazole label, an acridinium salt label, an oxalate ester label, a luciferin label, a luciferase label, and an aequorin label.

Examples of nuclear magnetic resonance contrasting agents include heavy metal nuclei such as Gd, Mn, and Fe.

Typical techniques for binding the above-described labels to glycolipids or polypeptides of the invention are provided by Kennedy et al., Clin. Chim. Acta 70:1-31 (1976), and Schurs et al., Clin. Chim. Acta 81:1-40 (1977). Coupling techniques mentioned in the latter are the glutaraldehyde method, the periodate method, the dimaleimide method, the m-maleimidobenzyl-N-hydroxy-succinimide ester method, all of which methods are incorporated by reference herein.

The ability of a composition of the present invention to modulate an immune response can be readily determined by an *in vitro* assay. NKT cells for use in the assays include transformed NKT cell lines, or NKT cells which are isolated from a mammal, *e.g.,* from a human or from a rodent such as a mouse. NKT cells can be isolated from a mammal by sorting cells that bind CD1d:a-GalCer tetramers. See, for example, Benlagha et al., J Exp Med 191:1895-1903 (2000); Matsuda et al., J Exp Med 192:741-754 (2000); and Karadimitris et al., Proc Natl Acad Sci USA 98:3294-3298 (2001). A suitable assay to determine if a compound or composition of the present invention is capable of modulating the activity of NKT cells is conducted by co-culturing NKT cells and antigen presenting cells, adding the particular compound or composition of interest to the culture medium that targets either the antigen presenting cells or the NKT cells directly, and measuring IL-4 or IFN-γ production. A significant increase or decrease in IL-4 or IFN-γ production over the same co-culture of cells in the absence of the compound or composition of the invention indicates stimulation or inhibition of NKT cells.

The NKT cells employed in the assays are incubated under conditions suitable for proliferation. For example, an NKT cell hybridoma is suitably incubated at about 37°C and 5% CO2 in complete culture medium (RPMI 1640 supplemented with 10% FBS, penicillin/streptomycin, L-glutamine and 5x10⁻⁵ M 2-mercaptoethanol). Serial dilutions of the compound can be added to the NKT cell culture medium. Suitable concentrations of the compound added to the NKT cells typically will be in the range of from 10⁻¹² to 10⁻⁶ M. Use of antigen dose and APC numbers giving slightly submaximal NKT cell activation can be used to detect stimulation or inhibition of NKT cell responses by the compounds of the invention.

Alternatively, rather than measurement of an expressed protein such as IL-4 or IFN-γ, modulation of NKT cell activation can be determined by changes in antigen-dependent T cell proliferation as measured by radiolabelling techniques as are recognized in the art. For example, a labeled (*e*.*g*., tritiated) nucleotide can be introduced to an assay culture medium. Incorporation of such a tagged nucleotide into DNA serves as a measure of T cell proliferation. This assay is not suitable for NKT cells that do not require antigen presentation for growth, *e*.*g*., NKT cell hybridomas. A difference in the level of T cell proliferation following contact with the compound or composition of the invention indicates the complex modulates activity of the T cells. For example, a decrease in NKT cell proliferation indicates the compound or composition can suppress an immune response. An increase in NKT cell proliferation indicates the compound or composition can stimulate an immune response.

Additionally, the ⁵¹Cr release assay can be used to determine cytotoxic activity.

These *in vitro* assays can be employed to select and identify modified glycolipids and modified glycolipid/protein complexes and compositions comprising same that are capable of appropriately modulating an immune response. Assays described above, *e*.*g*., measurement of IL-4 or IFN-γ production or NKT cell proliferation, are employed to determine if contact with the compound modulates T cell activation.

In addition or alternatively, immunization challenge experiments in animals, *e*.*g*., mice, rabbits, non-human primates, can be used to identify modified glycolipids and modified glycolipid/protein complexes and compositions comprising same that are capable of appropriately modulating an immune response and that may be efficacious for treatment and/or prevention of bacterial diseases, *e*.*g*., tuberculosis, in humans.

A modified glycolipid and modified glycolipid/protein complex, composition, or vaccine composition of the present invention can be used both to prevent a disease, and also to therapeutically treat a disease, *e.g.,* a viral disease, a bacterial disease, a fungal disease, a parasitic disease, an allergic disease, or a proliferative disease, *e*.*g*., cancer, or an autoimmune or inflammatory disease. In individuals already suffering from a disease, the present invention is used to further stimulate or modulate the immune system of the animal, thus reducing or eliminating the symptoms associated with that disease or disorder. As defined herein, "treatment" refers to the use of one or more modified bacteria, compositions, or vaccine compositions of the present invention to prevent, cure, retard, or reduce the severity of given disease symptoms in an animal, and/or result in no worsening of the disease over a specified period of time in an animal which has already contracted the disease and is thus in need of therapy.

The term "prevention" or "prevent" refers to the use of one or more modified glycolipids, modified glycolipid/protein complexes, compositions, or vaccine compositions of the present invention to generate immunity in an animal which has not yet contracted a disease, thereby preventing or reducing disease symptoms if the animal is later disposed to develop that disease. The methods therefore can be referred to as therapeutic methods or preventative or prophylactic methods. It is not required that any modified glycolipid, modified glycolipid/protein complex, composition, or vaccine composition of the present invention provide total immunity to a disease agent or totally cure or eliminate all disease symptoms.

As used herein, a "subject in need of therapeutic and/or preventative immunity" refers to an individual for whom it is desirable to treat, *i.e.,* to prevent, cure, retard, or reduce the severity of certain disease symptoms, and/or result in no worsening of disease over a specified period of time.

An "effective amount" is that amount the administration of which to an individual, either in a single dose or as part of a series, is effective for treatment and/or prevention. An amount is effective, for example, when its administration results in a reduced incidence or severity of disease symptoms associated with *M. tuberculosis* relative to an untreated individual, as determined about two weeks after challenge with infectious *M. tuberculosis.* This amount varies depending upon the health and physical condition of the individual to be treated, the taxonomic group of individual to be treated (*e*.*g*. human, nonhuman primate, primate, etc.), the responsive capacity of the individual's immune system, the degree of protection desired, the formulation of the vaccine, a professional assessment of the medical situation, and other relevant factors. It is expected that the effective amount will fall in a relatively broad range that can be determined through routine trials.

The term "vertebrate" is intended to encompass a singular "vertebrate" as well as plural "vertebrates" and comprises mammals and birds, as well as fish, reptiles, and amphibians.

The term "mammal" is intended to encompass a singular "mammal" and plural "mammals," and includes, but is not limited to humans; primates such as apes, monkeys (*e*.*g*., owl, squirrel, cebus, rhesus, African green, patas, cynomolgus, and cercopithecus), orangutans, baboons, gibbons, and chimpanzees; canids such as dogs and wolves; felids such as cats, lions, and tigers; equines such as horses, donkeys, and zebras, food animals such as cows, pigs, and sheep; ungulates such as deer and giraffes; ursids such as bears; and others such as rabbits, mice, ferrets, seals, whales. In particular, the mammal can be a human subject, a food animal or a companion animal.

The term "bird" is intended to encompass a singular "bird" and plural "birds," and includes, but is not limited to feral water birds such as ducks, geese, terns, shearwaters, and gulls; as well as domestic avian species such as turkeys, chickens, quail, pheasants, geese, and ducks. The term "bird" also encompasses passerine birds such as starlings and budgerigars.

The disclosure provides methods of preventing or treating a disease in a subject in need of such treatment or prevention, comprising administering to the subject with that disease, or prone to contract that disease, a composition comprising a modified glycolipid or a modified glycolipid/protein complex, such as a modified ceramide-like glycolipid/CD1d complex as described herein. In embodiments of the invention, the protein of the modified glycolipid/protein complex of the invention can be used as an antigen carrier for delivery of heterologous antigens or targeting molecules for a pathogen or a tumor specific antigen.

The present disclosure also includes a method of modulating, *i*.*e*., either stimulating or inhibiting an immune response, comprising administering to an animal an effective amount of a modified glycolipid or a modified glycolipid/protein complex, such as a modified ceramide-like glycolipid/CD1d complex as described herein. In embodiments of the invention, the composition of the invention further comprises a heterologous antigen or a targeting molecule from another pathogen or a tumor specific antigen, and the immune response is a priming immune response against the heterologous antigen or a targeting molecule.

In certain embodiments, the methods include treating a disease, *e*.*g*., an infectious or proliferative disease, in a subject with the disease by administering to the subject with the disease a composition of the invention, *e*.*g*., a modified glycolipid/CD1d complex of the invention, in an amount sufficient to alter the progression of said disease.

The methods include preventing a disease, *e*.*g*., an infectious or proliferative disease, in a subject in need of prevention of the disease by administering to the subject in need thereof a composition of the invention, *e*.*g*., a modified glycolipid/CD1d complex of the invention, in an amount sufficient to enhance an immune response against an antigen relative to administration of an unmodified glycolipid/CD1d complex (e.g., one lacking a photoreactive group).

In some embodiments, the disease being treated or prevented can be, without limitation a viral, bacterial, fungal, or parasitic infectious disease, an allergy or a proliferative disease such as cancer or an autoimmune or inflammatory disease such as multiple sclerosis, diabetes, or Sjogren's Syndrome,. More specifically, the disease can be, *e*.*g*., tuberculosis, Hansen's disease, pulmonary disease resembling tuberculosis, lymphadenitis, skin disease, disseminated disease, bubonic plague, pneumonic plague, tularemia, Legionnaire's disease, anthrax, typhoid fever, paratyphoid fever, foodborne illness, listeriosis, malaria, HIV, SIV, HPV, RSV, influenza, hepatitis (HAV, HBV, and HCV).

In another aspect, the methods disclosed herein include enhancing an immune response in a subject, comprising administering to the subject a modified glycolipid or modified glycolipid/protein complex of the invention (e.g., modified ceramide-like glycolipid/CD1d complex); and wherein the modified glycolipid or modified glycolipid/protein complex is administered in an amount sufficient to enhance antigen specific CD8 T-cell responses against an antigen and enhance the activity of Natural Killer T (NKT) cells in said animal.

As used herein, a "subject in need thereof' refers to an individual for whom it is desirable to treat, *i.e.,* to prevent, cure, retard, or reduce the severity of the symptoms of a disease, *e.g.,* a bacterial infection, and/or result in no worsening of a disease over a specified period of time.

According to these methods, a modified glycolipid or modified glycolipid/protein complex (e.g., modified ceramide-like glycolipid/CD1d complex), composition, or vaccine composition of the present invention can be administered in an amount sufficient to alter the progression of a disease.

"Immunization" (administration of a vaccine) is a common and widespread procedure and the vaccines of the invention used can be essentially any preparation intended for active immunological prophylaxis, including without limitation preparations of killed microbes of virulent strains and living microbes of attenuated strains. Stedman's Illustrated Medical Dictionary (24th edition), Williams & Wilkins, Baltimore, p. 1526 (1982). In some cases, vaccines must be administered more than once in order to induce effective protection; for example, known anti-toxin vaccines must be given in multiple doses.

The terms "priming" or "primary" and "boost" or "boosting" as used herein refer to the initial and subsequent immunizations, respectively, *i*.*e*., in accordance with the definitions these terms normally have in immunology. However, in certain embodiments, *e*.*g*., where the priming component and boosting component are in a single formulation, initial and subsequent immunizations may not be necessary as both the "prime" and the "boost" compositions are administered simultaneously. *See also,* McShane H, Curr Opin Mol Ther 4(1):13-4 (2002) and Xing Z and Charters TJ, Expert Rev Vaccines 6(4):539-46 (2007).

In certain embodiments, one or more compositions of the present invention are utilized in a "prime boost" regimen. In certain embodiments, one or more vaccine compositions of the present invention are delivered to a vertebrate, thereby priming the immune response of the vertebrate to a bacterial antigen, *e*.*g*., a mycobacterial antigen, or a tumor antigen, and then a second immunogenic composition is utilized as a boost vaccination. In certain embodiments, one or more vaccine compositions of the present invention are delivered to a vertebrate, thereby priming the immune response of the vertebrate to a heterologous antigen or a targeting molecule, *e.g.,* a heterologous antigen or a targeting molecule carried by a modified ceramide-like glycolipid/CD1d complex, and then a second immunogenic composition is utilized as a boost vaccination. In another embodiment, one or more vaccine compositions of the present invention are used to prime immunity, and then a second immunogenic composition, *e*.*g*., a recombinant bacterial or tumor vaccine, is used to boost the anti-bacterial or anti-tumor immune response. The vaccine compositions can comprise one or more vectors for expression of one or more genes that encode immunogenic polypeptides as described herein.

The present disclosure further provides a method for generating, enhancing, or modulating a protective and/or therapeutic immune response to a pathogen, *e.g.,* a bacterial, fungal, viral, or parasitic pathogen, or a tumor antigen, in a vertebrate, comprising administering to a vertebrate in need of therapeutic and/or preventative immunity one or more of the modified glycolipids, modified glycolipid/protein complexes, compositions, or vaccine compositions described herein. In some of these aspects, the composition includes a modified ceramide-like glycolipid/CD1d complex. In certain aspects, the modified glycolipid/CD1d complex further comprises a heterologous antigen or a targeting molecule.

In certain embodiments, the modified glycolipids, modified glycolipid/protein complexes, composition, or vaccine composition of the invention can be used to reduce the dose required to obtain a favorable response to the vaccine. This would have the potential benefits of reducing local and systemic toxicity, thus increasing the safety profile of the vaccine. In addition, this could have the benefit of allowing for reduced cost of production.

Certain aspects of the disclosure include a method of reducing or eliminating the anergic response of NKT cells to multiple administrations of ceramide-like glycolipid antigens administered by themselves, for example, those which are presented to NKT cells in the context of a bacterial cell wall. It has been shown that multiple administrations of α-GalCer, administered by itself, causes NKT cells to become non-responsive for an extended period of time. The present invention, in which modified glycolipids (α-GalCer) are administered as part of a modified ceramide-like glycolipid/CD1d complex, may protect NKT cells from anergy in response to antigen, and allow for a prolonged response upon multiple administrations. Accordingly, NKT cells are activated in response to stimulation with modified ceramide-like glycolipid/CD1d complexes loaded with a modified ceramide-like glycolipid of the present invention and furthermore, NKT cells can be reactivated in response to restimulation by modified ceramide-like glycolipid/CD1d complexes loaded with a modified ceramide-like glycolipid of the present invention.

According to the methods of the present disclosure, a composition comprising a modified glycolipid or modified glycolipid/protein complex (e.g., ceramide-like glycolipid antigen/CD1d complex) as described herein is administered to modulate an immune response in an animal, *e.g.,* a vertebrate, *e.g.,* a mammal, *e.g.,* a human. In certain aspects, the methods of the present disclosure result in the enhancement of an immune response, *e.g.,* to an immunogen delivered before, after, or concurrently with a modified glycolipid or modified glycolipid/protein complex (e.g., modified ceramide-like glycolipid/CD1d complex). Administration of a modified glycolipid or modified glycolipid/protein complex of the invention, *e*.*g*., with an immunogen, may typically result in the release of cytokines from immune cells, *e.g.,* NKT cells or NK cells. Cytokines released in response to administration of a composition, or vaccine composition of the invention may be those associated with a TH1-type immune response, *e*.*g*., interferon gamma and TNF-alpha. Alternatively, or in addition, administration of a modified glycolipid, composition, or vaccine composition of the present invention may result in the release of cytokines associated with a TH2-type immune response, *e.g.,* IL-4, IL-5, IL-10, or IL-13. Alternatively, or in addition, administration of a modified glycolipid, composition, or vaccine composition of the present invention may result in the release of other cytokines, *e*.*g*., IL-2, IL-1β, IL-12, IL-17, IL-23, TNF-β/LT, MCP-2, oncostatin-M, and RANTES. Methods to modulate the type of cytokines released include varying the ceramide-like glycolipid antigen of the ceramide-like glycolipid/CD1d protein complex. Choosing and testing various ceramide-like glycolipid antigens for their effect on cytokine release from NKT or other immune cells can be performed using *in vitro* assays described elsewhere herein and in Porcelli, U.S. Patent Appl. Publ. No. 2006/0052316, as well as by additional methods well-known to those of ordinary skill in the art. Administration of the modified glycolipids and modified glycolipid/protein complexes of the present invention (modified ceramide-like glycolipid/CD1d complexes) and vaccine compositions comprising the same may further modulate an immune response by inducing proliferation of NKT cells, and also by inducing recruitment and or activation of other immune and inflammatory cells including, but not limited to NK cells, CTLs, other T lymphocytes, *e*.*g*., CD8+ or CD4+ T lymphocytes, dendritic cells, B lymphocytes, and others.

In certain embodiments, administration of the modified glycolipids or modified glycolipid/protein complexes of the present invention and compositions comprising the same affects one or more NKT cell activities such as, but not limited to cell proliferation, the production of one or more cytokines, or recruitment and/or activation of non-NKT immune system cells including, but not limited to NK cells, CTLs, other T lymphocytes, *e.g*., CD8+ or CD4+ T lymphocytes, dendritic cells, B lymphocytes, and others.

In some embodiments, the modified glycolipid is a modified ceramide-like glycolipid that shows a bias towards inducing Type 2 cytokines, i.e., cytokines that have anti-inflammatory effect, (e.g., IL-4) in iNKT cells with blunted Type 1 cytokine (e.g., IFNγ) induction. Non-limiting examples of such ceramide-like glycolipids are described in U.S. Patent Nos. 7,772,380 and 8,022,043. In some of these embodiments, the ceramide-like glycolipid has one of the following two structures.

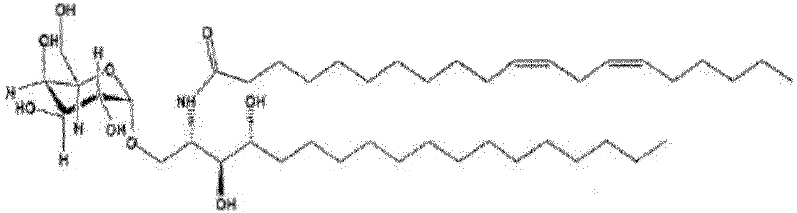

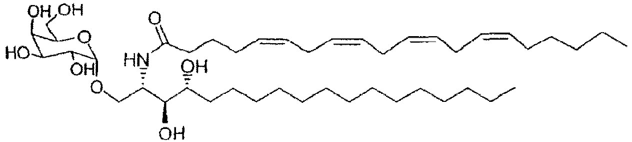

Also described is the use of modified glycolipids or modified glycolipid/protein complexes of the invention (modified ceramide-like glycolipid/CD1d complexes) as recombinant vaccines used to modulate an immune response to an immunogen, *e*.*g*., a pathogen antigen or tumor antigen, that is administered together or just before or soon after the modified glycolipid or modified 72 glycolipid/protein complex (modified ceramide-like glycolipid/CD1d complex). Accordingly, the present disclosure provides a method of inducing an immune response to an immunogen in an animal, where the method comprises administering to an animal in need thereof a composition comprising an immunogen, which is present in a modified glycolipid/protein complex (e.g., modified ceramide-like glycolipid/CD1d complex). The modified glycolipid/CD1d complex (e.g., the modified ceramide-like glycolipid/CD1d complex) is administered in an amount sufficient to induce the immune response against the immunogen, *e*.*g*., bacterial pathogen or immunogen expressed by recombinant bacteria, relative to administration of the immunogen without the modified glycolipid/CD1d complex (e.g., modified ceramide-like glycolipid/CD1d complex). A modified glycolipid or modified glycolipid/CD1d complex for use as a vaccine can in certain embodiments be targeted to a particular organ, tissue, cell or cell surface marker as described, *e*.*g*., in U.S. Patent Appl. Publ. No. 2006/0269540.

In certain embodiments, the modified glycolipids or modified glycolipid/protein complexes of the present invention (modified ceramide-like glycolipid/CD1d complexes) or compositions comprising the same are administered as a therapeutic vaccine, *e*.*g*., to an animal already suffering from a disease. According to these methods, the immune response elicited by a modified glycolipid or modified glycolipid/protein complex of the invention is effective in treating, *e*.*g*., affecting the outcome of the disease by reducing symptoms or lessening the severity of the disease, and the modified glycolipid or modified glycolipid/protein complex is administered in an amount sufficient to modulate the immune response against the immunogen relative to administration of the immunogen in the absence of the modified glycolipid/protein complex. Alternatively, modified glycolipids or modified glycolipid/protein complexes of the present invention (modified ceramide-like glycolipid/CD1d complexes) and compositions comprising the same are administered as a prophylactic vaccine, *i.e.,* to prevent, or reduce symptoms to a disease, such as an infectious disease that might be contracted by that animal in the future. According to these methods, the immune response elicited by the modified glycolipids or modified glycolipid/protein complexes (e.g., modified ceramide-like glycolipid/CD1d complexes) is effective in preventing, *e*.*g*., affecting the outcome of the disease by reducing symptoms or lessening the severity of the disease, and the modified glycolipid or modified glycolipid/protein complex is administered in an amount sufficient to modulate the immune response against the immunogen relative to administration of the immunogen in the absence of the modified glycolipid or modified glycolipid/protein complex (e.g., modified ceramide-like glycolipid/CD1d complex).

The methods, modified glycolipids, modified glycolipid/protein complexes, compositions, or vaccine compositions as described herein are also useful for raising an immune response against infectious agents, *e.g.,* a modified glycolipid/protein complex wherein the complex comprises a heterologous antigen or a targeting molecule, *e.g.,* a viral antigen, a bacterial antigen, a fungal antigen, or a parasitic antigen. Infectious agents that can cause disease or symptoms that can be treated by the modified glycolipids, modified glycolipid/protein complexes, compositions, or vaccine compositions of the invention include, but are not limited to viral, bacterial, fungal, and parasitic agents. Examples of viruses, include, but are not limited to the following DNA and RNA viral families: Arbovirus, Adenoviridae, Arenaviridae, Arterivirus, Birnaviridae, Bunyaviridae, Caliciviridae, Circoviridae, Coronaviridae, Flaviviridae, Hepadnaviridae (hepatitis), Herpesviridae (such as, Cytomegalovirus, Herpes Simplex, Herpes Zoster), Mononegavirus (*e*.*g*., Paramyxoviridae, Morbillivirus, Rhabdoviridae), Orthomyxoviridae (*e*.*g*., Influenza), Papovaviridae, Parvoviridae, Picornaviridae, Poxviridae (such as Smallpox or Vaccinia), Reoviridae (*e*.*g*., Rotavirus), Retroviridae (HTLV-I, HTLV-II, Lentivirus), and Togaviridae (*e*.*g*., Rubivirus). Viruses falling within these families can cause a variety of diseases or symptoms, including, but not limited to: arthritis, bronchiollitis, encephalitis, eye infections (*e*.*g*., conjunctivitis, keratitis), chronic fatigue syndrome, hepatitis (A, B, C, E, Chronic Active, Delta), meningitis, opportunistic infections (*e*.*g*., AIDS), pneumonia, Burkitt's Lymphoma, chickenpox, hemorrhagic fever, measles, mumps, parainfluenza, rabies, the common cold, Polio, leukemia, Rubella, sexually transmitted diseases, skin diseases (*e*.*g*., Kaposi's, warts), and viremia.

Similarly, bacterial or fungal agents that can cause disease or symptoms can be treated or prevented by the modified glycolipids, modified glycolipid/protein complexes, compositions, or vaccine compositions of the invention. These include, but are not limited to, the following Gram-Negative and Gram-positive bacterial families and fungi: Actinomycetales (*e*.*g*., Corynebacterium, Mycobacterium, Norcardia), Aspergillosis, Bacillaceae (*e*.*g*., Anthrax, Clostridium), Bacteroidaceae, Blastomycosis, Bordetella, Borrelia, Brucellosis, Candidiasis, Campylobacter, Coccidioidomycosis, Cryptococcosis, Dermatocycoses, Enterobacteriaceae (Klebsiella, Salmonella, Serratia, Yersinia), Erysipelothrix, Helicobacter, Legionellosis, Leptospirosis, Listeria, Mycoplasmatales, Neisseriaceae (*e*.*g*., Acinetobacter, Gonorrhea, Menigococcal), Pasteurellacea Infections (*e*.*g*., Actinobacillus, Heamophilus, Pasteurella), Pseudomonas, Rickettsiaceae, Chlamydiaceae, Syphilis, and Staphylococcal. These bacterial or fungal families can cause the following diseases or symptoms, including, but not limited to: bacteremia, endocarditis, eye infections (conjunctivitis, tuberculosis, uveitis), gingivitis, opportunistic infections (*e*.*g*., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis, Chlamydia, Syphilis, Diphtheria, Leprosy, Paratuberculosis, Tuberculosis, Hansen's disease, pulmonary disease resembling tuberculosis, Lymphadenitis, skin disease, disseminated disease, Lupus, Botulism, gangrene, tetanus, impetigo, Rheumatic Fever, Scarlet Fever, sexually transmitted diseases, skin diseases (*e*.*g*., cellulitis, dermatocycoses), toxemia, urinary tract infections, wound infections.

Moreover, the modified glycolipids, modified glycolipid/protein complexes, compositions, or vaccine compositions of the present invention can be used to treat or prevent diseases caused by parasitic agents. Those that can be treated by the compounds of the invention include, but are not limited to, the following families: amebiasis, babesiosis, coccidiosis, cryptosporidiosis, dientamoebiasis, dourine, ectoparasitic, giardiasis, helminthiasis, leishmaniasis, theileriasis, toxoplasmosis, trypanosomiasis, and trichomonas.

According to the disclosed methods, modified glycolipids, modified glycolipid/protein complexes, compositions, or vaccine compositions for use in the methods can be administered, for example, by intramuscular (i.m.), intravenous (i.v.), subcutaneous (s.c.), or intrapulmonary routes. Other suitable routes of administration include, but are not limited to intratracheal, transdermal, intraocular, intranasal, inhalation, intracavity, intraductal (*e.g.,* into the pancreas), and intraparenchymal (*i*.*e*., into any tissue) administration. Transdermal delivery includes, but not limited to intradermal (*e.g.,* into the dermis or epidermis), transdermal (*e.g.,* percutaneous) and transmucosal administration (*i*.*e*., into or through skin or mucosal tissue). Intracavity administration includes, but not limited to administration into oral, vaginal, rectal, nasal, peritoneal, or intestinal cavities as well as, intrathecal (*i.e*., into spinal canal), intraventricular (*i.e*., into the brain ventricles or the heart ventricles), intraatrial (*i.e*., into the heart atrium) and sub arachnoid (*i*.*e*., into the sub arachnoid spaces of the brain) administration.

Compositions of the present invention further comprise a suitable carrier. Such compositions comprise a therapeutically effective amount of the modified glycolipid or the modified glycolipid/protein complex and a pharmaceutically acceptable carrier or excipient. Such a carrier includes but is not limited to saline, buffered saline, dextrose, water, glycerol, ethanol, and combinations thereof. The formulation should suit the mode of administration.

The term "pharmaceutically acceptable" refers to compositions that are, within the scope of sound medical judgment, suitable for contact with the tissues of human beings and animals without excessive toxicity or other complications commensurate with a reasonable benefit/risk ratio. In some embodiments, the compositions and vaccines of the present invention are pharmaceutically acceptable.

Modified glycolipids or modified glycolipid/protein complexes of the present invention (modified ceramide-like glycolipid/CD1d complexes) can be administered in pharmaceutical compositions, *e.g*., vaccine compositions, in combination with one or more pharmaceutically acceptable excipients, carriers, or diluents. In certain embodiments, the pharmaceutical compositions, *e*.*g*., vaccine compositions of the invention further comprise a heterologous antigen or a targeting molecule. It will be understood that, when administered to a human patient, the total single or daily usage of the pharmaceutical compositions of the present invention will be decided by the attending physician within the scope of sound medical judgment. The specific therapeutically effective dose level for any particular patient will depend upon a variety of factors including the type and degree of the response to be achieved; the specific composition of another agent, if any, employed; the age, body weight, general health, sex and diet of the patient; the time of administration, route of administration, and rate of excretion of the composition; the duration of the treatment; drugs (such as a chemotherapeutic agent) used in combination or coincidental with the specific composition; and like factors well known in the medical arts. Suitable formulations, known in the art, can be found in Remington's Pharmaceutical Sciences (latest edition), Mack Publishing Company, Easton, PA.

A composition to be used in a given preventative or therapeutic treatment will be formulated and dosed in a fashion consistent with good medical practice, taking into account the clinical condition of the individual patient (especially the side effects of prevention or treatment with the compounds alone), the site of delivery of the compound, the method of administration, the scheduling of administration, and other factors known to practitioners. The "effective amount" of the compounds of the invention for purposes herein is thus determined by such considerations.

Appropriate dosage of the compositions, *e*.*g*., vaccine compositions, of the invention to be administered to a patient will be determined by a clinician. However, as a guide, a suitable amount of a composition of the invention can be between about 10¹ to 10¹² CFU per dose, *e.g*., 10¹, 10², 10³, 10⁴, 10⁵, 10⁶, 10⁷, 108, 109, 10¹⁰, 10¹¹, or 10¹² CFU, suspended in 0.05 to 0.1 ml of an immunologically inert carrier, *e.g.,* a pharmaceutical carrier. In one embodiment, an effective amount of a vaccine of the invention to induce immunity sufficient to prevent or treat, *i.e.,* cure, ameliorate, lessen the severity of, or prevent or reduce a diseases described herein is about 10³ to about 10⁷ colony forming units (CFU)/kg body weight. A composition of the invention can be administered as a single dose or multiple doses. The vaccine formulations of the present invention can be employed in dosage forms such as capsules, liquid solutions, suspensions, or elixirs, for oral administration, or sterile liquid for formulations such as solutions or suspensions for, *e*.*g*., parenteral, intranasal or topical administration.

Compositions of the invention can be administered orally, intravenously, rectally, parenterally, intracisternally, intradermally, intravaginally, intraperitoneally, topically (as by powders, ointments, gels, creams, drops or transdermal patch), bucally, or as an oral or nasal spray. The term "parenteral" as used herein refers to modes of administration which include intravenous, intramuscular, intraperitoneal, intrasternal, subcutaneous and intraarticular injection and infusion.

Compositions, *e*.*g*, vaccine compositions, of the invention can be formulated according to known methods. Suitable preparation methods are described, for example, in Remington's Pharmaceutical Sciences, 16th Edition, A. Osol, ed., Mack Publishing Co., Easton, PA (1980), and Remington's Pharmaceutical Sciences, 19th Edition, A.R. Gennaro, ed., Mack Publishing Co., Easton, PA (1995). Although the composition can be administered as an aqueous solution, it can also be formulated as an emulsion, gel, solution, suspension, lyophilized form, or any other form known in the art. In addition, the composition can contain pharmaceutically acceptable additives including, for example, diluents, binders, stabilizers, and preservatives. Once formulated, the compositions of the invention can be administered directly to the subject. The subjects to be treated can be animals; in particular, human subjects can be treated.

Compositions of the invention ordinarily will be stored in unit or multi-dose containers, for example, sealed ampules or vials, as an aqueous solution or as a lyophilized formulation for reconstitution. Mycobacterial compositions with directly incorporated glycolipid adjuvant can be lypohilized and the adjuvant activity will be recovered intact when the composition is rehydrated and suspended for injection. As an example of a lyophilized formulation, 10-ml vials are filled with 5 ml of sterile-filtered 1% (w/v) aqueous solution, and the resulting mixture is lyophilized. An infusion solution is prepared by reconstituting the lyophilized composition using water, *e*.*g*., bacteriostatic Water-for-Injection.

The disclosure also provides a pharmaceutical pack or kit comprising one or more containers filled with one or more of the ingredients of the pharmaceutical compositions of the invention. Associated with such containers can be a notice in the form prescribed by a governmental agency regulating the manufacture, use or sale of pharmaceuticals or biological products, which notice reflects approval by the agency of manufacture, use or sale for human administration. In addition, the compositions of the present invention can be employed in conjunction with other therapeutic compositions.

Suitable preparations of such compositions include, but are not limited to injectables, either as liquid solutions or suspensions; solid forms suitable for solution in, or suspension in liquid prior to injection, can also be prepared. The preparation can also be emulsified, or the polypeptides encapsulated in liposomes. The active ingredients are often mixed with excipients which are pharmaceutically acceptable and compatible with the active ingredient. Suitable excipients are, for example, water, saline, dextrose, glycerol, or the like and combinations thereof. In addition, if desired, the preparation can also include minor amounts of auxiliary substances such as wetting or emulsifying agents, pH buffering agents, and/or adjuvants which enhance the effectiveness of the active ingredient.

The composition, if desired, can also contain minor amounts of wetting or emulsifying agents, or pH buffering agents. The composition can be a liquid solution, suspension, emulsion, tablet, pill, capsule, sustained release formulation, or powder. Oral formulation can include standard carriers such as pharmaceutical grades of mannitol, lactose, starch, magnesium stearate, sodium saccharine, cellulose, magnesium carbonate, etc.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., Sambrook et al., ed., Cold Spring Harbor Laboratory Press: (1989); Molecular Cloning: A Laboratory Manual, Sambrook et al., ed., Cold Springs Harbor Laboratory, New York (1992), DNA Cloning, D. N. Glover ed., Volumes I and II (1985); Oligonucleotide Synthesis, M. J. Gait ed., (1984); Mullis *et al.* U.S. Pat. No: 4,683,195; Nucleic Acid Hybridization, B. D. Hames & S. J. Higgins eds. (1984); Transcription And Translation, B. D. Hames & S. J. Higgins eds. (1984); Culture Of Animal Cells, R. I. Freshney, Alan R. Liss, Inc., (1987); Immobilized Cells And Enzymes, IRL Press, (1986); B. Perbal, A Practical Guide To Molecular Cloning (1984); the treatise, Methods In Enzymology, Academic Press, Inc., N.Y.; Gene Transfer Vectors For Mammalian Cells, J. H. Miller and M. P. Calos eds., Cold Spring Harbor Laboratory (1987); Methods In Enzymology, Vols. 154 and 155 (Wu et al. eds.); Immunochemical Methods In Cell And Molecular Biology, Mayer and Walker, eds., Academic Press, London (1987); Handbook Of Experimental Immunology, Volumes I-IV, D. M. Weir and C. C. Blackwell, eds., (1986); Manipulating the Mouse Embryo, Cold Spring Harbor Laboratory Press, Cold Spring Harbor, N.Y., (1986); and in Ausubel et al., Current Protocols in Molecular Biology, John Wiley and Sons, Baltimore, Maryland (1989).

General principles of antibody engineering are set forth in Antibody Engineering, 2nd edition, C.A.K. Borrebaeck, Ed., Oxford Univ. Press (1995). General principles of protein engineering are set forth in Protein Engineering, A Practical Approach, Rickwood, D., et al., Eds., IRL Press at Oxford Univ. Press, Oxford, Eng. (1995). General principles of antibodies and antibody-hapten binding are set forth in: Nisonoff, A., Molecular Immunology, 2nd ed., Sinauer Associates, Sunderland, MA (1984); and Steward, M.W., Antibodies, Their Structure and Function, Chapman and Hall, New York, NY (1984). Additionally, standard methods in immunology known in the art and not specifically described are generally followed as in Current Protocols in Immunology, John Wiley & Sons, New York; Stites et al. (eds), Basic and Clinical -Immunology (8th ed.), Appleton & Lange, Norwalk, CT (1994) and Mishell and Shiigi (eds), Selected Methods in Cellular Immunology, W.H. Freeman and Co., New York (1980).

Standard reference works setting forth general principles of immunology include Current Protocols in Immunology, John Wiley & Sons, New York; Klein, J., Immunology: The Science of Self-Nonself Discrimination, John Wiley & Sons, New York (1982); Kennett, R., et al., eds., Monoclonal Antibodies, Hybridoma: A New Dimension in Biological Analyses, Plenum Press, New York (1980); Campbell, A., "Monoclonal Antibody Technology" in Burden, R., et al., eds., Laboratory Techniques in Biochemistry and Molecular Biology, Vol. 13, Elsevere, Amsterdam (1984), Kuby Immunnology 4th ed. Ed. Richard A. Goldsby, Thomas J. Kindt and Barbara A. Osborne, H. Freemand & Co. (2000); Roitt, I., Brostoff, J. and Male D., Immunology 6th ed. London: Mosby (2001); Abbas A., Abul, A. and Lichtman, A., Cellular and Molecular Immunology Ed. 5, Elsevier Health Sciences Division (2005); Kontermann and Dubel, Antibody Engineering, Springer Verlan (2001); Sambrook and Russell, Molecular Cloning: A Laboratory Manual. Cold Spring Harbor Press (2001); Lewin, Genes VIII, Prentice Hall (2003); Harlow and Lane, Antibodies: A Laboratory Manual, Cold Spring Harbor Press (1988); Dieffenbach and Dveksler, PCR Primer Cold Spring Harbor Press (2003).

All technical and scientific terms used herein have the same meaning. Efforts have been made to ensure accuracy with respect to numbers used (e.g. amounts, temperature, etc.) but some experimental errors and deviations should be accounted for. It is to be noted that the term "a" or "an" entity refers to one or more of that entity; for example, "a protein" is understood to represent one or more proteins. As such, the terms "a" (or "an"), "one or more," and "at least one" can be used interchangeably herein.

Throughout this specification and the claims, the words "comprise," "comprises," and "comprising" are used in a non-exclusive sense, except where the context requires otherwise.

As used herein, the term "about," when referring to a value is meant to encompass variations of, in some embodiments ± 50%, in some embodiments ± 20%, in some embodiments ± 10%, in some embodiments ± 5%, in some embodiments ± 1%, in some embodiments ± 0.5%, and in some embodiments ± 0.1% from the specified amount, as such variations are appropriate to perform the disclosed methods or employ the disclosed compositions.

Where a range of values is provided, it is understood that each intervening value, to the tenth of the unit of the lower limit, unless the context clearly dictates otherwise, between the upper and lower limit of the range and any other stated or intervening value in that stated range, is encompassed within the invention. The upper and lower limits of these small ranges which may independently be included in the smaller rangers is also encompassed within the invention, subject to any specifically excluded limit in the stated range. Where the stated range includes one or both of the limits, ranges excluding either or both of those included limits are also included in the invention.

### EXPERIMENTAL

### EXAMPLE 1

### Synthesis of αGalCer-benzophenone derivatives

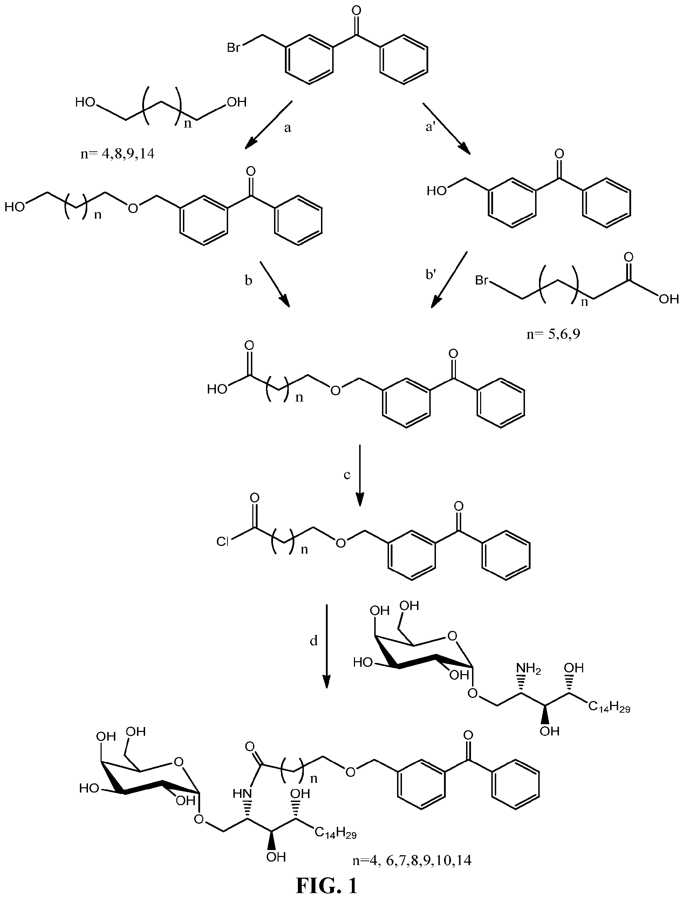

The basic scheme for synthesis of α-GalCer-benzophenone derivatives is shown in FIG. 1.

### Step a: General method for monoalkylation procedure.

To a cold solution of the diol (10 mmol, 4.0 eq) in anhydrous DMF (40 mL) was added sodium hydride (10 mmol, 4.0 eq) in small portions at 0°C and the mixture allowed to stir for 30 minutes. A solution of 3-bromomethyl-phenyl-methanone 1 (2.5 mmol, 1.0 eq) in DMF (20 mL) was then added dropwise. The reaction mixture was allowed to stir overnight at room temperature and then taken up in water (100 mL). The resulting mixture was then extracted with CH₂Cl₂ (3 x 30 mL). The combined organic layers were washed with brine (30 mL), dried over anhydrous Na₂SO₄ and concentrated in vacuo. The residue was then purified by flash chromatography using Hexanes: EtOAc (5:1) as eluent.

### Step b: General method for oxidation of the monoalkylated benzophenone alcohol derivatives to the corresponding carboxylic acids.

The monoalkylated benzophenone derivatives (1mmol) from above were dissolved in THF (20 mL) and pyridinium dichromate (PDC) (3 mmol, 3 eq) was added. The reaction mixture was allowed to stir at room temperature for 48 hours and then taken up in water (20 mL). The resulting mixture was then extracted with CH₂Cl₂ (3 x 30 mL). The combined organic layers were washed with brine (30 mL), dried over anhydrous Na₂SO₄ and concentrated in vacuo. The residue was then purified by flash chromatography using 10% methanol in chloroform as eluent.

### Step a': 3-(hydroxymethyl)benzophenone

3-(hydroxymethyl)benzophenone (10mmol) was obtained from 3-(bromomethyl)benzophenone (12 mmol) by refluxing the latter in a mixture of THF:H₂O (1:1) in the presence of CaCO₃ as described in the reported literature.

### Step b': Reaction with bromoacids.

To a cold solution of 3-(hydroxymethyl)benzophenone (1 mmol, 1eq) in a mixture of DMF (10ml) and hexamethylphosphoramide (HMPA) (1ml) was added sodium hydride (NaH) (60% in mineral oil) (1.2 mmol, 1.2 eq) at 0°C in small amounts. The reaction mixture was allowed to stir for 20 minutes before the dropwise addition of the respective bromoacids [n= 5, 6, 9] (1.2 mmol, 1.2 eq) dissolved in DMF (5ml). The reaction was left at room temperature overnight and the excess NaH was quenched using methanol. The reaction was then taken up in water (50 mL) and extracted with EtOAc (3x40ml). The combined organic layers were washed with brine (30 mL), dried over anhydrous Na₂SO₄ and concentrated in vacuo. The residue was then purified by flash chromatography using 10% methanol in chloroform as eluent.

### Step c: Formation of acid chloride

The benzophenone carboxylic acid derivatives obtained from steps b and b' (0.5 mmol) were added to neat oxalyl chloride (1 mL) and stirred at 70°C for 2 h, after which time the solution was cooled to room temperature and the unreacted oxalyl chloride was removed under a stream of argon. The residual volatiles were removed under reduced pressure.

### Step d: Synthesis of compounds DB11-1 (n=4), DB12-6 (n=6), DB12-7 (n=7), DB11-2 (n=8), DB12-8 (n=9), DB12-9 (n=10), DB11-3 (n=14)

The resulting crude acyl chlorides from above were dissolved in THF (1 mL) and added to a solution of amine (0.5 mmol, 1 eq) in THF/NaOAc(sat) (1:1, 1 mL). The reaction was stirred vigorously overnight after which the organic phase was removed. The aqueous phase was further extracted with THF (2 x 1 mL), and the combined organic phases were concentrated. The residue was finally purified by flash chromatography (gradient from CHCl₃ to 20% MeOH in CHCl₃) to give the acylated target compounds.

### EXAMPLE 2

### Synthesis of αGalCer-benzophenone/CD1d complex

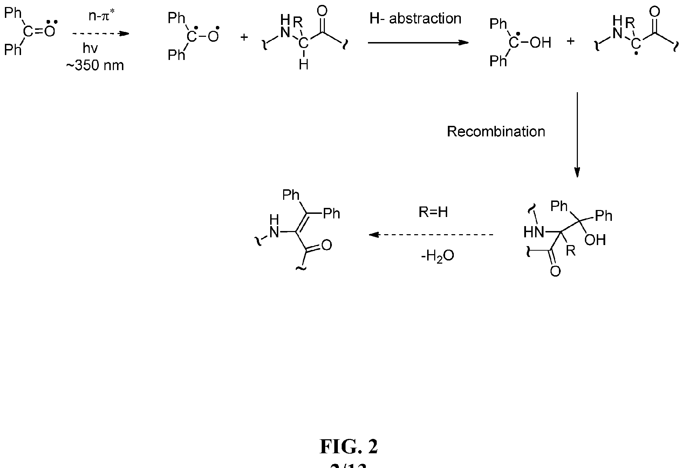

The basic scheme for synthesis of the α-GalCer-benzophenone derivatives is shown in FIG. 2.

In general, recombinant soluble murine CD1d (mCD1d) was diluted to 400 µg/ml in PBS pH 7.2. Glycolipids (i.e., benzophenone derivatives of alpha-galactosyl ceramide) were dissolved in 100% DMSO to yield 1 mg/ml final concentration and then diluted to 200 µM in PBS containing 0.1% Triton X-100. The glycolipid suspension was then heated to 80°C for 5 minutes, vortexed for 30 seconds, sonicated in a water bath sonicator for 5 minutes, and finally vortexed for 30 seconds. Equal volumes of CD1d and glycolipid stocks were then added to give final concentrations of 200 µg/ml CD1d (∼4 µM), 100 µM glycolipid and 0.05% TritonX-100, and allowed to incubate overnight (16 hrs) at room temperature. CD1d complexes were transferred to low binding 96 well plates (100 µl/well) for covalently linking the protein to the glycolipid present in its ligand binding site. A long wave UV lamp (Schleider & Schuell) emitting at fixed λ 365 was placed 1 inch above the sample level and complexes were irradiated for 1 hr on ice. The end result is that the CD1d protein is covalently bound to the αGalCer-benzophenone glycolipid.

### EXAMPLE 3

### Tryptophan Flourescence Spectra of mCDld with and without glycolipid ligands

The effect of glycolipid binding to mouse CD1d was tested. Fluorescence quenching of mCD1d due to glycolipid binding was measured using a Horibo Jobin Yvon Fluoromax-3 fluorescent spectrophotometer and the data were analyzed using FluorEssence software. Protein (0.1 µM) was mixed either with glycolipid antigens (at 4 µM) or the ligand vehicle in a quartz cuvette 1 cm wide, to a final volume of 100 µl and placed in the spectrometer already set at 25°C. The samples were excited at λ₂₉₅ and emission measured from λ₂₉₀ to λ₄₀₀. No shift in wavelength for fluorescence emission maximum was observed, but all of the glycolipids caused fluorescence quenching to various levels indicating that ligand binding leads to observable structural alterations in CD1d.

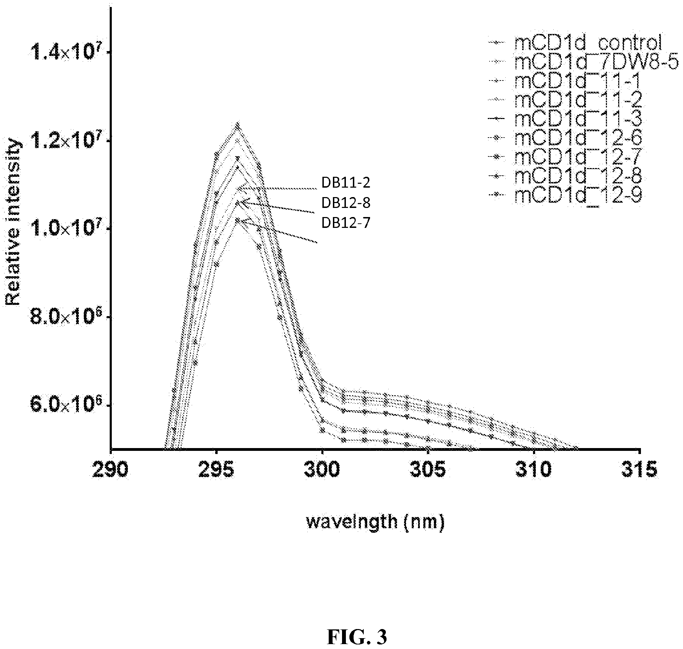

The results are shown in FIG. 3. These results show that DB12-7 induced the greatest quenching compared to the other benzophenone-modified glycolipids (BPGCs), suggesting a higher binding affinity for this glycolipid compared to the others tested. Although this indicates that DB12-7 is the most avid binding ligand from this group, this analysis does not provide information on whether the complexes formed by the binding of these glycolipids result in the appropriate structure for recognition by iNKT cell antigen receptors and biological activity.

### EXAMPLE 4

### Activation of NKT cells (with mCDld expressing DCs)

The activated natural killer cells were calculated based on those secreting IL-2. Specifically, bone marrow-derived dendritic cells (BMDCs) from C57BL/6 mice (10,000 cells in 100 µl culture medium) were plated in wells of a 96 well tissue culture plate and allowed to adhere to the plates for 30 minutes at 37°C. The cultures were then exposed to varying BPGC concentrations ranging between 5 and 0.01 µg/ml in 100 µl for 3 hrs at 37°C. Glycolipids not taken up by BMDCs were removed by washing with culture medium. To detect the BPGC presentation by CD1d, iNKT hybridoma DN3A4-1.2 (5000 cells in 100 µl volume) was added and the stimulation was detected by the level of IL-2 secreted in the supernatant following 18 hrs of incubation at 37°C.

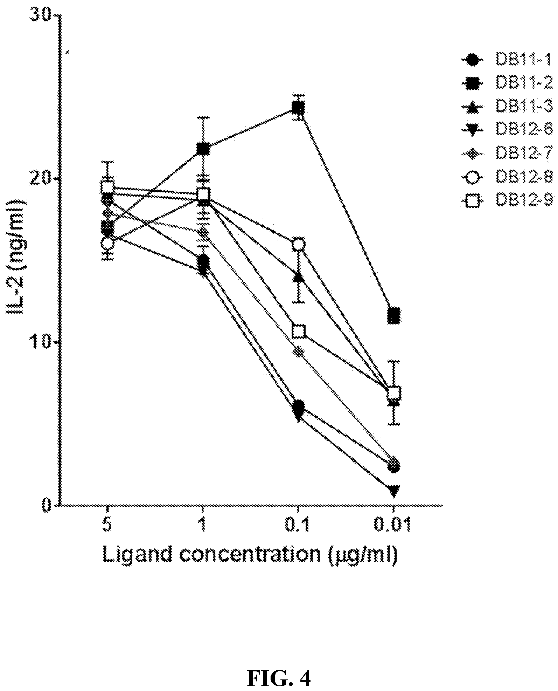

The results are shown in FIG. 4. These results show that DB11-2 stimulated iNKTs most efficiently at a low concentration of 0.1 µg/ml, and was the most potent in terms of biological activity in this assay among this group of glycolipids. DB12-7 induced lower but still significant IL-2 secretion.

### EXAMPLE 5

### Activation of NKT cells (with hCD1d expressing HeLa cells)

The activated natural killer cells were again calculated based on those secreting IL-2. Specifically, plate bound human CD1d transfected HeLa cells (10,000 cells in 100 µl medium) were plated in a 96 well tissue culture plate and allowed to adhere to the plates for 30 minutes at 37°C. Adherent HeLa cells were treated with varying BPGC concentrations ranging between 5 and 0.01 µg/ml in 100 µl for 3 hrs at 37°C. Glycolipid not taken up by cells was removed by washing with medium. To detect the BPGC presentation by CD1d, iNKT hybridoma DN3A4-1.2 (5000 cells in 100 µl volume) was added and the stimulation was detected by the level of IL-2 secreted in the supernatant following incubation at 37°C for 18 hours.

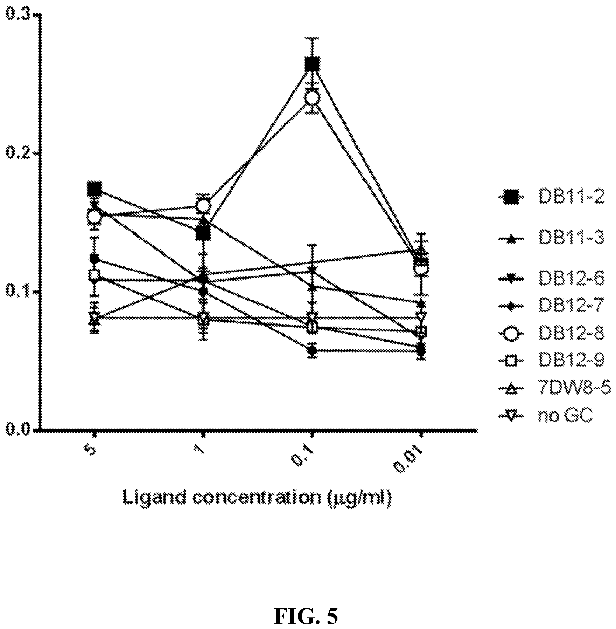

The results are shown in FIG. 5. These results confirmed that DB11-2 and DB12-8 were stimulatory toward iNKT cells when presented by human CD1d, and these were efficiently presented at a low concentration of 0.1 µg/ml.

### EXAMPLE 6

### Detection of ligand binding affinity of plate bound mCD1d with and without UV activation (by ELISA using mAb L363)

The ligand binding affinity of plate bound mCD1d was measured by ELISA using mAb L363 to detect murine CD1d/aGalCer complexes. ELISA plates were coated with mCDld (10 µg/ml, 30 µl/well) overnight at 4°C in PBS pH 8.2. Supernatant was then removed along with any unbound protein. Glycolipids (5 µM, 30µl/well) were loaded and allowed to incubate overnight at 25°C. Supernatant was again removed and the complexes were washed three times with PBS to remove any unbound glycolipid. For those complexes that underwent UV activation, UV crosslinking was performed in solution (30µl/well PBS) and at a fixed wavelength of 365 nm from a UV lamp (RAD-FREE long wave UV lamp, Schleicher & Schuell). The UV lamp was placed one inch from the sample and the sample was allowed to sit on ice for 1 hr. Both the complexes that underwent UV activation and those that did not were then allowed to dissociate for three days (i. remove supernatant and add 200 µl of PBS + 0.05% Triton X-100 (PBS-Tx), ii. incubate for one day at 25°C, iii. repeat steps i and ii two more times) before ELISA was performed to quantify binding of mAb L363.

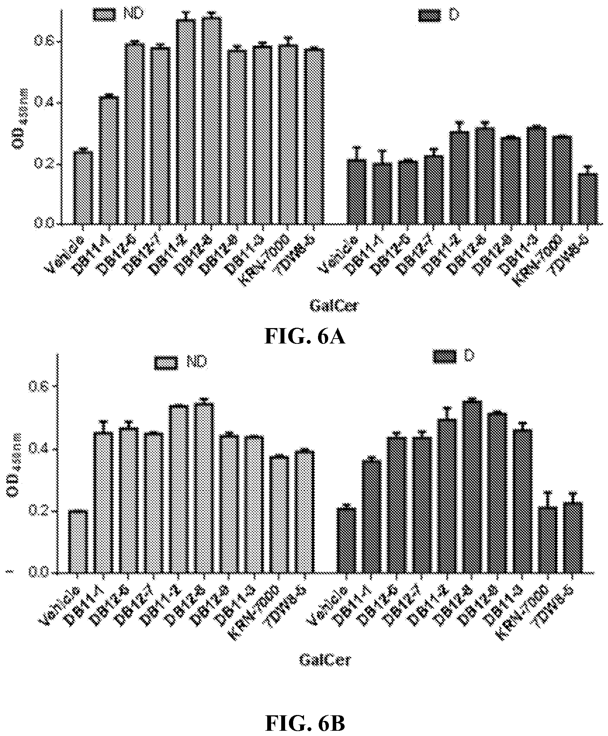

The results are shown in FIGS. 6A-6B. All of the glycolipids showing affinity for CD1d and the complexes could be detected using L363 ELISA. As a result of incubating the plate bound CD1d:GC complexes in PBS-Tx for 3 days, the non-UV crosslinked glycolipid ligands dissociated significantly (FIG. 6A), while UV cross-linked BPGCs did not (FIG. 6B). KRN7000 and 7DW8-5 do not have a UV-activatable group and hence both of them dissociated after 3 days independently of UV exposure. These results show that DB12-8, DB11-2 and DB12-9 had the highest levels of immunoreactive complex formation in this assay and that these complexes showed no significant dissociation after UV exposure.

### EXAMPLE 7

### Detection of ligand binding affinity of plate bound mCD1d with and without UV activation (iNKT DN3A4-1.2 hybridoma stimulation assay)

The ligand binding affinity of plate bound mCD1d was detected by iNKT DN3A4-1.2 hybridoma stimulation assay. mCD1d (10 µg/ml) was loaded with glycolipids (5 µM) overnight at 25°C. ELISA plates were coated with mCD1d:GC complexes (2.5 µg/ml, 30 µl/well) for 12-18 hours at 4°C in PBS pH 8.2. Supernatant was then removed and the complexes were washed three times with PBS to remove any unbound glycolipid and protein. For those complexes that underwent UV activation, UV crosslinking was performed in solution (30µl/well PBS) and at a fixed wavelength of 365 nm from a UV lamp (RAD-FREE long wave UV lamp, Schleicher & Schuell). The UV lamp was placed one inch from the sample and the sample was allowed to sit on ice for 1 hr. Supernatant was then removed and the complexes were washed three times with PBS to remove any unbound glycolipid and protein. Both the complexes that underwent UV activation and those that did not were then allowed to dissociate for three days (i. remove any residual liquid from wells and add 200 µl per well of PBS+0.05% Triton X-100, ii. incubate for 24 hours at 25°C, iii. repeat steps i and ii two more times). iNKT hybridoma DN3A4-1.2 cells (about 30,000 cells) were then added and allowed to incubate for 24 hours at 37°C. iNKT stimulatory activity of the complexes was determined by IL-2 ELISA of iNKT secretion.

7DW8-5 did not stimulate iNKT post-dissociation as expected, but non-crosslinked mCD1d complexes with DB12-7, DB11-2, DB12-8 and DB12-9 showed some, albeit reduced, iNKT stimulation even after dissociation, perhaps because the dissociation was not absolute because of high binding affinities of these glycolipids.

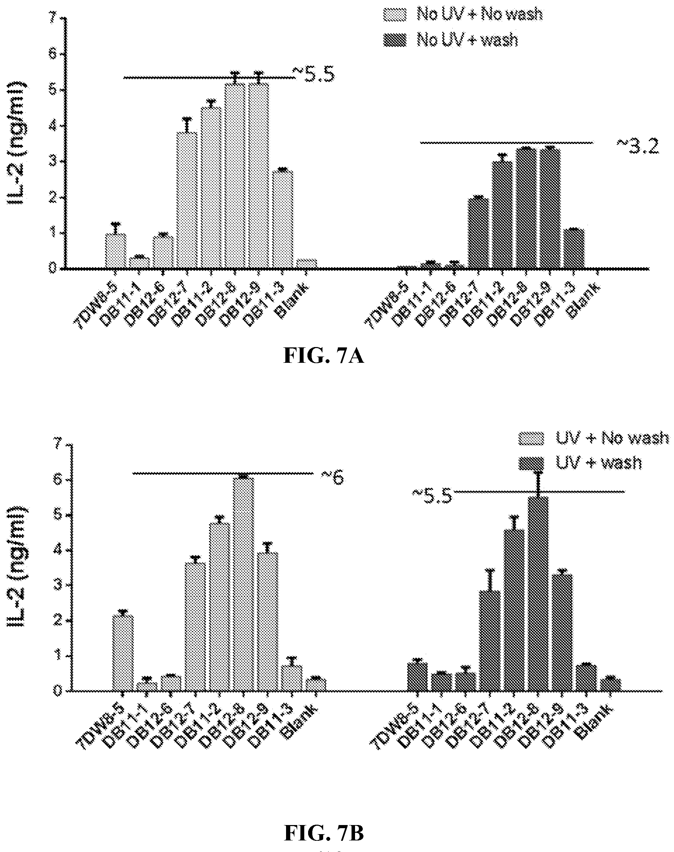

The results are shown in FIGS. 7A-7B. The results of the iNKT stimulation assay following UV crosslinking show that DB12-8 not only bound efficiently to CD1d, it also stimulated iNKT hybridoma better than other glycolipids. DB11-2 is also confirmed here as the second best stimulator of the iNKT hybridoma DN3A4-1.2.

### EXAMPLE 8

### Direct comparison of DB12-8 and 7DW8-5 complexes with mCD1d (detection by L363 ELISA)

A soluble recombinant mCDld protein (10µg/ml) was loaded with glycolipids (5 µM) overnight at 25°C in PBS pH8.2 with 0.05% tyloxapol. The mCD1d:GC complexes (10 µg/ml, 30 µl/well) were coated onto the high binding ELISA plates, overnight at 4°C. UV exposure and incubation to allow dissociation over 3 days was performed as described in Examples 6 and 7.

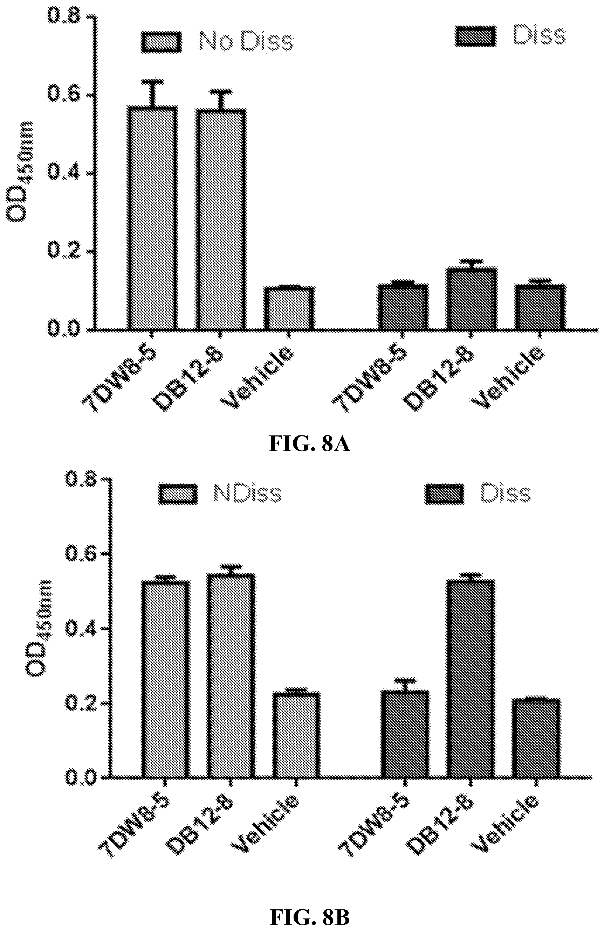

The results are shown in FIGS. 8A-8B. Both DB12-8 and 7DW8-5 showed affinity to CD1d and the complexes could be detected using L363 ELISA. Allowing extended dissociation for 3 days led to almost complete loss of signal for 7DW8-5 irrespective of UV-exposure. DB12-8 also dissociated almost completely but only in non UV-crosslinked state, UV exposure at 365 nm covalently coupled the mCD1d:DB12-8 complex and hence no dissociation was observed in this case.

### EXAMPLE 9

### Direct comparison of DB12-8 and 7DW8-5 complexes with mCD1d.CEA fusion protein (detection by L363 ELISA)

mCD1d.CEA (10 µg/ml), a recombinant mCD1d protein genetically fused with a single chain Fv antibody fragment specific for the tumor associated antigen CEA, was loaded with glycolipids (5 µM) overnight at 25°C in PBS pH8.2 with 0.05% tyloxapol. The mCD1d.CEA:GC complexes (10 µg/ml, 30 µl/well) were coated onto wells of high binding ELISA 96 well plates overnight at 4°C. UV exposure and incubation to allow dissociation over 3 days were performed as described in Examples 6 and 7.

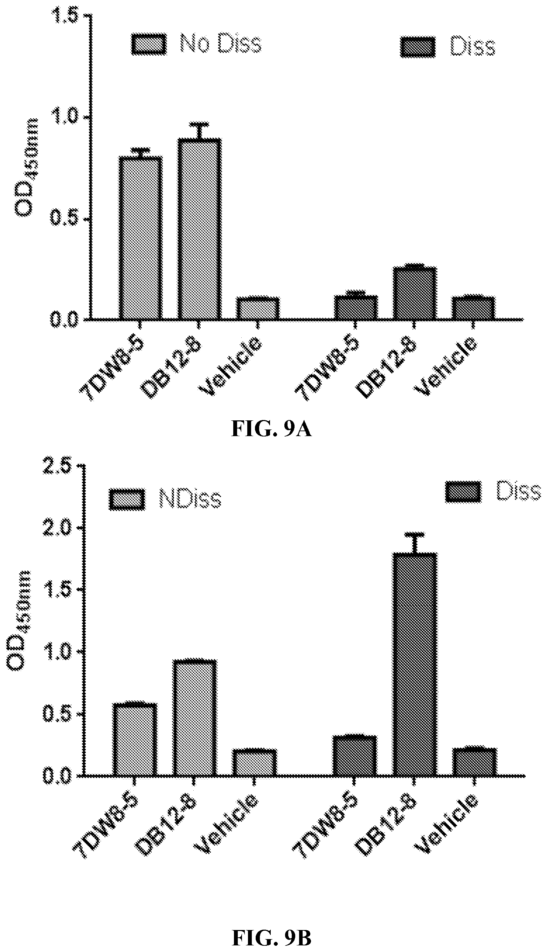

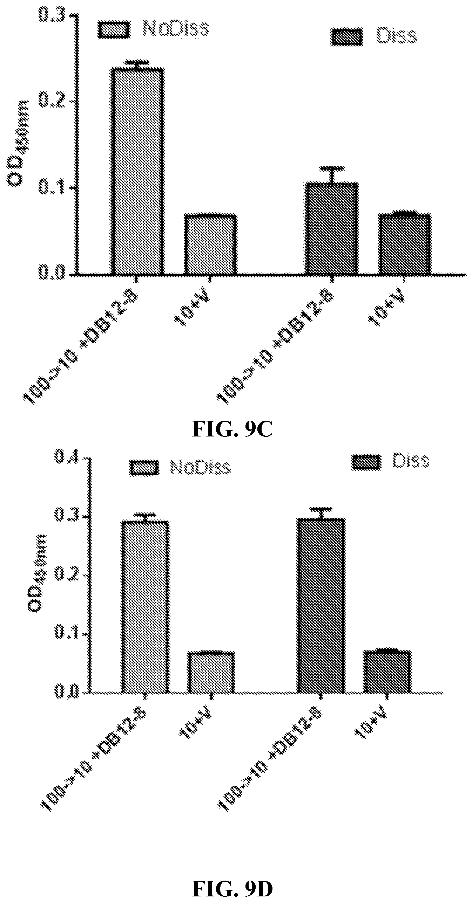

The results are shown in FIGS. 9A-9B. Both DB12-8 and 7DW8-5 showed affinity to CD1d and the complexes could be detected using L363 ELISA. Allowing extended dissociation for 3 days led to almost complete loss of signal for 7DW8-5 irrespective of UV-exposure. DB12-8 also almost completely dissociated but only in non UV-crosslinked state, UV exposure at 365 nm covalently coupled the mCD1d.CEA-DB12-8 complex and hence no dissociation was observed in this case. The results indicate that the DB12-8 signal increases significantly following UV-crosslinking.

A scale up of the complex formation in solution was performed using a 10-fold increased concentration of protein and glycolipid and the results are shown in FIG. 9C-9D. mCD1d.CEA (100 µg/ml) was loaded with DB12-8 (50 µM respectively) overnight at 25°C in PBS pH8.2 with 0.05% tyloxapol. DB12-8:mCD1d.CEA complexes were diluted down to 10 µg/ml and were coated onto the high binding ELISA plates (30 µl/well), overnight at 4°C. UV exposure and incubation to allow dissociation over 3 days were performed as described in Examples 6 and 7.

Incubation of non-crosslinked complexes for 3 days led to ∼75% decrease in OD₄₅₀ whereas there was no detectable reduction in OD₄₅₀ of UV-crosslinked samples after 3 days.

### EXAMPLE 10

### Direct comparison of cytokine release in vivo after injection of sCD1d-anti-CEA non-covalently loaded with aGalCer or UV cross-linked with DB12-8

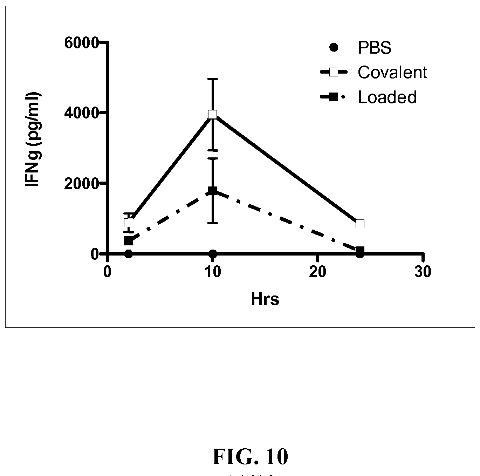

The kinetics and magnitude of interferon γ (IFNg) and Interleukin-2 (IL-2) cytokine release in serum of groups of 3 mice each was determined at 2, 10 and 24 hrs after injection with saline or with 30 µg of either αGalCer non-covalently loaded on sCD1d-anti-CEA or UV cross-linked DB12-8:sCD1d-anti-CEA complexes. The covalently-linked DB12-8 complex with sCD1d-anti-CEA is approximately twice as potent as the non-covalently loaded fusion protein in inducing the release of IFNg and IL-2. As shown in FIG. 10, the covalent fusion protein leads to a longer duration of IFNg release.

### EXAMPLE 11

### Sustained production of IFNγ upon repeated stimulation of iNKT cells with DB12-8 covalently-linked to sCD1d-anti-CEA fusion protein

An important biological property of stimulation with αGalCer-loaded sCDld is the sustained production of IFNg following repeated stimulation. This is in contrast to stimulation with free αGalCer which, as previously described, results in iNKT cell anergy (US Application Publication No. 2008/0254045A1; Stirnemann et al., J Clinical Invest. 118:994-1005, 2008; each of which is herein incorporated by reference in its entirety). It was important to confirm that this biological property is retained by covalently linked αGalCer-CD1d complexes. Mice from the experiment described in Example 10 were further restimulated on day 2 and again on day 8 with the same agent as at the initiation of the experiment. Mice were sacrificed 1h after the third injection and spleen iNKT cells were stained for intracellular IFNg with anti-IFNg-APC after fixation and permeabilization with Cytofix/Cytoperm (BD). To determine the percentage of iNKT that produce IFNg, iNKT cells were gated on positive staining for both CD3-FITC and αGalCer-loaded CD1d tetramer-PE and scored for expression of intracellular IFNg by flow cytometry on a FACS Calibur.

As shown in FIG. 11 for representative mice, less than 1% of iNKT cells of control PBS-treated mice stained for IFNg, 31% of iNKT cells of mice stimulated with non-covalently associated αGalCer-loaded sCD1d-anti-CEA, and 46% of iNKT cells of mice stimulated with UV cross-linked DB12-8:sCD1d-anti-CEA complexes stained for intracellular IFNg. These results demonstrate that production of IFNg by iNKT cells is at least as sustained following repeated stimulation with UV cross-linked DB12-8:sCD1d-anti-CEA as with non-covalently associated αGalCer-loaded sCD1d-anti-CEA complexes. Importantly, the increased potency of the covalent fusion protein was not associated with any apparent toxicity even after multiple injections.

### EXAMPLE 12

### In vivo anti-tumor activity of DB12-8 covalently-linked to sCD1d-anti-CEA fusion protein and non-covalently loaded αGC/sCD1d-anti-CEA fusion protein.

Four groups of C57BL/6 mice or C57BL/6 mice transgenic for CEA are grafted s.c. in the flank with 7x10⁵ MC38 tumor cells that have been stably transfected with human CEA (MC38-CEA). When tumors have grown to approximately 100 mm³, mice are treated with i.v. injections of either PBS (control), or equimolar amounts of aGalCer (0.4 µg), αGalCer non-covalently loaded onto sCD1d-anti-CEA fusion protein (40 µg), or DB12-8 covalently-linked to sCD1d-anti-CEA fusion protein (40 µg) each in 200 µl volume. Treatment is repeated every 4-5 days for a total of 5 injections. Mean tumor volume is measured every two days using the formula (length x width x thickness)/2. The kinetics of tumor growth (mm3) is determined as the mean of all mice in each group.

### EXAMPLE 13

### In vivo MC38-CEA tumor growth inhibition with α-galactosylceramide covalently linked to sCD1d-anti-CEA fusion protein.

Three groups of seven age and sex matched C57BL/6 mice were grafted with MC38 tumor cells that have been transfected with cDNA encoding carcino-embryonic antigen (CEA). Mice were treated on days 7, 11, 15 and 20 post-graft with either PBS, 3 µg of free α-galactosylceramide (αGalCer), or the molar equivalent of αGalCer covalently linked to the sCD1d-anti-CEA fusion protein (30 µg). Tumor growth was measured every 2 or 3 days by calipers and measurements were used to calculate tumor volume using the formula (length x width x thickness)/2. Animals were sacrificed on day 24.

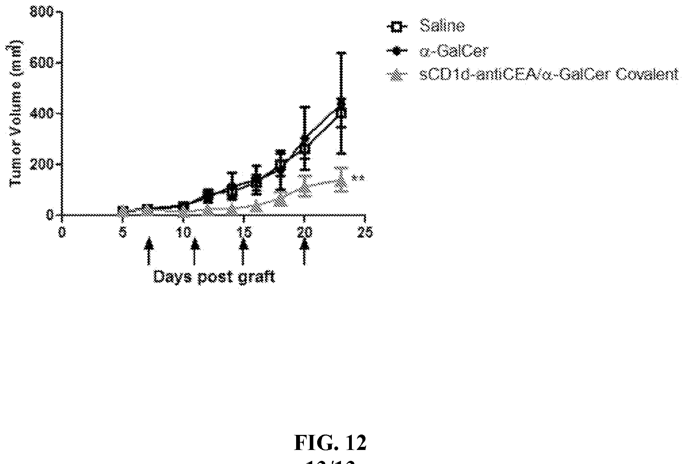

As shown in FIG. 12, treatment with αGalCer covalently linked to sCD1d-anti-CEA was significantly more effective at inhibiting tumor growth than treatment with PBS or αGalCer. These results were significant as determined by Two-way Analysis of Variance (ANOVA) (p=0.0079).

## Claims

1. A ceramide-like glycolipid comprising a photoreactive group, wherein:
- said photoreactive group is a benzophenone group covalently bound to the N-acyl lipophilic moiety of said ceramide-like glycolipid; and
- said ceramide-like glycolipid is an α-galactosylceramide;
wherein said ceramide-like glycolipid is capable of covalently binding to CD1d and enhancing the activity of natural killer (NKT) cells.

2. The ceramide-like glycolipid of claim 1, wherein said N-acyl lipophilic moiety is a linear acyl chain, and wherein said linear acyl chain optionally has at least 11 atoms, optionally between 11 and 14 atoms, wherein the numbering of atoms excludes hydrogens.

3. The ceramide-like glycolipid of claim 2, wherein said acyl chain is selected from the group consisting of: 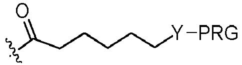 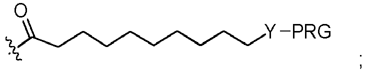 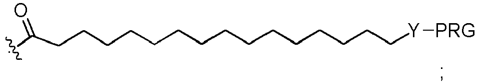 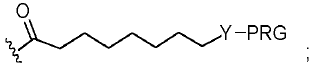 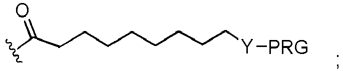 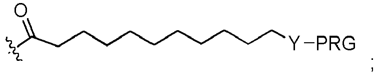 and 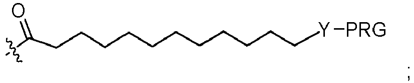 wherein Y is -O-, -CH₂-, -S-, -OCH₂-, -SCH₂-, -CH₂CH₂-; or a bond, and PRG is said photoreactive group.

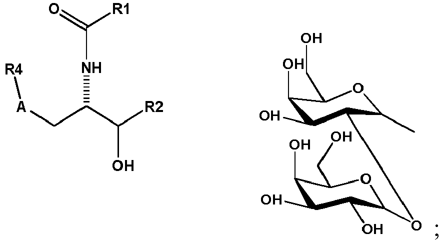

4. The ceramide-like glycolipid of claim 1, wherein said α-galactosylceramide is selected from the group consisting of:
a) Formula II: 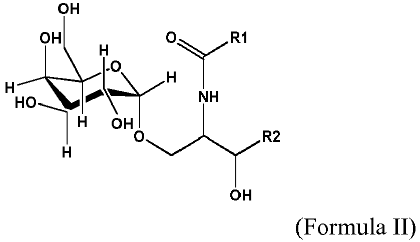 wherein
R1 is a linear or branched C₁-C₂₇ alkane or C₂-C₂₇ alkene; R1 is -C(OH)-R3; or wherein R1 is selected from the group consisting of: (CH₂)₉CH=CH-CH₂-CH=CH(CH₂)₄CH₃, (CH₂)₈CH=CH-CH₂-CH=CH(CH₂)₄CH₃, (CH₂)₇CH=CH-CH₂-CH=CH(CH₂)₄CH₃, (CH₂)₃CH=CH-CH₂-CH=CH-CH₂-CH=CH-CH₂-CH=CH-(CH₂)₄CH₃, (CH₂)₃CH=CH-CH₂-CH=CH-CH₂-CH=CH-CH₂-CH=CH-CH₂-CH=CH-CH₂CH₃,(CH₂)₇CH=CH-CH₂-CH=CH=(CH₂)₄CH₃, (CH₂)₇CH=CH-CH=CH(CH₂)₅CH₃, (CH₂)₈CH=CH-CH=CH(CH₂)₄CH₃, (CH₂)₉CH=CH-CH=CH(CH₂)₅CH₃, (CH₂)₆CH=CH-CH=CH-CH=CH(CH₂)₄CH₃, (CH₂)₆CH=CH-CH=CH-CH=CH(CH₂)₄CH₃ and (CH₂)₇CH=CH-CH=CH-CH=CH(CH₂)₃CH₃;
wherein R3 is linear or branched C₁-C₂₆ alkane or C₂-C₂₆ alkene; and
R2 is one of the following (a)-(e):
(a) -CH₂(CH₂)ₓCH₃,
(b) -CH(OH)(CH₂)ₓCH₃,
(c) -CH(OH)(CH₂)ₓCH(CH₃)₂,
(d) -CH=CH(CH₂)ₓCH₃,
(e) -CH(OH)(CH₂)ₓCH(CH₃)CH₂CH₃,
wherein X is an integer ranging from 4-17,
wherein R2 is optionally -CH(OH)(CH₂)ₓCH₃, wherein X is an integer ranging from 4-13,
wherein R2 is optionally -CH(OH)-(CH₂)₁₃CH₃;
b) Formula III: 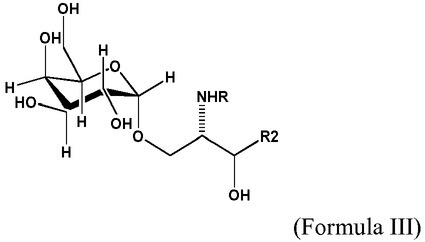 wherein R is -C(O)R1, wherein R1 is a linear or branched C₁-C₂₇ alkane or C₂-C₂₇ alkene; or R1 is -C(OH)-R3 wherein R3 is a linear or branched C₁-C₂₆ alkane or C₂-C₂₆ alkene; or R1 is a C₆-C₂₇ alkane or alkene wherein (i) the C₆-C₂₇ alkane or alkene is substituted with a C₅-C₁₅ cycloalkane, C₅-C₁₅ cycloalkene, heterocycle, or aromatic ring or (ii) the C₆-C₂₇ alkane or alkene includes, within the C₆-C₂₇ alkyl or alkenyl chain, a C₅-C₁₅ cycloalkane, C₅-C₁₅ cycloalkene, heterocycle, or aromatic ring; or R1 is an optionally substituted aromatic ring, or an aralkyl, or R1 is selected from the group consisting of: 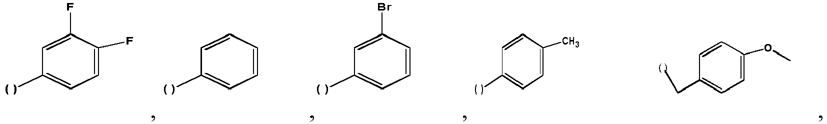 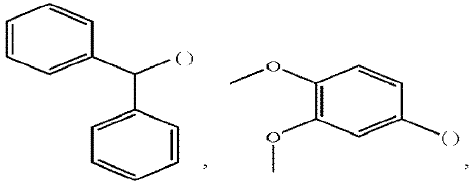 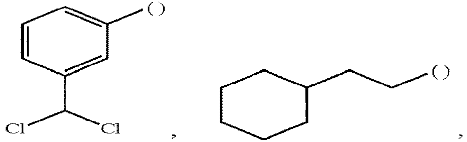 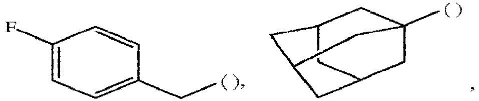 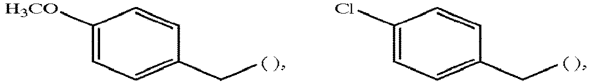 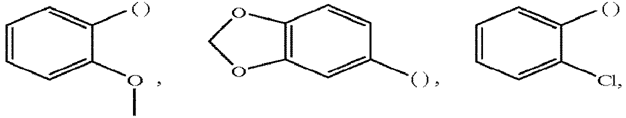 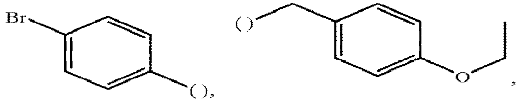 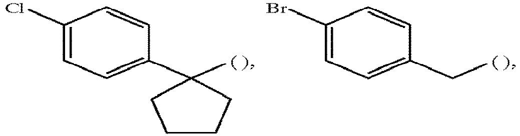 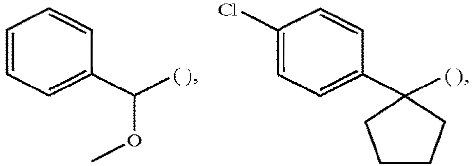 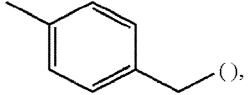 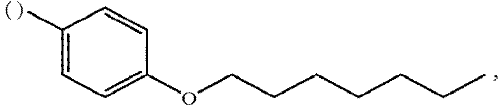 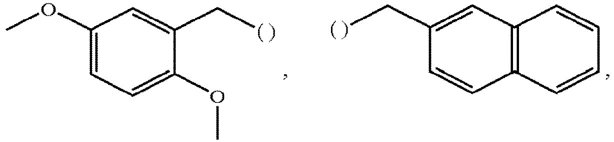 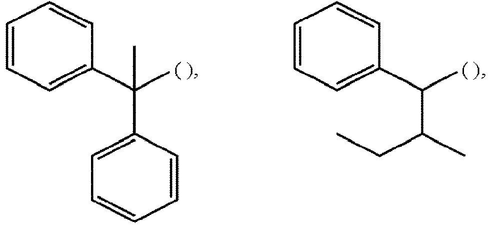 and 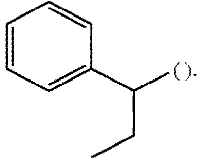
where ( ) represent the point of attachment of R1 to the compound of Formula III; and
R2 is one of the following (a)-(e):
(a) -CH₂(CH₂)ₓCH₃,
(b) -CH(OH)(CH₂)ₓCH₃,
(c) -CH(OH)(CH₂)ₓCH(CH₃)₂,
(d) -CH=CH(CH₂)ₓCH₃,
(e) -CH(OH)(CH₂)ₓCH(CH₃)CH₂CH₃,
wherein X is an integer ranging from 4-17,
wherein optionally R1 is substituted with oxo; hydroxy; halogen; phenyl; - OC(O)R6; -OR6; -C(O)R6; or N(R6)₂,
wherein each R6 is independently a C₁-C₆ substituted alkyl, or a substituted aromatic ring optionally substituted with halogen; hydroxy; -OC(O)R7; -OR7; -C(O)R7 or N(R7)₂, and
wherein each R7 is a substituted C₁-C₆ alkyl; and
c) (2S, 3S, 4R)-1-O-(α-D-galactopyranosyl)-N-hexacosanoyl-2-amino-1,3,4-octadecanetriol (KRN7000), (2S,3S)-1-O-(α-D-galactopyranosyl)-N-hexacosanoyl-2-amino-1,3-octadecanediol), or (2S, 3S, 4R)-1-CH₂-(α-galactopyranosyl)-N-hexacosanoyl-2-amino-1,3,4-octadecanetriol (α-C-GalCer).

5. The ceramide-like glycolipid of claim 1, wherein said ceramide-like glycolipid has the structure selected from the group consisting of:
a) 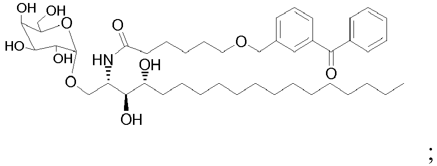
b) 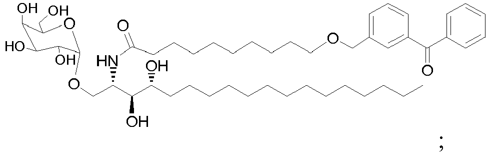
c) 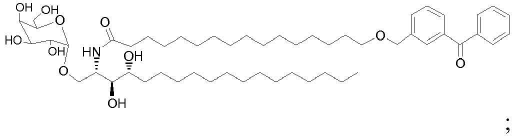
d) 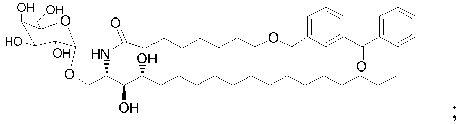
e) 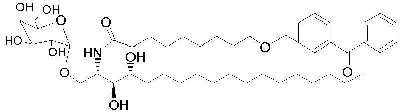 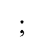
f) 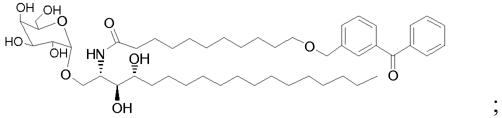 and
g)

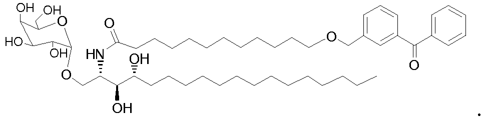

6. A ceramide-like glycolipid/protein complex comprising a CD1d protein and the ceramide-like glycolipid of any one of claims 1-6, wherein said ceramide-like glycolipid is covalently linked to said protein via photoactivation of the benzophenone group.

7. The ceramide-like glycolipid/protein complex of claim 6, wherein said CDld:
a) has an amino acid sequence selected from the group consisting of:
i) an amino acid sequence at least 90% identical to amino acids 21 to 295 of SEQ ID NO:1;
ii) the amino acid sequence set forth as amino acids 21 to 295 of SEQ ID NO:1;
iii) an amino acid sequence identical to amino acids 21 to 295 of SEQ ID NO:1, except for at least one but less than 10 conservative amino acid substitutions;
iv) the amino acid sequence set forth as amino acids 1 to 295 of SEQ ID NO:1;
v) an amino acid sequence at least 90% identical to amino acids 1 to 295 of SEQ ID NO:1;
vi) an amino acid sequence identical to amino acids 21 to 295 of SEQ ID NO:1, except for at least one but less than 10 conservative amino acid substitutions;
vii) the amino acid sequence set forth in SEQ ID NO:1;
viii) an amino acid sequence at least 90% identical to SEQ ID NO:1; and
ix) an amino acid sequence identical to SEQ ID NO:1, except for at least one but less than 10 conservative amino acid substitutions; or
b) is physically associated with a β2-microglobulin,
wherein said β2-microglobulin optionally has an amino acid sequence selected from the group consisting of:
i) an amino acid sequence at least 90% identical to amino acids 21 to 113 of SEQ ID NO:2;
ii) the amino acid sequence set forth as amino acids 21 to 113 of SEQ ID NO:2;
iii) an amino acid sequence identical to amino acids 21 to 113 of SEQ ID NO:2, except for at least one but less than 10 conservative amino acid substitutions;
iv) the amino acid sequence set forth in SEQ ID NO:2;
v) an amino acid sequence at least 90% identical to SEQ ID NO:2; and
vi) an amino acid sequence identical to SEQ ID NO:2, except for at least one but less than 10 conservative amino acid substitutions.

8. The ceramide-like glycolipid/protein complex of claim 6 or 7, wherein said ceramide-like glycolipid/protein complex:
a) further comprises an antibody or fragment thereof specific for a target antigen, and wherein said antibody or antigen-binding fragment thereof is linked to said CD1d,
wherein said antigen-binding fragment is optionally selected from the group consisting of a F(ab) fragment, a F(ab')2 fragment, and a single-chain antibody,
wherein said target antigen is optionally a cell surface marker of tumor cells,
wherein said cell surface marker is optionally selected from the group consisting of CEA, Her2/neu, EGFR type I or type II, CD19, CD20, CD22, Muc-1, PSMA, or STEAP,
wherein said CD1d is optionally attached to the heavy chain or light chain of said antibody or heavy chain fragment or light chain fragment of said antigen-binding antibody fragment; or
b) further comprises an antigen,
wherein said antigen is optionally an immunogenic polypeptide of a virus or a cell selected from the group consisting of a virus, bacterium, fungus, and tumor cell.

9. A composition comprising the ceramide-like glycolipid/protein complex of any one of claims 6-8, and a pharmaceutical carrier,
wherein said pharmaceutical carrier is optionally selected from the group consisting of saline, buffered saline, dextrose, water, glycerol, and combinations thereof.

10. The ceramide-like glycolipid/protein complex of any one of claims 6-8 or the composition of claim 9 for use in a method of treating or preventing a disease in a subject, said method comprising administering to a subject in need of said treatment or prevention the ceramide-like glycolipid/protein complex or composition, wherein said ceramide-like glycolipid/protein complex is administered in an amount sufficient to alter the progression of said disease
wherein an immune response is optionally enhanced or modified relative to an immune response produced by the protein not covalently linked to the glycolipid,
wherein said disease is optionally selected from the group consisting of a viral disease, a bacterial disease, a fungal disease, a parasitic disease, a proliferative disease, an autoimmune disease, an inflammatory disease, tuberculosis, pulmonary disease resembling tuberculosis, lymphadenitis, skin disease, disseminated disease, bubonic plague, pneumonic plague, tularemia, Legionairre's disease, anthrax, typhoid fever, paratyphoid fever, foodborne illness, listeriosis, malaria, HIV, SIV, HPV, RSV, influenza, hepatitis (HAV, HBV, and HCV), multiple sclerosis, diabetes, Sjogren's Syndrome, and cancer;
wherein said subject is optionally a mammal, and wherein said mammal is optionally a human.

11. The ceramide-like glycolipid/protein complex of any one of claims 6-8 or the composition of claim 9 for use in a method of inducing an immune response against an antigen in a subject, said method comprising administering to said subject the ceramide-like glycolipid/protein complex or composition
wherein said ceramide-like glycolipid/protein complex optionally comprises said antigen,
wherein said antigen is optionally covalently bound to said protein,
wherein said ceramide-like glycolipid/protein complex is optionally administered in an amount sufficient to enhance the activity of Natural Killer T (NKT) cells in said subject,
wherein said immune response optionally comprises an antibody response,
wherein said subject is optionally a mammal, and wherein said mammal is optionally a human.

12. A heterologous antigen and the ceramide-like glycolipid/protein complex of any one of claims 6-8 or the composition of claim 9 for use in a method for enhancing an immune response in a subject to the heterologous antigen, said method comprising administering to said subject the heterologous antigen and the ceramide-like glycolipid/protein complex or composition simultaneously, prior to, or subsequent to the administration of said heterologous antigen
wherein said subject is optionally a mammal, and wherein said mammal is optionally a human.

13. A method for synthesizing a ceramide-like glycolipid of Formula (VI) comprising a benzophenone group, said method comprising the steps of:
activating a carboxylic acid derivative of Formula (IV) to form an activated acyl derivative 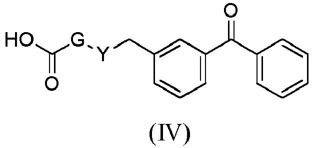 ; and
coupling the activated acyl derivative with a ceramide-like-glycolipid of Formula (V), wherein said ceramide-like glycolipid is capable of covalently binding to CD1d and enhancing the activity of natural killer T (NKT) cells 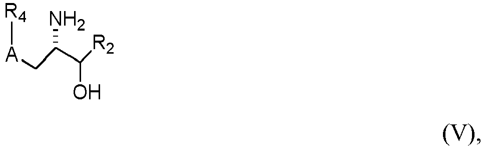
thereby forming the ceramide-like glycolipid comprising a benzophenone group of Formula (VI) 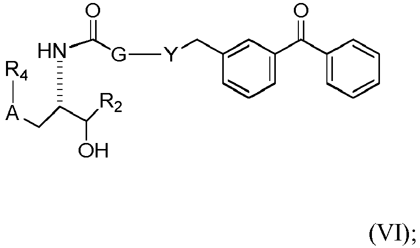 wherein
G is a linear or branched C₁-C₂₇ alkane or C₂-C₂₇ alkene; or G is -C(OH)-R3 wherein R3 is a linear or branched C₁-C₂₆ alkane or C₂-C₂₆ alkene; or R1 is a C₆-C₂₇ alkane or alkene wherein (i) the C₆-C₂₇ alkane or alkene is substituted with a C₅-C₁₅ cycloalkane, C₅-C₁₅ cycloalkene, heterocycle, or aromatic ring or (ii) the C₆-C₂₇ alkane or alkene includes, within the C₆-C₂₇ alkyl or alkenyl chain, a C₅-C₁₅ cycloalkane, C₅-C₁₅ cycloalkene, heterocycle, or aromatic ring; or R1 is an optionally substituted aromatic ring, or an aralkyl;
R₄ is an α-galactosyl;
A is -O-, -CH₂-; or is a single bond,
Y is -O-, -CH₂- or -S-, and
R₂ is a substituted or an unsubstituted C3-C40 alkyl, alkenyl or alkynyl chain,
wherein R2 of Formula (VI) is optionally one of the following (a)-(e):
(a) -CH₂(CH₂)ₓCH₃,
(b) -CH(OH)(CH₂)ₓCH₃,
(c) -CH(OH)(CH₂)ₓCH(CH₃)₂,
(d) -CH=CH(CH₂)ₓCH₃,
(e) -CH(OH)(CH₂)ₓCH(CH₃)CH₂CH₃, wherein x is an integer ranging from 0-40, or
wherein R2 of Formula (VI) is optionally:
-CH(OH)(CH₂)ₓCH₃, wherein x is an integer ranging from 4-17; or
-CH(OH)(CH₂)₁₃CH₃; or
wherein optionally G is -(CH₂)n-, wherein n is an integer ranging from 0-20 and Y is O; or
wherein:
G is -(CH₂)n-, wherein n is an integer ranging from 0-20 and Y is O;
R2 is -CH(OH)(CH₂)ₓCH₃, wherein x is an integer ranging from 4-17;
A is O;
Y is O; and
wherein said ceramide-like glycolipid is capable of covalently binding to CD1d and enhancing the activity of natural killer T (NKT) cells.

14. A method for producing a ceramide-like glycolipid/protein complex, said method comprising the steps of:
a) contacting the ceramide-like glycolipid of any one of claims 1-5 or a ceramide-like glycolipid produced by the method of claim 13 with a CD1d protein; and
b) irradiating said ceramide-like glycolipid and said CD1d protein with ultraviolet light to produce a ceramide-like glycolipid/protein complex,
wherein said modified glycolipid/protein complex enhances the activity of natural killer T (NKT) cells.

## Patentansprüche

1. Ceramidartiges Glykolipid, das eine photoreaktive Gruppe umfasst, wobei:
- die photoreaktive Gruppe eine Benzophenongruppe ist, die kovalent an die lipophile N-Acyl-Gruppe des ceramidartigen Glykolipids gebunden ist; und
- das ceramidartige Glykolipid ein α-Galactosylceramid ist;
wobei das ceramidartige Glykolipid zur kovalenten Bindung an CD1d und zur Steigerung der Aktivität von natürlichen Killerzellen (NKT-Zellen) fähig ist.

2. Ceramidartiges Glykolipid nach Anspruch 1, wobei der lipophile N-Acylrest eine lineare Acylkette ist und wobei die lineare Acylkette optional mindestens 11 Atome, optional zwischen 11 und 14 Atome aufweist. wobei die Anzahl der Atome Wasserstoffatome ausschließt.

3. Ceramidartiges Glykolipid nach Anspruch 2, wobei die Acylkette ausgewählt ist aus der Gruppe bestehend aus: 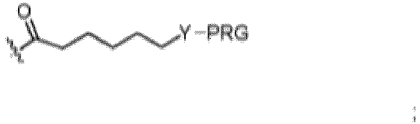 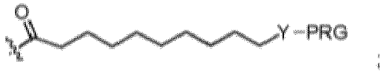 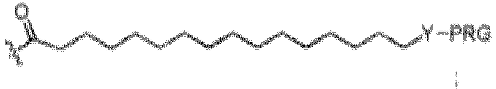 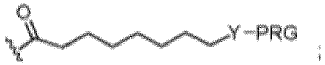 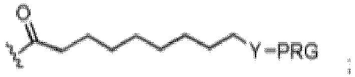 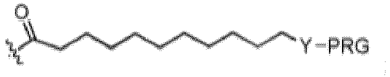 und 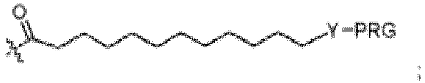 wobei Y -O-, -CH₂-, -S-, -OCH₂-, -SCH₂-, -CH₂CH₂- ist; oder eine Bindung, und PRG die photoreaktive Gruppe ist.

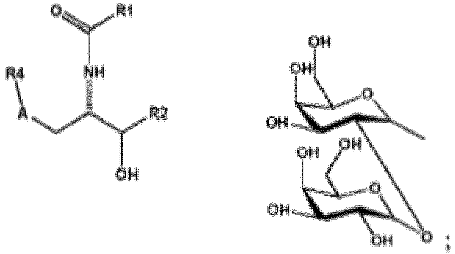

4. Ceramidartiges Glykolipid nach Anspruch 1, wobei das a-Galactosylceramid ausgewählt ist aus der Gruppe bestehend aus:
a) Formel II: 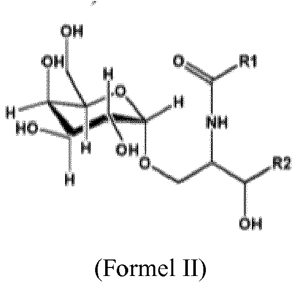 wobei
R1 ein lineares oder verzweigtes C₁-C₂₇-Alkan oder C₂-C₂₇-Alken ist; R1 -C(OH)-R3 ist; oder wobei
R1 ausgewählt ist aus der Gruppe bestehend aus: (CH₂)₉CH=CH-CH₂-CH=CH(CH₂)₄CH₃, (CH₂)₈CH=CH-CH₂-CH=CH(CH₂)₄CH₃, (CH₂)₇CH=CH-CH₂-CH=CH(CH₂)₄CH₃, (CH₂)₃CH=CH-CH₂-CH=CH-CH₂-CH=CH-CH₂-CH=CH-(CH₂)₄CH₃, (CH₂)₃CH=CH- CH₂-CH=CH-CH₂-CH=CH-CH₂-CH=CH-CH₂-CH=CH-CH₂CH₃,(CH₂)₇CH=CH-CH₂- CH=CH=(CH₂)₄CH₃, (CH₂)₇CH=CH-CH=CH(CH₂)₅CH₃, (CH₂)₈CH=CH- CH=CH(CH₂)₄CH₃, (CH2)9CH=CH-CH=CH(CH₂)₅CH₃, (CH₂)₆CH=CH-CH=CH- CH=CH(CH₂)₄CH₃, (CH₂)₆CH=CH-CH=CH-CH=CH(CH₂)₄CH₃ und (CH₂)₇CH=CH- CH=CH-CH=CH(CH₂)₃CH₃;
wobei R3 lineares oder verzweigtes C₁-C₂₆-Alkan oder C₂-C₂₆-Alken ist; und
R2 eines der folgenden (a) - (e) ist:
(a) -CH₂(CH₂)^{x}CH₃,
(b) -CH(OH)(CH₂)^{x}CH₃,
(c) -CH(OH)(CH₂)^{x}CH(CH₃)₂,
(d) -CH=CH(CH₂)^{x}CH₃,
(e) -CH(OH)(CH₂)^{x}CH(CH₃)CH₂CH₃,
wobei X eine ganze Zahl im Bereich von 4-17 ist,
wobei R2 optional -CH(OH)(CH₂)xCH₃ ist, wobei X eine ganze Zahl im Bereich von 4-13 ist,
wobei R2 optional -CH(OH)-(CH₂)₁₃CH₃ ist;
b) Formel III: 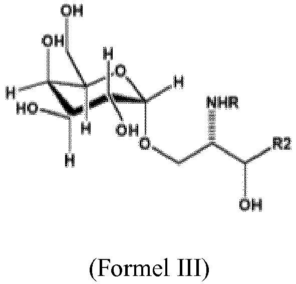 wobei R -C(O)R1 ist, wobei R1 ein lineares oder verzweigtes C₁-C₂₇-Alkan oder C₂-C₂₇-Alken ist; oder R1 -C(OH)-R3 ist, wobei R3 ein lineares oder verzweigtes C₁-C₂₆-Alkan oder C₂-C₂₆-Alken ist; oder R1 ein C₆-C₂₇-Alkan oder Alken ist, wobei (i) das C₆-C₂₇-Alkan oder Alken mit einem C₅-C₁₅-Cycloalkan, C₅-C₁₅-Cycloalken, Heterocyclus oder aromatischen Ring substituiert ist oder (ii) das C₆-C₂₇-Alkan oder Alken innerhalb der C₆-C₂₇-Alkyl- oder Alkenylkette ein C₅-C₁₅-Cycloalkan, C₅-C₁₅-Cycloalken, einen Heterocyclus oder einen aromatischen Ring umfasst; oder R1 ein optional substituierter aromatischer Ring oder ein Aralkyl ist oder R1 aus der Gruppe ausgewählt ist, die aus Folgendem besteht: 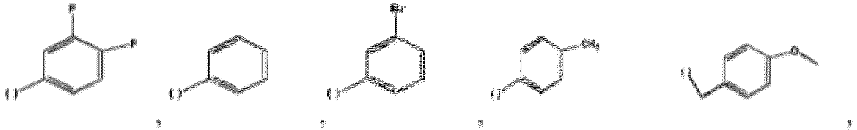 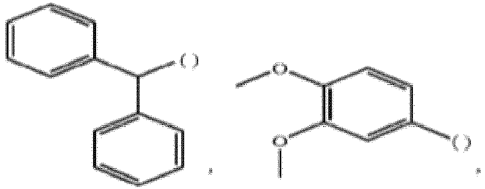 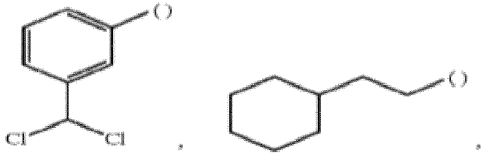 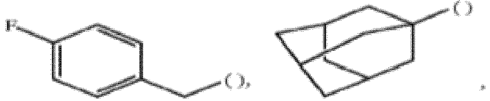 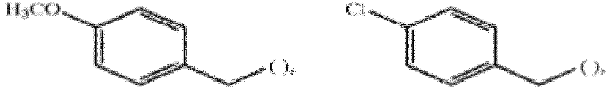 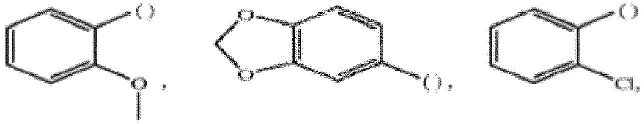 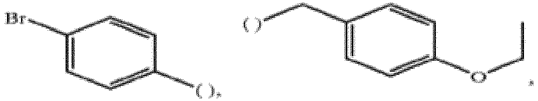 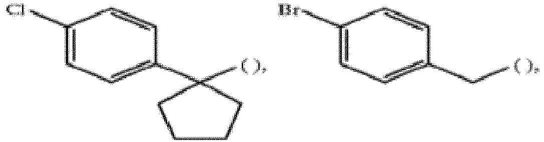 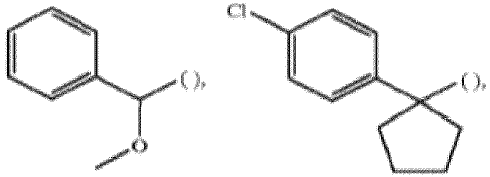 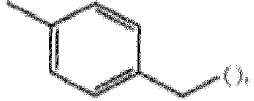 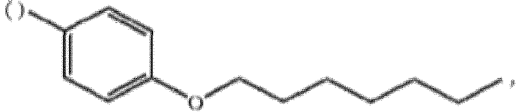 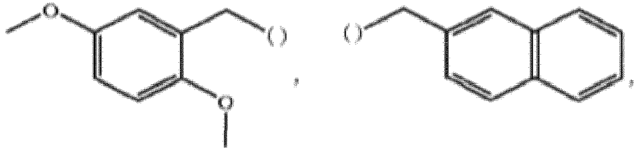 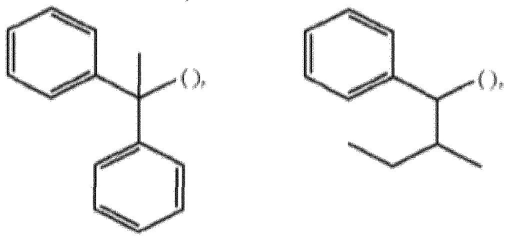 and 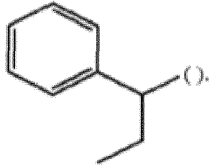
wobei () den Bindungspunkt von R1 an die Verbindung der Formel III darstellt; und
R2 eines der folgenden (a) - (e) ist:
(a) -CH₂(CH₂)ₓCH₃,
(b) -CH(OH)(CH₂)ₓCH₃,
(c) -CH(OH)(CH₂)ₓCH(CH₃)₂,
(d) -CH=CH(CH₂)ₓCH₃,
(e) -CH(OH)(CH₂)ₓCH(CH₃)CH₂CH₃,
wobei X eine ganze Zahl im Bereich von 4-17 ist,
wobei optional R1 mit Oxo; Hydroxy; Halogen; Phenyl; -OC(O)R6; -OR6;-C(O)R6; oder N(R6)₂ substituiert ist,
wobei jedes R6 unabhängig ein C₁-C₆-substituiertes Alkyl oder ein substituierter aromatischer Ring ist, der optional mit Halogen; Hydroxy; -OC(O)R7; -OR7; -C(O)R7 oder N(R7)₂ substituiert ist und
wobei jedes R7 ein C₁-C₆-Alkyl ist; und
c) (2S, 3S, 4R)-1-O-(α-D-Galactopyranosyl)-N-hexacosanoyl-2-amino-1,3,4-octadecanetriol (KRN7000), (2S,3S)-1-O-(α-D-Galactopyranosyl)-N-hexacosanoyl-2-amino-1,3-octadecanediol) oder (2S, 3S, 4R)-1-CH₂-(α-Galactopyranosyl)-N-hexacosanoyl-2-amino-1,3,4-octadecanetriol (α-C-GalCer).

5. Ceramidartiges Glykolipid nach Anspruch 1, wobei das ceramidartige Glykolipid die Struktur hat, die aus der Gruppe ausgewählt ist, die aus Folgendem besteht:
a) 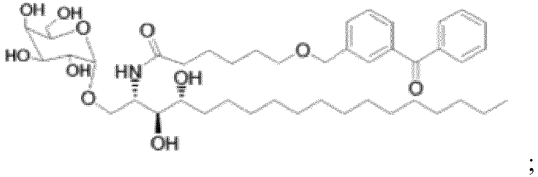
b) 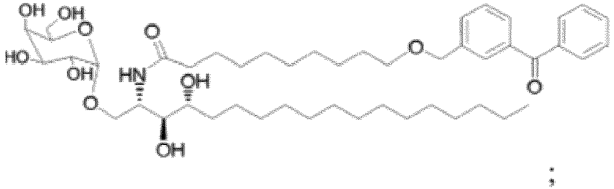
c) 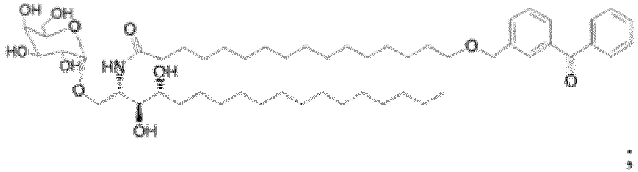
d) 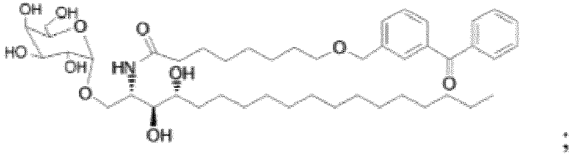
e) 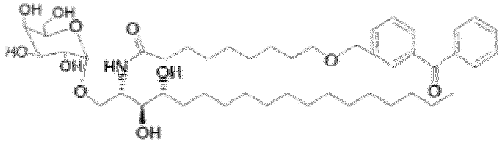
f) 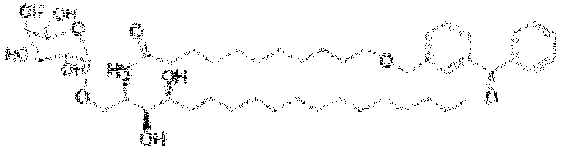 und
g)

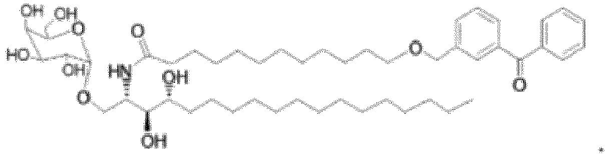

6. Ceramidartiger Glykolipid-/Protein-Komplex, der ein CD1d-Protein umfasst und das ceramidartige Glykolipid nach einem der Ansprüche 1 bis 6, wobei das ceramidartige Glykolipid über Photoaktivierung der Benzophenongruppe kovalent an das Protein gebunden ist.

7. Ceramidartiger Glykolipid-/Protein-Komplex nach Anspruch 6, wobei die CD1d:
a) eine Aminosäuresequenz umfasst, die aus der Gruppe ausgewählt wird, die aus Folgendem besteht:
i) eine Aminosäuresequenz, die zu mindestens 90 % mit den Aminosäuren 21 bis 295 von SEQ ID NO: 1 identisch ist;
ii) die Aminosäuresequenz, die als Aminosäuren 21 bis 295 von SEQ ID NO: 1 dargestellt ist;
iii) eine Aminosäuresequenz, die mit den Aminosäuren 21 bis 295 von SEQ ID NO: 1 identisch ist, mit Ausnahme von mindestens einer, jedoch weniger als 10 konservativen Aminosäuresubstitutionen;
iv) die Aminosäuresequenz, die als Aminosäuren 1 bis 295 von SEQ ID NO: 1 dargestellt ist;
v) eine Aminosäuresequenz, die zu mindestens 90 % mit den Aminosäuren 1 bis 295 von SEQ ID NO: 1 identisch ist;
vi) eine Aminosäuresequenz, die mit den Aminosäuren 21 bis 295 von SEQ ID NO: 1 identisch ist, mit Ausnahme von mindestens einer, jedoch weniger als 10 konservativen Aminosäuresubstitutionen;
vii) die Aminosäuresequenz, die in SEQ ID NO: 1 angegeben ist,
viii) eine Aminosäuresequenz, die mindestens zu 90 % mit SEQ ID NO: 1 identisch ist; und
ix) eine Aminosäuresequenz, die mit SEQ ID NO: 1 identisch ist, mit Ausnahme von mindestens einer jedoch weniger als 10 konservativen Aminosäuresubstitutionen; oder
b) physikalisch einem β2-Mikroglobulin zugeordnet ist,
wobei das β2-Mikroglobulin optional eine Aminosäuresequenz aufweist, die aus der Gruppe ausgewählt wird, die aus Folgendem besteht:
i) eine Aminosäuresequenz, die zu mindestens 90 % mit den Aminosäuren 21 bis 113 von SEQ ID NO: 2 identisch ist;
ii) die Aminosäuresequenz, die als Aminosäuren 21 bis 113 von SEQ ID NO: 2 dargestellt ist;
iii) eine Aminosäuresequenz, die mit den Aminosäuren 21 bis 113 von SEQ ID NO: 2 identisch ist, mit Ausnahme von mindestens einer, jedoch weniger als 10 konservativen Aminosäuresubstitutionen;
iv) die Aminosäuresequenz, die in SEQ ID NO: 2 angegeben ist,
v) eine Aminosäuresequenz, die mindestens zu 90 % mit SEQ ID NO: 2 identisch ist; und
vi) eine Aminosäuresequenz, die mit SEQ ID NO: 2 identisch ist, mit Ausnahme von mindestens einer jedoch weniger als 10 konservativen Aminosäuresubstitutionen.

8. Ceramidartiger Glykolipid-/Protein-Komplex nach Anspruch 6 oder 7, wobei der ceramidartige Glykolipid-/Protein-Komplex
a) ferner einen Antikörper oder ein Fragment für ein spezifisches Zielantigen umfasst, und wenn der Antikörper oder das antigenbindende Fragment an die CD1d gebunden ist,
wobei das antigenbindende Fragment optional aus der Gruppe ausgewählt ist, die aus einem F(ab)-Fragment, einem F(ab')2-Fragment und einem einkettigen Antikörper besteht,
wobei das Zielantigen optional ein Zelloberflächenmarker von Tumorzellen ist,
wobei der Zelloberflächenmarker optional aus der Gruppe ausgewählt ist, die aus CEA, Her2/neu, EGFR Typ I oder Typ II, CD19, CD20, CD22, Muc-1, PSMA oder STEAP besteht,
wobei die CD1d optional an die schwere Kette oder leichte Kette des Antikörpers oder des schweren Kettenfragments oder des leichten Kettenfragments des antigenbindenden Antikörperfragments gebunden ist; oder
b) ferner ein Antigen umfasst,
wobei das Antigen optional ein immunogenes Polypeptid eines Virus oder einer Zelle ist, ausgewählt aus der Gruppe bestehend aus einem Virus, Bakterium, Pilz und Tumorzelle.

9. Zusammensetzung, die den ceramidartigen Glykolipid-/Protein-Komplex nach einem der Ansprüche 6 bis 8 und einen pharmazeutischen Träger umfasst,
wobei der pharmazeutische Träger optional aus der Gruppe ausgewählt ist, die aus Salzlösung, gepufferter Salzlösung, Dextrose, Wasser, Glycerol und Kombinationen davon besteht.

10. Ceramidartiger Glykolipid/Protein-Komplex nach einem der Ansprüche 6 bis 8 oder Zusammensetzung nach Anspruch 9 zur Verwendung in einem Verfahren zur Behandlung oder Vorbeugung einer Erkrankung bei einem Subjekt, wobei das Verfahren die Verabreichung des ceramidartigen Glykolipid/Protein-Komplexes oder der Zusammensetzung an ein Subjekt umfasst, das die Behandlung oder Vorbeugung benötigt, wobei der ceramidartige Glykolipid-/Protein-Komplex in einer Menge verabreicht wird, die ausreicht, um das Fortschreiten der Krankheit zu verändern,
wobei eine Immunreaktion optional verstärkt oder modifiziert wird im Vergleich zu einer Immunreaktion, die von dem Protein erzeugt wird, das nicht kovalent an das Glykolipid gebunden ist,
wobei die Krankheit optional aus der Gruppe ausgewählt ist, die aus Folgendem besteht: einer Viruserkrankung, einer bakteriellen Erkrankung, einer Pilzerkrankung, einer parasitären Erkrankung, einer proliferativen Erkrankung, einer Autoimmunerkrankung, einer entzündlichen Erkrankung, Tuberkulose, einer pulmonalen Erkrankung, die Tuberkulose ähnelt, Lymphadenitis, Hauterkrankung, disseminierter Erkrankung, Beulenpest, Lungenpest, Tularämie, Legionärskrankheit, Anthrax, Typhus, Paratyphus, lebensmittelbedingter Krankheit, Listeriose, Malaria, HIV, SIV, HPV, RSV, Influenza, Hepatitis (HAV, HBV und HCV), Multipler Sklerose, Diabetes, Sjögren-Syndrom und Krebs;
wobei das Subjekt optional ein Säuger ist, und wobei der Säuger optional ein Mensch ist.

11. Ceramidartiger Glykolipid-/Protein-Komplex nach einem der Ansprüche 6 bis 8 oder Zusammensetzung nach Anspruch 9 zur Verwendung in einem Verfahren zur Auslösung einer Immunantwort gegen ein Antigen in einem Subjekt, wobei das Verfahren die Verabreichung des ceramidartigen Glykolipid-/Protein-Komplexes oder der Zusammensetzung an das Subjekt umfasst,
wobei der ceramidartige Glykolipid-/Protein-Komplex optional das Antigen umfasst,
wobei das Antigen optional kovalent an das Protein gebunden ist,
wobei der ceramidartige Glykolipid-/Protein-Komplex optional in einer Menge verabreicht wird, die ausreicht, um die Aktivität von natürlichen Killer-T-Zellen (NKT-Zellen) in dem Subjekt zu erhöhen,
wobei die Immunantwort optional eine Antikörperantwort umfasst,
wobei das Subjekt optional ein Säuger ist, und wobei der Säuger optional ein Mensch ist.

12. Heterologes Antigen und der ceramidartige Glykolipid-/Protein-Komplex nach einem der Ansprüche 6 bis 8 oder die Zusammensetzung nach Anspruch 9 zur Verwendung in einem Verfahren zur Verstärkung einer Immunantwort bei einem Subjekt auf das heterologe Antigen, wobei das Verfahren die Verabreichung des heterologen Antigens und des ceramidartigen Glykolipid-/Protein-Komplexes oder der Zusammensetzung gleichzeitig, vor oder nach der Verabreichung des heterologen Antigens an das Subjekt umfasst,
wobei das Subjekt optional ein Säuger ist, und wobei der Säuger optional ein Mensch ist.

13. Verfahren zur Synthese eines ceramidartigen Glykolipids der Formel (VI), das eine Benzophenongruppe umfasst, wobei das Verfahren folgende Schritte umfasst:
Aktivieren eines Carbonsäurederivats der Formel (IV) zur Bildung eines aktivierten Derivatacyls 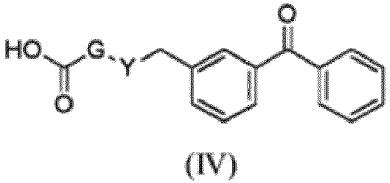 und
Koppeln des aktivierten Acylderivats mit einem ceramidartigen Glykolipid der Formel (V), wobei das ceramidartige Glykolipid zur kovalenten Bindung an CD1d und zur Steigerung der Aktivität von natürlichen Killer-T-Zellen (NKT-Zellen) fähig ist 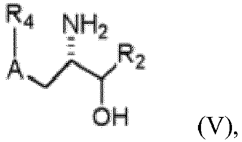
wodurch das ceramidartige Glykolipid gebildet wird, das die Benzophenongruppe der Formel (VI) umfasst 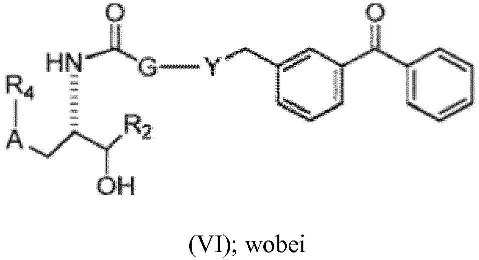
G ein lineares oder verzweigtes C₁-C₂₇-Alkan oder C₂-C₂₇-Alken ist; oder G-C(OH)-R3 ist, wobei R3 ein lineares oder verzweigtes C₁-C₂₆-Alkan oder C₂-C₂₆-Alken ist; oder R1 ein C₆-C₂₇-Alkan oder Alken ist, wobei (i) das C₆-C₂₇-Alkan oder Alken mit einem C₅-C₁₅-Cycloalkan, C₅-C₁₅-Cycloalken, Heterocyclus oder aromatischen Ring substituiert ist oder (ii) das C₆-C₂₇-Alkan oder Alken innerhalb der C₆-C₂₇-Alkyl- oder Alkenylkette ein C₅-C₁₅-Cycloalkan, C₅-C₁₅-Cycloalken, einen Heterocyclus oder einen aromatischen Ring umfasst; oder R1 ein optional substituierter aromatischer Ring oder ein Aralkyl;
R4 ein α-Galactosyl ist;
A -O-, -CH₂- oder eine Einfachbindung ist,
Y -O-, -CH₂- oder -S- ist und
R2 eine substituierte oder unsubstituierte C3-C40-Alkyl-, Alkenyl- oder Alkinylkette ist,
wobei R2 der Formel (VI) optional eines der folgenden (a) - (e) ist:
(a) -CH₂(CH₂)ₓCH₃,
(b) -CH(OH)(CH₂)ₓCH₃,
(c) -CH(OH)(CH₂)ₓCH(CH₃)₂,
(d) -CH=CH(CH₂)ₓCH₃,
(e) -CH(OH)(CH₂)ₓCH(CH₃)CH₂CH₃, wobei x eine ganze Zahl im Bereich von 0-40 ist oder
wobei R2 der Formel (VI) optional Folgendes ist:
-CH(OH)(CH₂)ₓCH₃, wobei x eine ganze Zahl im Bereich von 4-17 ist oder
-CH(OH)(CH₂)₁₃CH₃ oder
wobei G optional -(CH₂)n- ist, wobei n eine ganze Zahl im Bereich von 0-20 ist und Y O ist; oder
wobei:
G -(CH₂)n- ist, wobei n eine ganze Zahl im Bereich von 0-20 ist und Y O ist;
R2 -CH(OH)(CH₂)ₓCH₃ ist, wobei x eine ganze Zahl im Bereich von 4-17 ist;
A O ist;
Y O ist und
wobei das ceramidartige Glykolipid zur kovalenten Bindung an CD1d und zur Steigerung der Aktivität von natürlichen Killer-T-Zellen (NKT-Zellen) fähig ist.

14. Verfahren zur Herstellung eines ceramidartigen Glykolipid-/Protein-Komplexes, wobei das Verfahren folgende Schritte umfasst:
a) Kontaktieren des ceramidartigen Glykolipids nach einem der Ansprüche 1 bis 5 oder eines ceramidartigen Glykolipids, das durch das Verfahren nach Anspruch 13 hergestellt wurde, mit einem CD1d-Protein; und
b) Bestrahlen des ceramidartigen Glykolipids und des CD1d-Proteins mit ultraviolettem Licht, um einen ceramidartigen Glykolipid-/Protein-Komplex herzustellen,
wobei der modifizierte Glykolipid-/Protein-Komplex die Aktivität von natürlichen Killer-T-Zellen (NKT-Zellen) erhöht.

## Revendications

1. Glycolipide sous forme de céramide, comprenant un groupe photoréactif, dans lequel :
- ledit groupe photoréactif est un groupe benzophénone lié de façon covalente au groupe caractéristique lipophile N-acyle dudit glycolipide sous forme de céramide ; et
- ledit glycolipide sous forme de céramide est un α-galactosylcéramide ;
dans lequel ledit glycolipide sous forme de céramide est capable de se lier de façon covalente à CD1d et d'améliorer l'activité de cellules tueuses naturelles (NKT).

2. Glycolipide sous forme de céramide selon la revendication 1, dans lequel ledit groupe caractéristique lipophile N-acyle est une chaîne d'acyle linéaire, et dans lequel ladite chaîne d'acyle linéaire a optionnellement au moins 11 atomes, optionnellement entre 11 et 14 atomes, dans lequel la numérotation d'atomes exclut des hydrogènes.

3. Glycolipide sous forme de céramide selon la revendication 2, dans lequel ladite chaîne d'acyle est sélectionnée à partir du groupe constitué de : 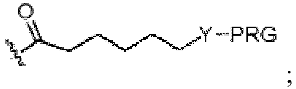 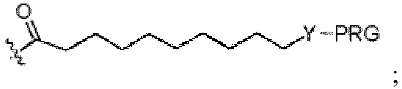 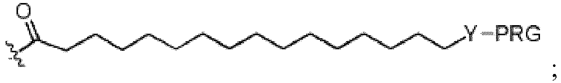 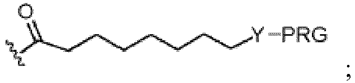 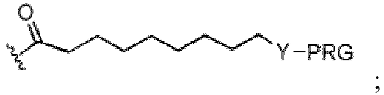 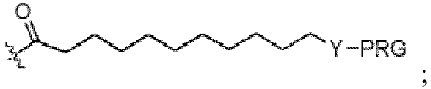 et 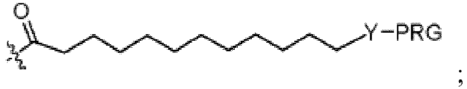 dans lequel Y est : -O-, -CH₂-, -S-, -OCH₂-, -SCH₂-, -CH₂CH₂- ; ou une liaison, et PRG est ledit groupe photoréactif,

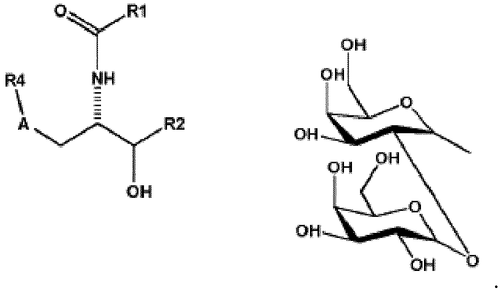

4. Glycolipide sous forme de céramide selon la revendication 1, dans lequel ledit α-galactosylcéramide est sélectionné à partir du groupe constitué de :
a) la formule II : 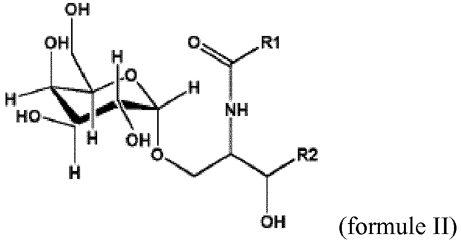 dans lequel
R1 est : un alcane C₁-C₂₇ ou alcène C₂-C₂₇ linéaire ou ramifié ; R1 est -C(OH)-R3 ; ou dans lequel R1 est sélectionné à partir du groupe constitué de : (CH₂)₉CH=CH-CH₂-CH=CH(CH₂)₄CH₃, (CH₂)₈CH=CH-CH₂-CH=CH(CH₂)₄CH₃, (CH₂)₇CH=CH-CH₂-CH=CH(CH₂)₄CH₃, (CH₂)₃CH=CH-CH₂-CH=CH-CH₂-CH=CH-CH₂-CH=CH-(CH₂)₄CH₃, (CH₂)₃CH=CH-CH₂-CH=CH-CH₂-CH=CH-CH₂-CH=CH-CH₂-CH=CH-CH₂CH₃, (CH₂)₇CH=CH-CH₂-CH=CH=(CH₂)₄CH₃, (CH₂)₇CH=CH-CH=CH(CH₂)₅CH₃, (CH₂)₈CH=CH-CH=CH(CH₂)₄CH₃, (CH₂)₉CH=CH-CH=CH(CH₂)₅CH₃, (CH₂)₆CH=CH-CH=CH-H=CH(CH₂)₄CH₃, (CH₂)₆CH=CH-CH=CH-CH=CH(CH₂)₄CH₃ et (CH₂)₇CH=CH-CH=CH-CH=CH(CH₂)₃CH₃ ;
dans lequel R3 est : alcane C₁-C₂₆ ou alcène C₂-C₂₆ linéaire ou ramifié ; et R2 est : un de (a) à (e) suivants :
(a) -CH₂(CH₂)ₓCH₃,
(b) -CH(OH)(CH₂)ₓCH₃,
(c) -CH(OH)(CH₂)ₓCH(CH₃)₂,
(d) -CH=CH(CH₂)ₓCH₃,
(e) -CH(OH)(CH₂)ₓCH(CH₃)CH₂CH₃,
dans lequel X est : un nombre entier relatif variant de 4 à 17,
dans lequel R2 est optionnellement : -CH(OH)(CH2)ₓCH3, dans lequel X est un nombre entier relatif variant de 4 à 13,
dans lequel R2 est optionnellement : -CH(OH)-(CH₂)₁₃CH₃ ;
b) la formule III : 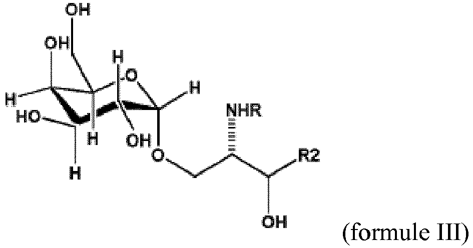 dans lequel R est : -C(O)R1, dans lequel R1 est : un alcane C₁-C₂₇ ou alcène C₂-C₂₇ linéaire ou ramifié ; ou R1 est : -C(OH)-R3, dans lequel R3 est : un alcane C₁-C₂₆ ou alcène C₂-C₂₆ linéaire ou ramifié ; ou R1 est : un alcane ou alcène C₆-C₂₇, dans lequel (i) l'alcane ou alcène C₆-C₂₇ est substitué avec un cycloalcane C₅-C₁₅, cycloalcène C₅-C₁₅, hétérocycle, ou anneau aromatique ou (ii) l'alcane ou alcène C₆-C₂₇ inclut, à l'intérieur de la chaîne d'alkyle ou d'alcényle C₆-C₂₇, un cycloalcane C₅-C₁₅, cycloalcène C₅-C₁₅, hétérocycle, ou anneau aromatique ; ou R1 est : un anneau aromatique optionnellement substitué, ou un aralkyle, ou R1 est sélectionné à partir du groupe constitué de : 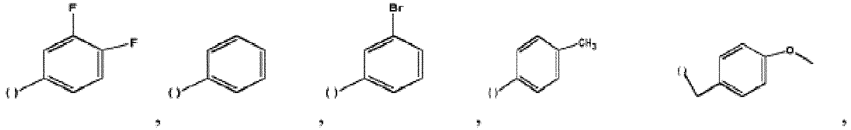 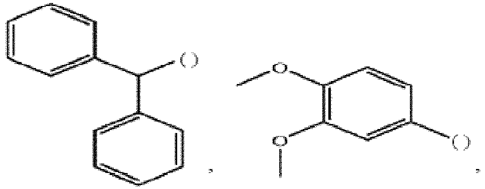 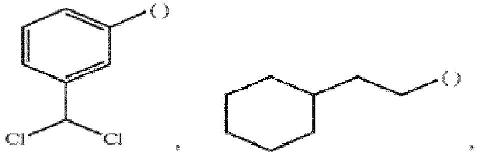 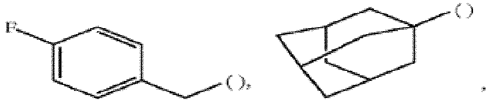 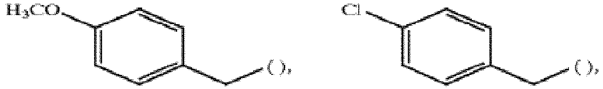 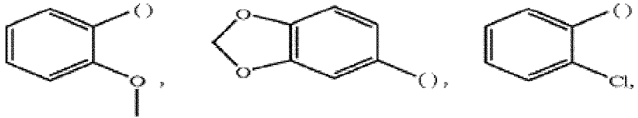 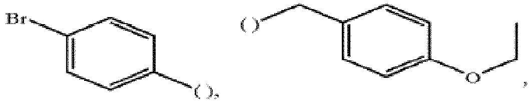 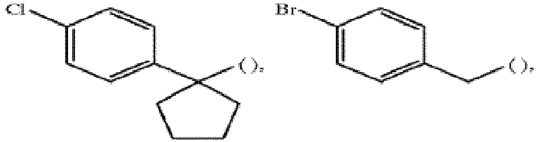 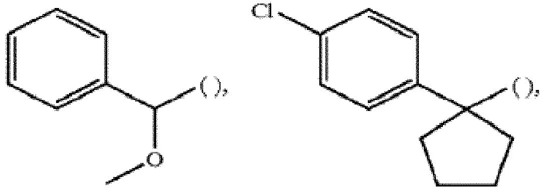 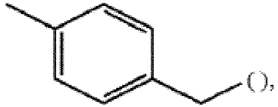 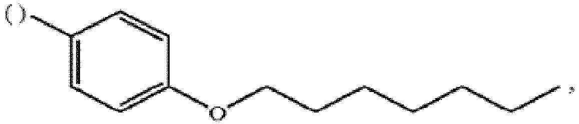 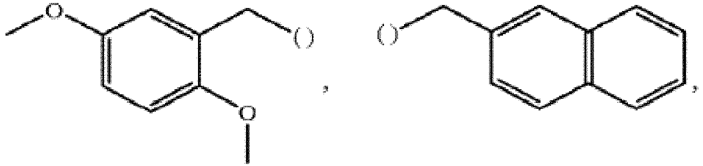 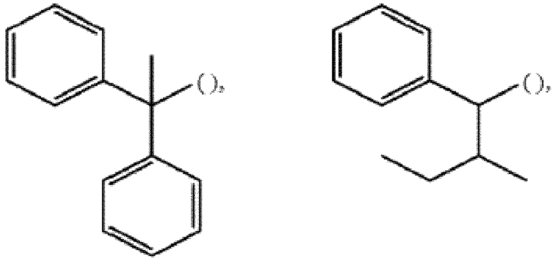 et 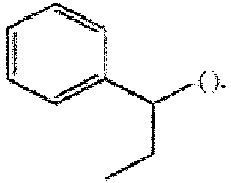
où () représente le point d'attachement de R1 au composé de la formule III ; et
R2 est : un de (a) à (e) suivants :
(a) -CH₂(CH₂)ₓCH₃,
(b) -CH(OH)(CH₂)ₓCH₃,
(c) -CH(OH)(CH₂)ₓCH(CH₃)₂,
(d) -CH=CH(CH₂)ₓCH₃,
(e) -CH(OH)(CH₂)ₓCH(CH₃)CH₂CH₃, dans lequel X est : un nombre entier relatif variant de 4 à 17,
dans lequel optionnellement R1 est substitué avec : oxo ; hydroxy ; halogène ; phényle ; - OC(O)R6 ; -OR6 ; -C(O)R6 ; ou N(R6)₂,
dans lequel chaque R6 est indépendamment : un alkyle substitué C₁-C₆, ou un anneau aromatique substitué optionnellement substitué avec : halogène ; hydroxy ;-OC(O)R7 ; -OR7 ; -C(O)R7 ou N(R7)₂, et dans lequel chaque R7 est : un alkyle C₁-C₆ substitué ; et
c) (2S, 3S, 4R)-1-O-(α-D-galactopyranosyl)-N-hexacosanoyl-2-amino-1,3,4-octadécanetriol (KRN7000), (2S,3S)-1-O-(α-D-galactopyranosyl)-N-hexacosanoyl-2-amino-1,3-octadécanediol), ou (2S, 3S, 4R)-1-CH₂-(α-galactopyranosyl)-N-hexacosanoyl-2-amino-1,3,4-octadécanetriol (α-C-GalCer).

5. Glycolipide sous forme de céramide selon la revendication 1, dans lequel ledit glycolipide sous forme de céramide a la structure sélectionnée à partir du groupe constitué de :
a) 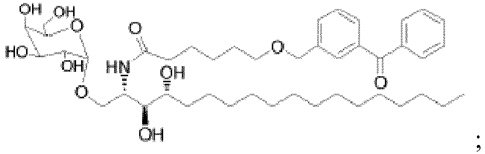
b) 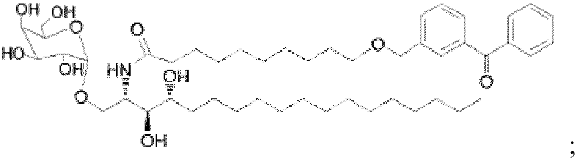
c) 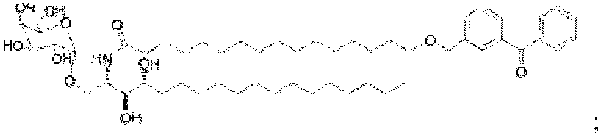
d) 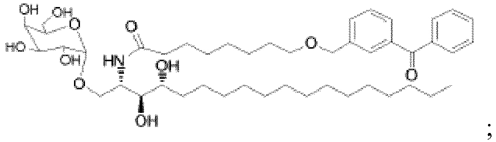
e) 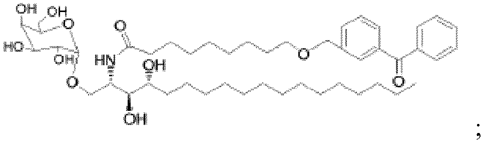
f) 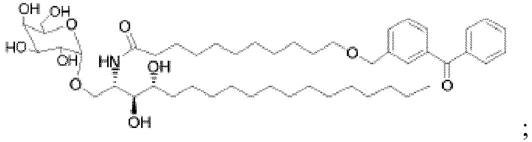 et
g)

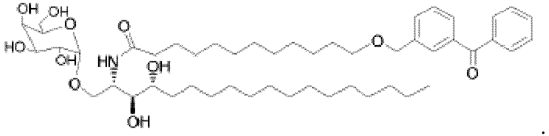

6. Complexe glycolipide sous forme de céramide/protéine, comprenant une protéine CD1d et le glycolipide sous forme de céramide de l'une quelconque des revendications 1 à 6, dans lequel ledit glycolipide sous forme de céramide est lié de façon covalente à ladite protéine par l'intermédiaire de photoactivation du groupe benzophénone.

7. Complexe glycolipide sous forme de céramide/protéine selon la revendication 6, dans lequel ladite CD1d :
a) a une séquence d'acides aminés sélectionnée à partir du groupe constitué de :
i) une séquence d'acides aminés au moins 90 % identique aux acides aminés 21 à 295 de SEQ ID n° : 1 ;
ii) la séquence d'acides aminés présentée sous la forme : acides aminés 21 à 295 de SEQ ID n° : 1 ;
iii) une séquence d'acides aminés identique à : acides aminés 21 à 295 de SEQ ID n° : 1, à l'exception de : au moins une mais moins de 10 substitutions conservatrices d'acides aminés ;
iv) la séquence d'acides aminés présentée sous la forme : acides aminés 1 à 295 de SEQ ID n° : 1 ;
v) une séquence d'acides aminés au moins 90 % identique à : acides aminés 1 à 295 de SEQ ID n° : 1 ;
vi) une séquence d'acides aminés identique à : acides aminés 21 à 295 de SEQ ID n° : 1, à l'exception de : au moins une mais moins de 10 substitutions conservatrices d'acides aminés ;
vii) la séquence d'acides aminés présentée dans : SEQ ID n° : 1 ;
viii) une séquence d'acides aminés au moins 90 % identique à SEQ ID n° : 1 ; et
ix) une séquence d'acides aminés identique à : SEQ ID n° : 1, à l'exception de : au moins une mais moins de 10 substitutions conservatrices d'acides aminés ; ou
b) est physiquement associée à une β2-microglobuline,
dans lequel ladite β2-microglobuline a optionnellement une séquence d'acides aminés sélectionnée à partir du groupe constitué de :
i) une séquence d'acides aminés au moins 90 % identique aux acides aminés 21 à 113 de SEQ ID n° : 2 ;
ii) la séquence d'acides aminés présentée sous la forme : acides aminés 21 à 113 de SEQ ID n° : 2 ;
iii) une séquence d'acides aminés identique à : acides aminés 21 à 113 de SEQ ID n° : 2, à l'exception de : au moins une mais moins de 10 substitutions conservatrices d'acides aminés ;
iv) la séquence d'acides aminés présentée dans : SEQ ID n° : 2 ;
v) une séquence d'acides aminés au moins 90 % identique à SEQ ID n° : 2 ; et
vi) une séquence d'acides aminés identique à : SEQ ID n° : 2, à l'exception de : au moins une mais moins de 10 substitutions conservatrices d'acides aminés.

8. Complexe glycolipide sous forme de céramide/protéine selon la revendication 6 ou 7, dans lequel ledit complexe glycolipide sous forme de céramide/protéine :
a) comprend en outre un anticorps ou fragment de celui-ci spécifique pour un antigène cible, et dans lequel ledit anticorps ou fragment de liaison à antigène de celui-ci est lié à ladite CD1d,
dans lequel ledit fragment de liaison à antigène est optionnellement sélectionné à partir du groupe constitué de : un fragment F(ab), un fragment F(ab')2, et un anticorps monocaténaire,
dans lequel ledit antigène cible est optionnellement : un marqueur de surface cellulaire de cellules tumorales,
dans lequel ledit marqueur de surface cellulaire est optionnellement sélectionné à partir du groupe constitué de : CEA, Her2/neu, EGFR type I ou type II, CD19, CD20, CD22, Muc-1, PSMA, ou STEAP,
dans lequel ladite CD1d est optionnellement attachée à la chaîne lourde ou chaîne légère dudit anticorps ou au fragment de chaîne lourde ou fragment de chaîne légère dudit fragment d'anticorps de liaison à antigène ; ou
b) comprend en outre un antigène,
dans lequel ledit antigène est optionnellement : un polypeptide immunogène d'un virus ou d'une cellule sélectionné à partir du groupe constitué de : un virus, une bactérie, un champignon, et une cellule tumorale.

9. Composition, comprenant le complexe glycolipide sous forme de céramide/protéine selon l'une quelconque des revendications 6 à 8, et un vecteur pharmaceutique,
dans laquelle ledit vecteur pharmaceutique est optionnellement sélectionné à partir du groupe constitué de : saline, saline tamponnée, dextrose, eau, glycérol, et associations de ceux-ci.

10. Complexe glycolipide sous forme de céramide/protéine selon l'une quelconque des revendications 6 à 8 ou composition selon la revendication 9 pour l'utilisation dans un procédé du traitement ou de la prévention d'une maladie chez un sujet, ledit procédé comprenant l'administration, à un sujet ayant besoin dudit traitement ou de ladite prévention, du complexe glycolipide sous forme de céramide/protéine ou de la composition, dans lequel/laquelle ledit complexe glycolipide sous forme de céramide/protéine est administré en une quantité suffisante pour altérer la progression de ladite maladie,
dans lequel/laquelle une réponse immunitaire est optionnellement améliorée ou modifiée relativement à une réponse immunitaire produite par la protéine liée de façon non covalente au glycolipide,
dans lequel/laquelle ladite maladie est optionnellement sélectionnée à partir du groupe constitué de : une maladie virale, une maladie bactérienne, une maladie fongique, une maladie parasitaire, une maladie proliférative, une maladie auto-immunitaire, une maladie inflammatoire, la tuberculose, une maladie pulmonaire ressemblant à la tuberculose, la lymphadénite, une maladie cutanée, une maladie disséminée, la peste bubonique, la peste pneumonique, la tularémie, la légionellose, la maladie du charbon, la fièvre typhoïde, la fièvre paratyphoïde, une maladie d'origine alimentaire, la listériose, le paludisme, le VIH, le VIS, le VPH, le RSV, la grippe, l'hépatite (VHA, VHB, et VHC), la sclérose en plaques, le diabète, le syndrome de Sjogren, et le cancer ;
dans lequel/laquelle ledit sujet est optionnellement un mammifère, et dans lequel/laquelle ledit mammifère est optionnellement un humain.

11. Complexe glycolipide sous forme de céramide/protéine selon l'une quelconque des revendications 6 à 8 ou composition selon la revendication 9 pour l'utilisation dans un procédé de l'entraînement d'une réponse immunitaire contre un antigène chez un sujet, ledit procédé comprenant l'administration, audit sujet, du complexe glycolipide sous forme de céramide/protéine ou de la composition,
dans lequel/laquelle ledit complexe glycolipide sous forme de céramide/protéine comprend optionnellement ledit antigène,
dans lequel/laquelle ledit antigène est optionnellement lié de façon covalente à ladite protéine,
dans lequel/laquelle ledit complexe glycolipide sous forme de céramide/protéine est optionnellement administré en une quantité suffisante pour améliorer l'activité de cellules T tueuses naturelles (NKT) chez ledit sujet,
dans lequel/laquelle ladite réponse immunitaire comprend optionnellement une réponse d'anticorps,
dans lequel/laquelle ledit sujet est optionnellement un mammifère, et dans lequel/laquelle ledit mammifère est optionnellement un humain.

12. Antigène hétérologue et complexe glycolipide sous forme de céramide/protéine de l'une quelconque des revendications 6 à 8 ou composition de la revendication 9 pour l'utilisation dans un procédé pour améliorer une réponse immunitaire, chez un sujet, à l'antigène hétérologue, ledit procédé comprenant l'administration, audit sujet, de l'antigène hétérologue et du complexe glycolipide sous forme de céramide/protéine ou de la composition simultanément, avant, ou après l'administration dudit antigène hétérologue,
dans lequel/laquelle ledit sujet est optionnellement un mammifère, et dans lequel/laquelle ledit mammifère est optionnellement un humain.

13. Procédé pour synthétiser un glycolipide sous forme de céramide de la formule (VI) comprenant un groupe benzophénone, ledit procédé comprenant les étapes de :
l'activation d'un dérivé d'acide carboxylique de la formule (IV) pour former un dérivé d'acyle activé 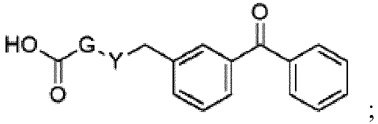 et
le couplage du dérivé d'acyle activé à un glycolipide sous forme de céramide de la formule (V), dans lequel ledit glycolipide sous forme de céramide est capable de se lier de façon covalente à la CD1d et d'améliorer l'activité de cellules T tueuses naturelles (NKT) 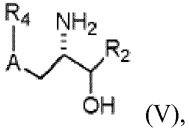
ainsi formant le glycolipide sous forme de céramide comprenant un groupe benzophénone de la formule (VI) 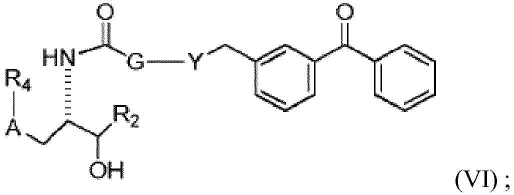 dans lequel
G est : un alcane C₁-C₂₇ ou alcène C₂-C₂₇ linéaire ou ramifié ; ou G est :-C(OH)-R3, dans lequel R3 est : un alcane C₁-C₂₆ ou alcène C₂-C₂₆ linéaire ou ramifié ; ou R1 est : un alcane ou alcène C₆-C₂₇, dans lequel (i) l'alcane ou alcène C₆-C₂₇ est substitué avec un cycloalcane C₅-C₁₅, cycloalcène C₅-C₁₅, hétérocycle, ou anneau aromatique, ou (ii) l'alcane ou alcène C₆-C₂₇ inclut, à l'intérieur de la chaîne d'alkyle ou d'alcényle C₆-C₂₇, un cycloalcane C₅-C₁₅, cycloalcène C₅-C₁₅, hétérocycle, ou anneau aromatique ; ou R1 est : un anneau aromatique optionnellement substitué, ou un aralkyle ;
R4 est : un α-galactosyl ;
A est : -O-, -CH₂- ; ou est une liaison unique,
Y est : -O-, -CH₂- ou-S-, et
R2 est : une chaîne alkyle, alcényle ou alkynyle C3-C40 substituée ou non substituée, dans lequel R2 de la formule (VI) est optionnellement un des (a) à (e) suivants :
(a) -CH₂(CH₂)ₓCH₃,
(b) -CH(OH)(CH₂)ₓCH₃,
(c) -CH(OH)(CH₂)ₓCH(CH₃)₂,
(d) -CH=CH(CH₂)ₓCH₃,
(e) -CH(OH)(CH₂)ₓCH(CH₃)CH₂CH₃, dans lequel x est un nombre entier relatif variant de 0 à 40, ou
dans lequel R2 de la formule (VI) est optionnellement :
-CH(OH)(CH₂)ₓCH₃, dans lequel x est un nombre entier relatif variant de 4 à 17 ; ou
-CH(OH)(CH₂)₁₃CH₃ ; ou
dans lequel, optionnellement, G est : -(CH₂)n-, dans lequel n est un nombre entier relatif variant de 0 à 20 et Y est : O ; ou
dans lequel :
G est : -(CH₂)n-, dans lequel n est un nombre entier relatif variant de 0 à 20 et Y est : O ;
R2 est : -CH(OH)(CH₂)ₓCH₃, dans lequel x est un nombre entier relatif variant de 4 à 17 ;
A est : O ;
Y est : O ; et
dans lequel ledit glycolipide sous forme de céramide est capable de se lier de façon covalente à CD1d et d'améliorer l'activité de cellules T tueuses naturelles (NKT).

14. Procédé pour produire un complexe glycolipide sous forme de céramide/protéine, ledit procédé comprenant les étapes de :
a) la mise en contact du glycolipide sous forme de céramide de l'une quelconque des revendications 1 à 5 ou d'un glycolipide sous forme de céramide produit par le procédé de la revendication 13 avec une protéine CD1d ; et
b) l'irradiation dudit glycolipide sous forme de céramide et de ladite protéine CD1d avec de la lumière ultraviolette pour produire un complexe glycolipide sous forme de céramide/protéine,
dans lequel ledit complexe glycolipide/protéine modifié améliore l'activité de cellules T tueuses naturelles (NKT).


### SEQUENCE LISTING

<110> Porcelli, Steven A. Zauderer, Maurice
<120> Modified glycolipids and methods of
   making and using the same
<130> 50827-441420
<150> 61/762,591
   <151> 2013-02-08
<150> 13/803,972
   <151> 2013-03-14
<150> 61/842,149
   <151> 2013-07-02
<160> 2
<170> FastSEQ for Windows Version 4.0
<210> 1
   <211> 335
   <212> PRT
   <213> Homo sapiens
<400> 1
   Met Gly Cys Leu Leu Phe Leu Leu Leu Trp Ala Leu Leu Gln Ala Trp
    1               5                  10                  15
   Gly Ser Ala Glu Val Pro Gln Arg Leu Phe Pro Leu Arg Cys Leu Gln
               20                  25                  30
   Ile Ser Ser Phe Ala Asn Ser Ser Trp Thr Arg Thr Asp Gly Leu Ala
           35                  40                  45
   Trp Leu Gly Glu Leu Gln Thr His Ser Trp Ser Asn Asp Ser Asp Thr
       50                  55                  60
   Val Arg Ser Leu Lys Pro Trp Ser Gln Gly Thr Phe Ser Asp Gln Gln
   65                  70                  75                  80
   Trp Glu Thr Leu Gln His Ile Phe Arg Val Tyr Arg Ser Ser Phe Thr
                   85                  90                  95
   Arg Asp Val Lys Glu Phe Ala Lys Met Leu Arg Leu Ser Tyr Pro Leu
               100                 105                 110
   Glu Leu Gln Val Ser Ala Gly Cys Glu Val His Pro Gly Asn Ala Ser
           115                 120                 125
   Asn Asn Phe Phe His Val Ala Phe Gln Gly Lys Asp Ile Leu Ser Phe
       130                 135                 140
   Gln Gly Thr Ser Trp Glu Pro Thr Gln Glu Ala Pro Leu Trp Val Asn
   145                 150                 155                 160
   Leu Ala Ile Gln Val Leu Asn Gln Asp Lys Trp Thr Arg Glu Thr Val
                   165                 170                 175
   Gln Trp Leu Leu Asn Gly Thr Cys Pro Gln Phe Val Ser Gly Leu Leu
               180                 185                 190
   Glu Ser Gly Lys Ser Glu Leu Lys Lys Gln Val Lys Pro Lys Ala Trp
           195                 200                 205
   Leu Ser Arg Gly Pro Ser Pro Gly Pro Gly Arg Leu Leu Leu Val Cys
       210                 215                 220
   His Val Ser Gly Phe Tyr Pro Lys Pro Val Trp Val Lys Trp Met Arg
   225                 230                 235                 240
   Gly Glu Gln Glu Gln Gln Gly Thr Gln Pro Gly Asp Ile Leu Pro Asn
                   245                 250                 255
   Ala Asp Glu Thr Trp Tyr Leu Arg Ala Thr Leu Asp Val Val Ala Gly
               260                 265                 270
   Glu Ala Ala Gly Leu Ser Cys Arg Val Lys His Ser Ser Leu Glu Gly
           275                 280                 285
   Gln Asp Ile Val Leu Tyr Trp Gly Gly Ser Tyr Thr Ser Met Gly Leu
       290                 295                 300
   Ile Ala Leu Ala Val Leu Ala Cys Leu Leu Phe Leu Leu Ile Val Gly
   305                 310                 315                 320
   Phe Thr Ser Arg Phe Lys Arg Gln Thr Ser Tyr Gln Gly Val Leu
                   325                 330                 335
<210> 2
   <211> 119
   <212> PRT
   <213> Homo sapiens
<400> 2
   Met Ser Arg Ser Val Ala Leu Ala Val Leu Ala Leu Leu Ser Leu Ser
    1               5                  10                  15
   Gly Leu Glu Ala Ile Gln Arg Thr Pro Lys Ile Gln Val Tyr Ser Arg
               20                  25                  30
   His Pro Ala Glu Asn Gly Lys Ser Asn Phe Leu Asn Cys Tyr Val Ser
           35                  40                  45
   Gly Phe His Pro Ser Asp Ile Glu Val Asp Leu Leu Lys Asn Gly Glu
       50                  55                  60
   Arg Ile Glu Lys Val Glu His Ser Asp Leu Ser Phe Ser Lys Asp Trp
   65                  70                  75                  80
   Ser Phe Tyr Leu Leu Tyr Tyr Thr Glu Phe Thr Pro Thr Glu Lys Asp
                   85                  90                  95
   Glu Tyr Ala Cys Arg Val Asn His Val Thr Leu Ser Gln Pro Lys Ile
               100                 105                 110
   Val Lys Trp Asp Arg Asp Met
           115